# EUROPEAN PATENT APPLICATION

(11) **EP 4 722 228 A1**
(43) Date of publication of application: **08.04.2026**
(21) Application number: 24815523.6
(22) Date of filing: 29.05.2024
(51) Int. Cl.: C07K 7/04, A61K 38/12, A61P 21/04, C12N 15/11, C12P 21/02

(54) **NEW COMPOUND TARGETING MUSK AND USE THEREOF**

(30) Priority: 29.05.2023 JP 2023088120
(71) Applicant: The University of Tokyo, Bunkyo-ku, Tokyo 113-8654 (JP); National Hospital Organization, Tokyo 152-8621 (JP)
(72) Inventor: SUGA Hiroaki, Tokyo 113-8654 (JP); PEACOCK Hayden, Tokyo 113-8654 (JP); YAMADA Mituhiro, Tokyo 113-8654 (JP); KAWAKAMI Naoya, Tokyo 113-8654 (JP); HIGUCHI Osamu, Higashisonogi-gun, Nagasaki 859-3615 (JP)
(74) Representative: ABG Intellectual Property Law, S.L.
(86) International application number: PCT/JP2024/019710
(87) International publication number: WO 2024/248042

(57) **Abstract**

An object of the present invention is to provide a novel compound that activates signal transmission from a nerve to a muscle at a neuromuscular junction and a novel therapeutic agent for myasthenia gravis. According to the present invention, a cyclic peptide having a structure of formula (I), a complex thereof, or a pharmaceutically acceptable salt thereof is provided. -X₁-Asn-X₂-Val- (I) wherein, X₁ represents Thr or Val, and X₂ represents Val or Ile. According to the present invention, there is provided a therapeutic agent for myasthenia gravis containing, as an active ingredient, the cyclic peptide, the complex thereof, or the pharmaceutically acceptable salt thereof.

## Description

### CROSS-REFERENCE TO RELATED APPLICATION

The present application enjoys the benefit of priority from the prior Japanese Patent Application No. 2023-88120 (filed on May 29, 2023), the entire disclosures of which are incorporated herein by reference.

### TECHNICAL FIELD

The present invention relates to a novel compound that activates signal transmission at a neuromuscular junction and to a therapeutic agent for myasthenia gravis. The present invention also relates to a method for selecting a ubiquitin molecule to which a pharmacophore of a cyclic peptide has been grafted.

### BACKGROUND ART

Myasthenia gravis (MG) is one of autoimmune diseases known as diseases in which autoantibodies are directly involved in the pathological condition, and is characterized in that generalized muscle weakness and easy fatigability appear, and in particular, eye symptoms such as blepharoptosis and double vision tend to occur (Non-Patent Document 1). In myasthenia gravis, it is considered that some molecules present on the muscle side (signal receiver) are destroyed or functionally inhibited by autoantibodies at the connection between a peripheral nerve and a muscle (neuromuscular junction), and a signal is not transmitted from the nerve to the muscle, so that muscle weakness occurs. The molecule most frequently targeted by autoantibodies is an acetylcholine receptor (AChR), which accounts for about 85% of the total, followed by muscle-specific tyrosine kinase (MuSK) and low-density lipoprotein receptor-related protein 4 (LRP4), which are considered to account for several percent in total. In addition, it is also known that there are seronegative type patients in whom all of these autoantibodies are negative.

Symptomatic treatments widely used for myasthenia gravis are steroid therapy and immunosuppressant therapy. However, patients inevitably bear various side effects as the price of treatment, and there are many cases that are difficult to treat. Recently, a monoclonal antibody, "Eculizumab" (trade name: Soliris) targeting complement protein C5 has been marketed as a molecular targeted drug for the treatment of myasthenia gravis, and a cyclic peptide, "Zilucoplan" having a similar mechanism of action is under clinical development (see Patent Documents 1 and 2). These drugs are intended for the treatment of myasthenia gravis positive for anti-acetylcholine receptor antibodies; however, none of these drugs can be applied to cases with an increased risk of infection with meningococcus or the like, to myasthenia gravis in which complement activation is not involved, or to myasthenia gravis other than the anti-acetylcholine receptor antibody-positive type.

### Reference List

### Patent Documents

Patent Document 1: JP 2019-517473 A
Patent Document 2: JP 2022-508952 A

### Non Patent Document

Non Patent Document 1: Gilhus N. E. & Verschuuren J. J, Lancet Neurol. 2015 Oct;14(10):1023-36.

### SUMMARY OF THE INVENTION

An object of the present invention is to provide a novel compound that activates signal transmission from a nerve to a muscle at a neuromuscular junction and a novel therapeutic agent for myasthenia gravis. Another object of the present invention is to provide a novel method for selecting a ubiquitin molecule to which a pharmacophore of a cyclic peptide has been grafted.

According to the present invention, the following inventions are provided.
[1] A cyclic peptide or a pharmaceutically acceptable salt thereof, the cyclic peptide having a structure of the following formula (I):

   -X₁-Asn-X₂-Val- (I)

   wherein, X₁ represents Thr or Val, and X₂ represents Val or Ile.
[2] The cyclic peptide or the pharmaceutically acceptable salt thereof according to [1] above, wherein the number of amino acid residues forming the cyclic structure of the cyclic peptide is 8 to 20.
[3] The cyclic peptide or the pharmaceutically acceptable salt thereof according to [1] or [2] above, wherein the cyclic structure of the cyclic peptide includes an N-CO-CH₂-S structure.
[4] The cyclic peptide or the pharmaceutically acceptable salt thereof according to [3] above, wherein the N is an amino group of D-tyrosine.
[5] The cyclic peptide or the pharmaceutically acceptable salt thereof according to [3] or [4] above, wherein the S is a thiol group of cysteine.
[6] The cyclic peptide or the pharmaceutically acceptable salt thereof according to any one of [1] to [5] above, wherein the cyclic peptide has a structure selected from the group consisting of the following formula (1) (SEQ ID NO: 2), formula (2) (SEQ ID NO: 3), and formula (3) (SEQ ID NO: 4): 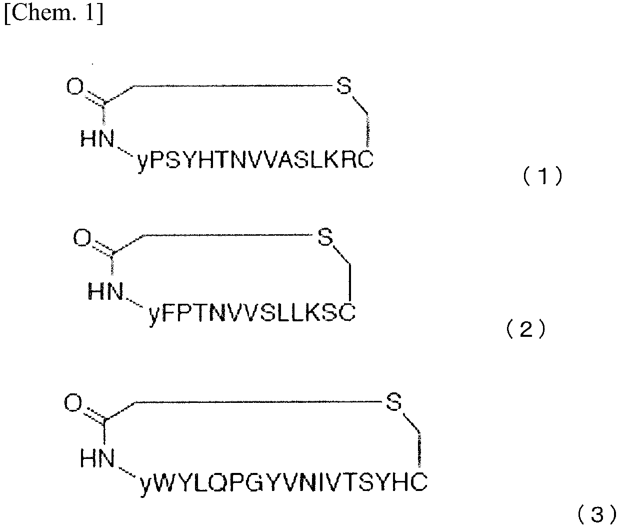 wherein, the carboxyl group of the cysteine residue (C) may form an acid amide.
[7] A cyclic peptide complex or the pharmaceutically acceptable salt thereof, the cyclic peptide complex comprising: two cyclic peptides according to any one of [1] to [6] above, wherein one of the cyclic peptides is bonded to the other cyclic peptide via a linker.
[8] The cyclic peptide complex or the pharmaceutically acceptable salt thereof according to [7] above, wherein the two cyclic peptides are the same cyclic peptide.
[9] The cyclic peptide complex or the pharmaceutically acceptable salt thereof according to [7] or [8] above, wherein the length of the linker is 20Å to 100Å.
[10] The cyclic peptide complex or the pharmaceutically acceptable salt thereof according to any one of [7] to [9] above, wherein the linker has a polyethylene glycol structure as at least a part of the structure.
[11] The cyclic peptide complex or the pharmaceutically acceptable salt thereof according to any one of [7] to [10] above, wherein the linker has at least one saturated hydrocarbon group having 8 to 20 carbon atoms as a side chain thereof.
[12] A MuSK-binding-region-grafted ubiquitin molecule or a pharmaceutically acceptable salt thereof, wherein a part or all of a loop structure region between a β1 domain and a β2 domain of the ubiquitin molecule is replaced with a peptide of the following structure (A), or the peptide of the structure (A) is inserted into the loop structure region between the β1 domain and the β2 domain of the ubiquitin molecule: 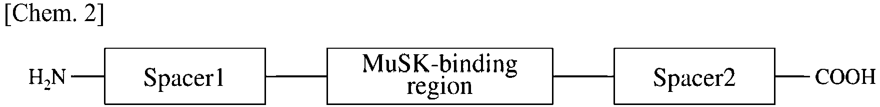 wherein
   the MuSK-binding region consists of the amino acid sequence YX₂₁SYHTNVVASLKR (X₂₁ is P or L) (SEQ ID NO: 11),
   spacer 1 consists of an amino acid sequence selected from the group consisting of IQPR (SEQ ID NO: 12), KPGV (SEQ ID NO: 13), WDRG (SEQ ID NO: 14), KPGT (SEQ ID NO: 15), PFGV (SEQ ID NO: 16), YFVG (SEQ ID NO: 17), and LEGM (SEQ ID NO: 18); and
   spacer 2 consists of an amino acid sequence selected from the group consisting of IRY, MMP, QGI, FMP, TGPQ (SEQ ID NO: 19), WNTP (SEQ ID NO: 20), and IRPQ (SEQ ID NO: 21).
[13] The MuSK-binding-region-grafted ubiquitin molecule or the pharmaceutically acceptable salt thereof according to [12] above, wherein the ubiquitin molecule is a protein selected from the group consisting of:
   (a) a protein consisting of the amino acid sequence of SEQ ID NO: 1,
   (b) a protein consisting of an amino acid sequence having 90% or more identity to the amino acid sequence of SEQ ID NO: 1, and
   (c) a protein consisting of an amino acid sequence in which one or a plurality of amino acids in the amino acid sequence of SEQ ID NO: 1 are modified.
[14] The MuSK-binding-region-grafted ubiquitin molecule or the pharmaceutically acceptable salt thereof according to [12] or [13] above, wherein the structure (A) has an amino acid sequence selected from the group consisting of the following.
   (Ub006) IQPRYPSYHTNVVASLKRIRY (SEQ ID NO: 22)
   (Ub007) KPGVYPSYHTNVVASLKRMMP (SEQ ID NO: 23)
   (Ub008) WDRGYPSYHTNVVASLKRQGI (SEQ ID NO: 24)
   (Ub009) KPGTYPSYHTNVVASLKRFMP (SEQ ID NO: 25)
   (Ub010) PFGVYPSYHTNVVASLKRTGPQ (SEQ ID NO: 26)
   (Ub011) YFVGYPSYHTNVVASLKRWNTP (SEQ ID NO: 27)
   (Ub012) LEGMYLSYHTNVVASLKRIRPQ (SEQ ID NO: 28)
   (Ub013) LEGMYPSYHTNVVASLKRIRPQ (SEQ ID NO: 29)
[15] The MuSK-binding-region-grafted ubiquitin molecule or the pharmaceutically acceptable salt thereof according to any one of [12] to [14] above, wherein the loop structure region is a region from the 7th amino acid residue to the 10th amino acid residue of the ubiquitin molecule.
[16] A MuSK-binding-region-grafted ubiquitin molecule complex or a pharmaceutically acceptable salt thereof, the MuSK-binding-region-grafted ubiquitin molecule complex comprising two or more (for example, 2 to 6, preferably 2 to 4) of the MuSK-binding-region-grafted ubiquitin molecules according to any one of [12] to [15] above, wherein one of the MuSK-binding-region-grafted ubiquitin molecules is bonded to the other MuSK-binding-region-grafted ubiquitin molecule via a linker.
[17] The MuSK-binding-region-grafted ubiquitin molecule complex or the pharmaceutically acceptable salt thereof according to [16] above, wherein the two or more MuSK-binding-region-grafted ubiquitin molecules are the same molecule.
[18] The MuSK-binding-region-grafted ubiquitin molecule complex or the pharmaceutically acceptable salt thereof according to [16] or [17] above, wherein the length of the linker is 20Å to 100Å.
[19] The MuSK-binding-region-grafted ubiquitin molecule complex or the pharmaceutically acceptable salt thereof according to any one of [16] to [18] above, wherein the linker is a peptide chain.
[20] A pharmaceutical composition or a therapeutic agent for myasthenia gravis, comprising, as an active ingredient: the cyclic peptide or the pharmaceutically acceptable salt thereof according to any one of [1] to [6] above; the cyclic peptide complex or the pharmaceutically acceptable salt thereof according to any one of [7] to [11] above; the MuSK-binding-region-grafted ubiquitin molecule or the pharmaceutically acceptable salt thereof according to any one of [12] to [15] above; or the MuSK-binding-region-grafted ubiquitin molecule complex or the pharmaceutically acceptable salt thereof according to any one of [16] to [19] above.
[20-1] A method for treating myasthenia gravis, comprising: administering to a subject in need of a therapeutically effective amount of the cyclic peptide or the pharmaceutically acceptable salt thereof according to any one of [1] to [6] above, the cyclic peptide complex or the pharmaceutically acceptable salt thereof according to any one of [7] to [11] above, the MuSK-binding-region-grafted ubiquitin molecule or the pharmaceutically acceptable salt thereof according to any one of [12] to [15] above, or the MuSK-binding-region-grafted ubiquitin molecule complex or the pharmaceutically acceptable salt thereof according to any one of [16] to [19] above, or a therapeutic agent or pharmaceutical composition containing the same.
[20-2] The cyclic peptide or the pharmaceutically acceptable salt thereof according to any one of [1] to [6] above, the cyclic peptide complex or the pharmaceutically acceptable salt thereof according to any one of [7] to [11] above, the MuSK-binding-region-grafted ubiquitin molecule or the pharmaceutically acceptable salt thereof according to any one of [12] to [15] above, or the MuSK-binding-region-grafted ubiquitin molecule complex or the pharmaceutically acceptable salt thereof according to any one of [16] to [19] above, for use as a medicament.
[20-3] The cyclic peptide or the pharmaceutically acceptable salt thereof, the cyclic peptide complex or the pharmaceutically acceptable salt thereof, the MuSK-binding-region-grafted ubiquitin molecule or the pharmaceutically acceptable salt thereof, or the MuSK-binding-region-grafted ubiquitin molecule complex or the pharmaceutically acceptable salt thereof, according to [20-2] above, for use in the treatment of myasthenia gravis.
[20-4] Use of the cyclic peptide or the pharmaceutically acceptable salt thereof according to any one of [1] to [6] above, the cyclic peptide complex or the pharmaceutically acceptable salt thereof according to any one of [7] to [11] above, the MuSK-binding-region-grafted ubiquitin molecule or the pharmaceutically acceptable salt thereof according to any one of [12] to [15] above, or the MuSK-binding-region-grafted ubiquitin molecule complex or the pharmaceutically acceptable salt thereof according to any one of [16] to [19] above, for the manufacture of a medicament or a therapeutic agent for myasthenia gravis, in a method for treating myasthenia gravis, or as a medicament or a therapeutic agent for myasthenia gravis.
[21] A method for presenting a cyclic peptide having binding activity to a desired molecule (preferably a MuSK molecule) together with two spacer sequences on a loop structure region between a β1 domain and a β2 domain of a ubiquitin molecule, the method comprising:
   fusing the cyclic peptide to the ubiquitin molecule while retaining binding activity to the desired molecule (preferably the MuSK molecule) by replacing a chemical cross-linking structure of the cyclic peptide with two amino acid residues constituting the loop structure via the spacer sequences, wherein
   the cyclic peptide has the chemical cross-linking structure for forming an intramolecular cyclic structure, and
   the spacer sequences consist of an amino acid sequence of 0 to 4 residues (preferably 3 or 4 residues) randomly generated.
[22] The presentation method according to [21] above, wherein
   the desired molecule is a MuSK molecule, and the cyclic peptide and the spacer sequences presented on the loop structure region of the ubiquitin molecule have the following structure (B): 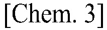 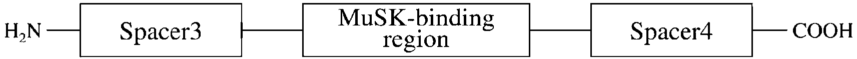
   wherein, spacer 3 is a peptide chain consisting of 0 to 4 (preferably 3 or 4) arbitrary amino acids, spacer 4 is a peptide chain consisting of 0 to 4 (preferably 3 or 4) arbitrary amino acids, and the MuSK-binding region is a region which is present in the cyclic peptide and has MuSK molecule-binding activity.
[23] The presentation method according to [21] or [22] above, wherein the loop structure region of the ubiquitin molecule is a loop structure region between a β1 domain and a β2 domain.
[24] The presentation method according to [23] above, wherein the two amino acid residues constituting the loop structure region are the 7th amino acid residue and the 10th amino acid residue of the ubiquitin molecule.
[25] The presentation method according to any one of [22] to [24] above, wherein the MuSK-binding region is a peptide chain having the amino acid sequence YX₂₂SYHTNVVASLKR (X₂₂ is P or L) (SEQ ID NO: 11).
[26] A method for producing a modified protein in which a cyclic peptide having binding activity to a desired molecule (preferably a MuSK molecule) is presented on a protein by fusing the cyclic peptide, together with two spacer sequences, to a loop structure region of a ubiquitin molecule, the method comprising:
   selecting a partial amino acid sequence to be presented on the protein from the amino acid sequence of the cyclic peptide, and selecting a nucleotide sequence corresponding to the partial amino acid sequence;
   selecting a nucleotide sequence corresponding to two amino acid residues constituting the loop structure region of the ubiquitin molecule, deleting bases present between the selected nucleotide sequences as necessary, and preparing a nucleic acid having an incorporated nucleotide sequence obtained by inserting the nucleotide sequence corresponding to the selected partial amino acid sequence of the cyclic peptide; and
   translating the nucleic acid, wherein
   the cyclic peptide has a chemical cross-linking structure for forming an intramolecular cyclic structure, and
   the spacer sequences consist of an amino acid sequence of 0 to 4 residues (preferably 3 or 4 residues) randomly generated.
[27] The production method according to [26] above, wherein
   the desired molecule is a MuSK molecule, and the cyclic peptide and the spacer sequences presented on the loop structure region of the ubiquitin molecule have the following structure (B): 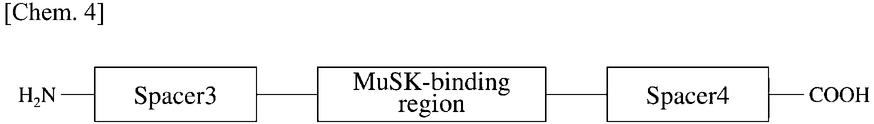
   wherein, spacer 3 is a peptide chain consisting of 0 to 4 (preferably 3 or 4) arbitrary amino acids, spacer 4 is a peptide chain consisting of 0 to 4 (preferably 3 or 4) arbitrary amino acids, and the MuSK-binding region is a region which is present in the cyclic peptide and has MuSK molecule-binding activity.
[28] The production method according to [26] or [27] above, wherein the loop structure region of the ubiquitin molecule is a loop structure region between a β1 domain and a β2 domain.
[29] The production method according to [28] above, wherein the two amino acid residues constituting the loop structure region are the 7th amino acid residue and the 10th amino acid residue of the ubiquitin molecule.
[30] The production method according to any one of [27] to [29] above, wherein the MuSK-binding region is a peptide chain having the amino acid sequence YX₂₂SYHTNVVASLKR (X₂₂ is P or L) (SEQ ID NO: 11).
[31] A method for screening for a modified protein which binds to a desired molecule (preferably a MuSK-binding modified protein), the method comprising:
   designing an mRNA library including a nucleotide sequence encoding a ubiquitin molecule in which a sequence of a cyclic peptide having binding activity to the desired molecule (preferably a MuSK molecule) and spacer sequences consisting of amino acid sequences of 0 to 4 residues (preferably 3 or 4 residues) randomly generated and linked upstream and downstream of the cyclic peptide sequence are fused to a loop structure region between a β1 domain and a β2 domain;
   preparing a modified protein according to the production method according to [28] above on the basis of the designed mRNA library; and
   selecting, from the obtained modified protein, a modified protein having binding property to the desired molecule (preferably the MuSK-binding modified protein), using binding property (preferably MuSK-binding activity) to the desired molecule as an index.
[32] A peptide or a pharmaceutically acceptable salt thereof, the peptide having a structure of the following formula (I):

   -X₁-Asn-X₂-Val- (I)

   wherein, X₁ represents Thr or Val, and X₂ represents Val or Ile.
[33] The peptide or the pharmaceutically acceptable salt thereof according to [32] above, wherein the peptide consists of 8 to 20 amino acid residues.
[34] The peptide or the pharmaceutically acceptable salt thereof according to [32] or [33] above, the peptide being grafted onto a biomolecule.

The cyclic peptides, MuSK-binding-region-grafted ubiquitin molecules, and complexes thereof provided by the present invention can directly enhance signals essential for the formation and maintenance of neuromuscular junctions. Accordingly, the present invention is advantageous in that it provides novel modality molecules that enable treatment of myasthenia gravis.

### BRIEF DESCRIPTION OF THE DRAWINGS

Fig. 1 is a diagram schematically illustrating a mechanism of homodimerization of MuSK and subsequent clustering of acetylcholine receptors.
Fig. 2 is a diagram schematically illustrating a mechanism of homodimerization of MuSK caused by binding of a MuSK-binding dimer peptide to a MuSK extracellular portion and subsequent clustering of acetylcholine receptors.
Fig. 3A is a schematic diagram of an approach to generate a library of thioether-cyclic peptides using a flexible in vitro translation (FIT) system and rapidly search for cyclic peptides binding to a target by RaPID screening. Fig. 3B is a schematic diagram of an approach to graft a pharmacophore region of a special cyclic peptide selected by RaPID screening to a scaffold protein by a lasso-grafting method, and to arrange spacers upstream and downstream of the grafted peptide, thereby preparing a library of the constructed mRNA fusion proteins.
Fig. 4 is a diagram showing the results of selection of peptide sequences identified from an initial RaPID screening for affinity to MuSK. The progress of affinity selection is expressed as the percentage (%) of the number of mRNAs bound to MuSK-bead complexes (Positive) or the percentage (%) of the number of mRNAs bound to beads alone (Negative) relative to the total number of mRNAs used in each round of affinity selection.
Fig. 5 is a diagram showing SPR sensorgrams of MuSK-binding monomer peptides analyzed using a Biacore T200 in a concentration range of 1.6 to 1000 nM. Curves fitted to the measured curves are also shown.
Fig. 6-1 is a diagram showing structures of homodimers of cyclic peptides (MuSK4, MuSK7, and MuSK9).
Fig. 6-2 is a diagram showing structures of homodimers of a linear peptide (MuSK1Ac).
Fig. 7 is a diagram showing the chemical structure of a homodimer of MuSK7 (MuSK7 PEG11 dimer) in which Bis-Mal-dPEG (trade name) 11 is used as a linker.
Fig. 8 is a diagram showing the chemical structure of a homodimer of MuSK7 into which a palmitoyl group is introduced.
Fig. 9A is a diagram showing that the cyclic peptide complex (MuSK7 PEG11 dimer) of the present invention induces clustering of acetylcholine receptors in a concentration-dependent manner at the cultured myotube cell level. Fig. 9B is a dose-response curve of the cyclic peptide complex (MuSK7 PEG11 dimer) of the present invention with respect to the number of clusters of the acetylcholine receptors. Fig. 9C is a diagram showing the effect of AST-487 on acetylcholine receptor cluster-inducing activity.
Fig. 10A is a diagram schematically illustrating two molecules used in a NanoBiT assay. When two extracellular portions of human MuSK interact, LgBiT and SmBiT bind to each other to form a luminescent enzyme, and luminescence can be observed upon addition of a substrate. Fig. 10B is an evaluation result using the NanoBiT assay and is a dose-response curve regarding MuSK dimerization of the cyclic peptide complex (MuSK7-pal) of the present invention.
Fig. 11 is a diagram showing evaluation results (body weight change) of therapeutic effects of the cyclic peptide complex (high-dose) of the present invention on myasthenia gravis model animals.
Fig. 12 is a diagram showing evaluation results (movement score) of therapeutic effects of the cyclic peptide complex (high-dose) of the present invention on myasthenia gravis model animals.
Fig. 13 is a diagram showing evaluation results (latency to fall (Latency) in the Hanging Wire Test) of therapeutic effects of the cyclic peptide (high-dose) complex of the present invention on myasthenia gravis model animals.
Fig. 14 is a diagram showing evaluation results (grip strength) of therapeutic effects of the cyclic peptide complex (high-dose) of the present invention on myasthenia gravis model animals.
Fig. 15 is a diagram showing evaluation results (body weight change) of therapeutic effects of the cyclic peptide complex (low-dose) of the present invention on myasthenia gravis model animals.
Fig. 16 is a diagram showing evaluation results (movement score) of therapeutic effects of the cyclic peptide (low-dose) complex of the present invention on myasthenia gravis model animals.
Fig. 17 is a diagram showing evaluation results (latency to fall (Latency) in the Hanging Wire Test) of therapeutic effects of the cyclic peptide complex (low-dose) of the present invention on myasthenia gravis model animals.
Fig. 18 is a diagram showing evaluation results (grip strength) of therapeutic effects of the cyclic peptide complex (low-dose) of the present invention on myasthenia gravis model animals.

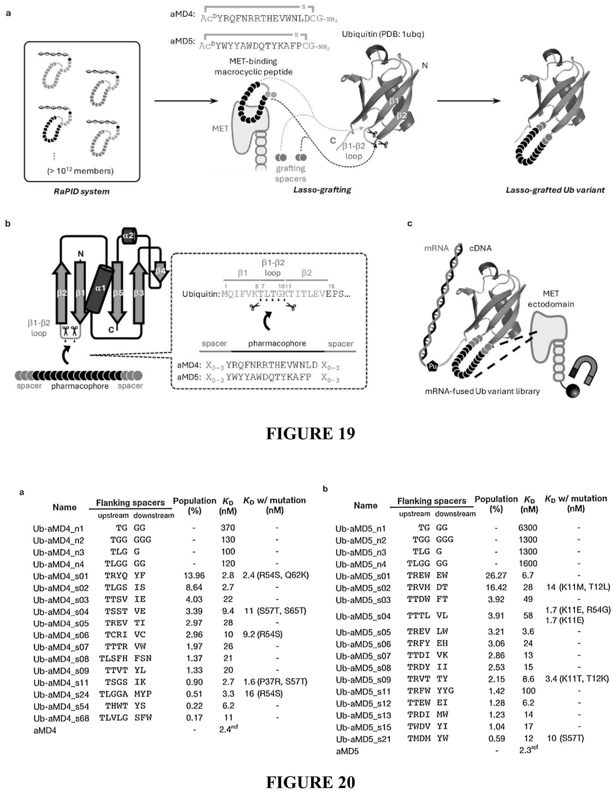

Fig. 19a is a diagram schematically illustrating an approach to lasso-graft MET-binding macrocyclic peptides (aMD4 and aMD5) obtained by a RaPID system to a ubiquitin molecule (β1-β2 loop). Fig. 19b is a diagram schematically illustrating a strategy to perform grafting by adding spacer sequences of 0 to 3 amino acid residues upstream and downstream of pharmacophore sequences of aMD4 and aMD5 when lasso-grafting aMD4 and aMD5 onto a ubiquitin molecule. Fig. 19c is a schematic diagram of an mRNA display strategy, and is a diagram schematically illustrating a translation product (ubiquitin molecule lasso-grafted with aMD4 or aMD5) bound to mRNA via puromycin. The ubiquitin molecule obtained by grafting a binding-active region is referred to as a U-body in the present specification, and ubiquitin molecule may be abbreviated as Ub.
Fig. 20 is a diagram illustrating results of selection of ubiquitin molecules lasso-grafted with aMD4 (Fig. 20a) or aMD5 (Fig. 20b) (the percentage (%) in the recovered library after selection (Population) and K_{D} values measured by SPR). "K_{D} w/mutation" indicates K_{D} values of U-bodies having mutations (mutations relative to wild-type ubiquitin molecules) within the ubiquitin molecules. Flanking spacer sequences (Flanking spacers) are shown for each ubiquitin molecule. The notation regarding amino acid mutations indicates, from the left, the amino acid residue before mutation, the mutation position, and the amino acid residue after mutation.
Fig. 21 is a diagram illustrating an approach for preparing ubiquitin molecules grafted with a MET-binding region, in which 0 to 4 consecutive residues in the β1-2 loop are replaced with pharmacophore sequences flanked upstream and downstream by various flanking spacer sequences (randomized 0 to 3 spacer residues, indicated as X in the drawing).
Fig. 22 is a diagram illustrating an approach for preparing ubiquitin molecules grafted with a MuSK-binding region, in which the 7th to 10th amino acid residues in the β1-2 loop are replaced with a pharmacophore sequence (MuSK7) flanked upstream and downstream by various flanking spacer sequences (randomized 3 or 4 spacer residues, indicated as X in the drawing).
Fig. 23 is a diagram showing results of selection of peptide sequences identified from a RaPID screening conducted to optimize flanking spacer sequences of ubiquitin molecules grafted with the MuSK-binding region shown in Fig. 22. The progress of affinity selection is expressed as the percentage (recovery rate) (%) of the number of mRNAs bound to MuSK-bead complexes (Positive) or the percentage (recovery rate) (%) of the number of mRNAs bound to beads alone (Negative) relative to the total number of mRNAs used in each round of affinity selection.
Fig. 24 is a diagram illustrating evaluation results obtained using a NanoBiT assay, showing dose-response curves for MuSK dimerization of homodimers (Dimeric U-bodies) of the MuSK-binding-region-grafted ubiquitin molecule (Ub012) of the present invention, the cyclic peptide complex (Dimeric peptide/MuSK7-pal), and the ubiquitin molecule dimer (Dimeric Ub).
Fig. 25 is a diagram illustrating test results for evaluation of AChR cluster formation using myotube cells, showing concentration-dependent effects of homodimers (Dimeric U-bodies) of the MuSK-binding-region-grafted ubiquitin molecule (Ub012) of the present invention and the ubiquitin molecule dimer (Dimeric Ub) on induction of acetylcholine receptor clustering.

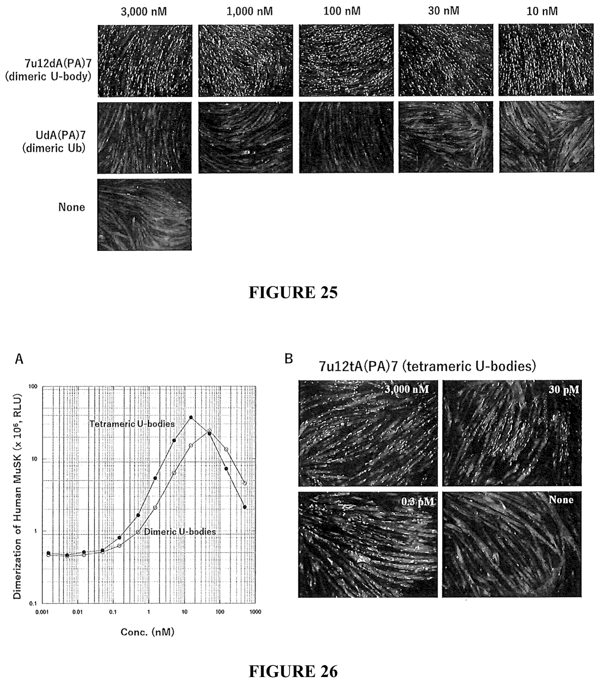

Fig. 26A is a diagram illustrating evaluation results obtained using a NanoBiT assay, showing dose-response curves for MuSK dimerization of homodimers (Dimeric U-bodies) and homotetramers (Tetrameric U-bodies) of the MuSK-binding-region-grafted ubiquitin molecule (Ub012) of the present invention. Fig. 26B is a diagram illustrating test results for evaluation of AChR cluster formation using myotube cells, showing concentration-dependent effects of homotetramers (tetrameric U-bodies) of the MuSK-binding-region-grafted ubiquitin molecule (Ub012) of the present invention on induction of acetylcholine receptor clustering.
Fig. 27A is a diagram illustrating evaluation results (body weight change) of therapeutic effects of homotetramers of the MuSK-binding-region-grafted ubiquitin molecule (Ub012) of the present invention on myasthenia gravis model animals (Example 4(6)). Fig. 27B is a diagram illustrating evaluation results (body weight change) of therapeutic effects of homotetramers of a C-terminal GG deletion variant of Ub012 (Example 4(6)).
Fig. 28A is a diagram illustrating evaluation results (movement score) of therapeutic effects of homotetramers of the MuSK-binding-region-grafted ubiquitin molecule (Ub012) of the present invention on myasthenia gravis model animals (Example 4(6)). Fig. 28B is a diagram illustrating evaluation results (movement score) of therapeutic effects of homotetramers of a C-terminal GG deletion variant of Ub012 (Example 4(6)).
Fig. 29 is a diagram illustrating evaluation results (latency to fall (Latency) in the Hanging Wire Test) of therapeutic effects of homotetramers of the MuSK-binding-region-grafted ubiquitin molecule (Ub012) of the present invention on myasthenia gravis model animals (Example 4(6)).
Fig. 30 is a diagram illustrating evaluation results (grip strength) of therapeutic effects of homotetramers of the MuSK-binding-region-grafted ubiquitin molecule (Ub012) of the present invention on myasthenia gravis model animals (Example 4(6)).

### DETAILED DESCRIPTION OF THE INVENTION

### [Definitions]

In the present specification, the "pharmaceutically acceptable salt" includes, for example, a salt with a pharmaceutically acceptable base or acid. Non-limiting specific examples of pharmaceutically acceptable salts include addition salts of inorganic acids (such as hydrochloric acid, hydrobromic acid, hydroiodic acid, sulfuric acid, and phosphoric acid), addition salts of organic acids (such as p-toluenesulfonic acid, methanesulfonic acid, oxalic acid, p-bromophenylsulfonic acid, carboxylic acid, succinic acid, citric acid, benzoic acid, and acetic acid), addition salts of inorganic bases (such as ammonium hydroxide, alkali or alkaline earth metal hydroxides, carbonates, and bicarbonates), and addition salts of amino acids.

### [Cyclic peptide]

A cyclic peptide of the present invention has, as part of its structure, a unit structure of formula (I). Formula (I) includes the following four structures.
- Thr-Asn-Val-Val- (Ia) (SEQ ID NO: 30)
- Thr-Asn-Ile-Val- (Ib) (SEQ ID NO: 31)
- Val-Asn-Val-Val- (Ic) (SEQ ID NO: 32)
- Val-Asn-Ile-Val- (Id) (SEQ ID NO: 33)

In the present specification, "cyclic peptide" means that the molecule has at least a cyclic structure formed of six or more amino acids. The molecular structure of the cyclic peptide may have, in addition to the cyclic structure, a chain structure in which amino acids are linked by peptide bonds, or may have a structure other than the peptide structure. In the present specification, "cyclic structure" means a closed-ring structure formed in the molecule in a linear peptide by two amino acids separated by four or more amino acid residues being directly bonded or bonded via a linker or the like. In the present specification, "separated by four or more amino acid residues" means that the number of amino acid residues present between the two amino acids forming the closed-ring structure is at least four.

In the specification of the present application, unless otherwise specified, a peptide identified by an amino acid sequence has its N-terminus on the left side and its C-terminus on the right side. In addition, in the specification of the present application, the order or positional relationship of amino acids from the N-terminus toward the C-terminus in a peptide may be referred to as "downstream", and the order or positional relationship of amino acids from the C-terminus toward the N-terminus may be referred to as "upstream".

The closed-ring structure in the cyclic structure is not particularly limited, but is formed by two amino acids covalently bonded, optionally via a linker or the like. Examples of covalent bonds between two amino acids include disulfide bonds, peptide bonds, alkyl bonds, alkenyl bonds, ester bonds, thioester bonds, ether bonds, thioether bonds, phosphonate ether bonds, azo bonds, C-S-C bonds, C-N-C bonds, C=N-C bonds, amide bonds, lactam cross-links, carbamoyl bonds, urea bonds, thiourea bonds, amine bonds, and thioamide bonds. In addition, the covalent bond between two amino acids may be formed by binding between side chains of two amino acids, between main chains of two amino acids, or between a side chain and a main chain of two amino acids. When two amino acids are bound in the main chains of amino acids, a closed-ring structure is formed by a peptide bond.

The cyclic structure may be formed by bonding between a terminal amino acid and a non-terminal amino acid, or by bonding between non-terminal amino acids, without being limited to bonding between the N-terminal and C-terminal amino acids of a linear peptide. When one of the amino acids bonded to form a cyclic structure is a terminal amino acid and the other is a non-terminal amino acid, the cyclic peptide has a structure in which a linear peptide is attached like a tail to the cyclic structure. When the cyclic peptide has a structure in which a linear peptide is attached like a tail to the cyclic structure, it is preferable that the linear peptide be bonded to the C-terminus of an amino acid constituting the cyclic structure in the linear peptide. In other words, it is preferable that the N-terminus of the linear peptide and an amino acid other than the C-terminal amino acid be bonded to form a cyclic structure.

The amino acids forming the cyclic structure include not only natural amino acids but also artificial amino acid variants and/or derivatives, and examples thereof include natural proteinogenic L-amino acids, non-natural amino acids, and chemically synthesized compounds having properties known in the art as characteristics of amino acids.

Proteinogenic amino acids can be identified by the following three-letter or one-letter notations known in the art.
Alanine: Ala A
Arginine: Arg R
Asparagine: Asn N
Aspartic acid: Asp D
Cysteine: Cys C
Glutamine: Gln Q
Glutamic acid: Glu E
Glycine: Gly G
Histidine: His H
Isoleucine: Ile I
Leucine: Leu L
Lysine: Lys K
Methionine: Met M
Phenylalanine: Phe F
Proline: Pro P
Serine: Ser S
Threonine: Thr T
Tryptophan: Trp W
Tyrosine: Tyr Y
Valine: Val V

Non-proteinogenic amino acids mean natural or non-natural amino acids other than proteinogenic amino acids. Examples of non-natural amino acids include amino acids having a main chain structure different from that of natural types (α,α-disubstituted amino acids (such as α-methylalanine), N-alkyl amino acids, D-amino acids, β-amino acids, γ-amino acids, δ-amino acids, α-hydroxy acids, and the like); amino acids having a side chain structure different from that of natural types (norleucine, homohistidine, and the like); amino acids having an extra methylene in the side chain ("homo" amino acids, homophenylalanine, homohistidine, and the like); and amino acids in which a carboxylic acid functional group in the side chain is substituted with a sulfonic acid group (cysteic acid and the like). Specific examples of non-natural amino acids include amino acids described in PCT International Publication No. WO2015/030014.

The number of amino acid residues forming the cyclic structure is not particularly limited as long as it is 6 or more, but the lower limit value (or more) thereof can be, for example, 7, 8, 9, 10, 11, 12, or 13, and the upper limit value (or less) thereof can be 30, 25, 20, 19, 18, 17, 16, or 15. These lower and upper limit values can be arbitrarily combined to define the range of the number of amino acid residues forming the cyclic structure. For example, the range can be 6 or more and 30 or less, 7 or more and 25 or less, 8 or more and 20 or less, 12 or more and 18 or less, or 13 or more and 17 or less.

In the present invention, the cyclic peptide may be modified with one or two or more modifications such as phosphorylation, methylation, acetylation, adenylylation, ADP-ribosylation, glycosylation, addition of polyethylene glycol, addition of fatty acids, and the like, and may be fused with other peptides and/or proteins. In addition, the cyclic peptide may be biotinylated via an appropriate linker.

In addition, in the present invention, the cyclic peptide may be a dimer having two cyclic structures in the molecule, in which two cyclic peptides having one cyclic structure are bonded via a linker structure, or may have an intramolecular lactam bridge structure in which a lactam structure is formed in the molecule. The intramolecular lactam bridge structure may be formed by bonding side chains of amino acids constituting the cyclic peptide, and for example, an amino group of a side chain of Lys and a carboxyl group of a side chain of Asp or Glu may be bonded to form a peptide bond, thereby forming an intramolecular lactam structure. Such cyclic peptides have another cyclic structure as a bridge structure in the molecule. Instead of Lys, for example, DAP, DAB, or Orn may be bonded to Asp or Glu. In the present specification, a dimer of a cyclic peptide in which two cyclic peptides including a cyclic structure having a structure represented by the above formula (I) are bonded via a linker structure is referred to as a "cyclic peptide complex" as described below.

Preferred examples of the cyclic peptide of the present invention include a cyclic peptide having a structure of the following formula (II):

(Z₁)m-X₁-Asn-X₂-Val-(Z₂)n (II)

wherein, X₁ and X₂ have the same meaning as defined in formula (I), Z₁ represents arbitrary amino acid, Z₂ represents arbitrary amino acid, m is an integer of 1 or more, and n is an integer of 1 or more.

The above formula (II) is a structure in which (Z₁)m is bonded to the N-terminal side of the structure represented by formula (I) and (Z₂)n is bonded to the C-terminal side. In formula (II), the amino acids constituting (Z₁)m and (Z₂)n each independently represent an arbitrary amino acid. It is preferable that one amino acid of (Z₁)m and one amino acid of (Z₂)n be bonded to each other directly or via a linker or the like to form a cyclic structure. This cyclic structure may be formed by bonding N-terminal Z₁ to C-terminal Z₂, may be formed by bonding N-terminal Z₁ to non-C-terminal Z₂, may be formed by bonding non-N-terminal Z₁ to C-terminal Z₂, or may be formed by bonding non-N-terminal Z₁ to non-C-terminal Z₂.

When the non-terminal Z₁ or Z₂ is involved in bonding, the cyclic peptide has a structure in which a linear peptide is attached like a tail to a cyclic structure. In the present invention, preferably, the N-terminal Z₁ may be bonded to the non-C-terminal Z₂ to form a cyclic structure. In addition, Z₁ and Z₂ may form a cyclic structure via a linker.

Regarding m and n, the total value (m+n) is an integer of 2 or more, but the lower limit value (or more) of m+n can be, for example, 2, 3, 4, 5, 6, 7, 8, or 9, and the upper limit value (or less) can be 26, 21, 16, 15, 14, 13, 12, or 11. These lower and upper limit values can be arbitrarily combined to define the numerical range of m+n, and can be, for example, 2 to 26, 3 to 24, 4 to 16, 8 to 14, or 9 to 13.

(Z₁)m and (Z₂)n may contain an amino acid having a branched side chain, such as Leu, Val, or Ile, an amino acid having a hydroxyl group in a side chain, such as Ser or Thr, a basic amino acid, or an aromatic amino acid, in addition to an amino acid involved in formation of a cyclic structure, and preferably contain Leu or Ser.

The cyclic peptide preferably has an N-CO-CH₂-S structure (that is, -NH-CO-CH₂-S- structure), and may form a cyclic structure by the N-CO-CH₂-S structure. The cyclic structure is preferably formed by bonding between an acetyl group, in which a hydrogen atom bonded to the amino group at the N-terminus of the peptide before forming the cyclic structure is substituted with a leaving group L, and a thiol group in cysteine (Cys) on the C-terminal side. Examples of the leaving group L include halogen atoms such as a chlorine atom, a bromine atom, a fluorine atom, and an iodine atom. The acetyl group in which a hydrogen atom is substituted with the leaving group L is not particularly limited, and examples thereof include a chloroacetyl group. Examples of the cyclic peptide having a structure of formula (II), in which a cyclic structure is formed by an N-CO-CH₂-S structure, include a case in which a cyclic structure is formed by bonding LCH₂CO- and a thiol group (HS-) of Cys in a peptide represented by the following formula (III):

LCH₂CO-(Z₃)-(Z₄)p-X₁-Asn-X₂-Val-(Z₅)q-Cys-(Z₆)r (III)

wherein, X₁ and X₂ have the same meaning as defined in formula (I), Z₃, Z₄, Z₅ and Z₆ represent an arbitrary amino acid, p is an integer of m-1, q is an integer of n-1, and r represents 0 or an integer of 1 or more. m and n have the same meaning as defined in formula (II).

In formula (III), the amino acids constituting Z₃, (Z₄)p, (Z₅)q, and (Z₆)r each independently represent an arbitrary amino acid. LCH₂CO- and the thiol group (HS-) of Cys are bonded to form a cyclic structure of the peptide of formula (III).

Regarding p and q, the total value (p+q) is an integer of 0 or more, but the lower limit value (or more) of p+q can be, for example, 0, 1, 2, 3, 4, 5, 6, or 7, and the upper limit value (or less) can be 24, 19, 14, 13, 12, 11, 10, or 9. These lower and upper limit values can be arbitrarily combined to define the numerical range of p+q, and can be, for example, 0 to 24, 1 to 22, 2 to 14, 6 to 12, or 7 to 11.

r is not particularly limited as long as it is an integer of 0 or more, but the lower limit value (or more) thereof can be, for example, 1, 2, 3, 4, 5, or 6, and the upper limit value (or less) thereof can be 20, 15, 14, 13, 12, 11, or 10. These lower and upper limit values can be arbitrarily combined to define the numerical range of r, and can be, for example, 0 to 20 or 1 to 15. In the present specification, when r is 0, the cyclic peptide does not have (Z₆)r, and when r is an integer of 1 or more, (R₆)r in the cyclic peptide corresponds to a chain structure in which amino acids are linked by a peptide bond.

Z₃ is preferably Tyr, and may be a D-form or an L-form.

(Z₄)p and (Z₅)q may contain an amino acid having a branched side chain, such as Leu, Val, or Ile, an amino acid having a hydroxyl group in a side chain, such as Ser or Thr, a basic amino acid, or an aromatic amino acid, and preferably contain Leu or Ser.

Z₆ is preferably Gly or Ser independently. Examples of (Z₆)r include a structure represented by (-Gly-Ser-)ra (ra is an integer of 0 to 10, and when ra is 2 or more, it represents a repeating structure consisting of -Gly-Ser-, provided that the C-terminus is -Ser).

The cyclic structure in the cyclic peptide may be a structure other than the cyclic structure formed by the N-CO-CH₂-S structure. For example, a cyclic peptide in which an amino acid having functional group 1 shown in Table 1 below and an amino acid having corresponding functional group 2 are cyclized can be used. In this aspect, for example, when the structure represented by the above formula (II) is included as a cyclic structure, at least one Z₁ may be an amino acid having functional group 1 and at least one Z₂ may be an amino acid having corresponding functional group 2, or at least one Z₁ may be an amino acid having functional group 2 and at least one Z₂ may be an amino acid having corresponding functional group 1.

Functional group 1 and functional group 2 may be disposed at the N-terminus and the C-terminus (either may be on the N-terminal side), one may be disposed at a terminal amino acid and the other may be disposed at a non-terminal amino acid, or both may be disposed at non-terminal amino acids. The bond formed by functional group 1 and functional group 2 is a chemical cross-linking structure for forming a molecular cyclic structure in the cyclic peptide.

**[Table 1]**

| | **Functional group 1** | **Functional group 2** |
|---|---|---|
| (A) | 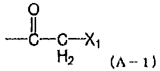 | HS- (A - 2) |
| (B) | -C≡C-H (B - 1) | N₃- (B - 2) |
| (C) | -Ar-CH₂NH₂ (C - 1) | 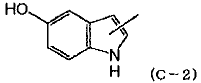 |
| (D) | -C≡C-CH₂-X₁ (D - 1) | HS- (D -2) |
| (E) | -Ar-CH₂-X₁ (E - 1) | HS- (E - 2) |

In the chemical structural formulas of Table 1, X₁ is a leaving group, and Ar is an aromatic ring optionally having a substituent. Examples of the leaving group include halogen atoms such as a chlorine atom, a bromine atom, a fluorine atom, and an iodine atom.

As the amino acid having functional group (A-1), for example, a chloroacetylated amino acid can be used. Examples of the chloroacetylated amino acid include N-chloroacetyl-L-alanine, N-chloroacetyl-L-phenylalanine, N-chloroacetyl-L-tyrosine, N-chloroacetyl-L-tryptophan, N-3-(2-chloroacetamido)benzoyl-L-phenylalanine, N-3-(2-chloroacetamido)benzoyl-L-tyrosine, and N-3-(2-chloroacetamido)benzoyl-L-tryptophan, β-N-chloroacetyl-L-diaminopropanoic acid, γ-N-chloroacetyl-L-diaminobutyric acid, δ-N-chloroacetyl-L-ornithine, ε-N-chloroacetyl-L-lysine, and D-amino acid derivatives corresponding thereto. As the amino acid having functional group (A-1), N-chloroacetyl-L-tyrosine and N-chloroacetyl-D-tyrosine are suitably used.

Examples of the amino acid having functional group (A-2) include cysteine, homocysteine, mercaptonorvaline, mercaptonorleucine, 2-amino-7-mercaptoheptanoic acid, and 2-amino-8-mercaptooctanoic acid. As the amino acid having functional group (A-2), cysteine is suitably used.

Examples of cyclization methods with an amino acid having functional group (A-1) and an amino acid having functional group (A-2) include methods described in Kawakami, T. et al., Nature Chemical Biology 5, 888-890 (2009); Yamagishi, Y. et al., ChemBioChem 10, 1469-1472 (2009); Sako, Y. et al., Journal of American Chemical Society 130, 7932-7934 (2008); Goto, Y. et al., ACS Chemical Biology 3, 120-129 (2008); Kawakami, T. et al., Chemistry & Biology 15, 32-42 (2008); PCT International Publication No. WO2008/117833; and the like.

Examples of the amino acid having functional group (B-1) include propargylglycine, homopropargylglycine, 2-amino-6-heptynoic acid, 2-amino-7-octynoic acid, and 2-amino-8-nonynoic acid. 4-pentynoylated or 5-hexynoylated amino acids may be used. Examples of 4-pentynoylated amino acids include N-(4-pentenoyl)-L-alanine, N-(4-pentenoyl)-L-phenylalanine, N-(4-pentenoyl)-L-tyrosine, N-(4-pentenoyl)-L-tryptophan, N-3-(4-pentynoylamido)benzoyl-L-phenylalanine, N-3-(4-pentynoylamido)benzoyl-L-tyrosine, N-3-(4-pentynoylamido)benzoyl-L-tryptophan, β-N-(4-pentenoyl)-L-diaminopropanoic acid, γ-N-(4-pentenoyl)-L-diaminobutyric acid, σ-N-(4-pentenoyl)-L-ornithine, ε-N-(4-pentenoyl)-L-lysine, and D-amino acid derivatives corresponding thereto. Examples of 5-hexynoylated amino acids include an amino acid in which the 4-pentynoyl group is substituted with a 5-hexynoyl group in the compounds exemplified as the 4-pentynoylated amino acids.

Examples of the amino acid having functional group (B-2) include azidoalanine, 2-amino-4-azidobutanoic acid, azidoptonorvaline, azidonorleucine, 2-amino-7-azidoheptanoic acid, and 2-amino-8-azidooctanoic acid. An azidoacetylated or 3-azidopentanoylated amino acid can also be used. Examples of azidoacetylated amino acids include N-azidoacetyl-L-alanine, N-azidoacetyl-L-phenylalanine, N-azidoacetyl-L-tyrosine, N-azidoacetyl-L-tryptophan, N-3-(4-pentynoylamido)benzoyl-L-phenylalanine, N-3-(4-pentynoylamido)benzoyl-L-tyrosine, N-3-(4-pentynoylamido)benzoyl-L-tryptophan, β-N-azidoacetyl-L-diaminopropanoic acid, γ-N-azidoacetyl-L-diaminobutyric acid, σ-N-azidoacetyl-L-ornithine, ε-N-azidoacetyl-L-lysine, and D-amino acid derivatives corresponding thereto. Examples of 3-azidopentanoylated amino acid include an amino acid in which the azidoacetyl group is substituted with a 3-azidopentanoyl group in the compounds exemplified as the azidoacetylated amino acids.

Examples of cyclization methods with an amino acid having functional group (B-1) and an amino acid having functional group (B-2) include methods described in Sako, Y. et al., Journal of American Chemical Society 130, 7932-7934 (2008); PCT International Publication No. WO2008/117833; and the like.

Examples of the amino acid having functional group (C-1) include N-(4-aminomethyl-benzoyl)-phenylalanine (AMBF) and 3-aminomethyltyrosine.

Examples of the amino acid having functional group (C-2) include 5-hydroxytryptophan (WOH).

Examples of cyclization methods with an amino acid having functional group (C-1) and an amino acid having functional group (C-2) include methods described in Yamagishi, Y. et al., ChemBioChem 10, 1469-1472 (2009) and PCT International Publication No. WO2008/117833.

Examples of the amino acid having functional group (D-1) include 2-amino-6-chloro-hexynoic acid, 2-amino-7-chloro-heptynoic acid, and 2-amino-8-chloro-octynoic acid.

Examples of the amino acid having functional group (D-2) include cysteine, homocysteine, mercaptonorvaline, mercaptonorleucine, 2-amino-7-mercaptoheptanoic acid, and 2-amino-8-mercaptooctanoic acid.

Examples of cyclization methods with an amino acid having functional group (D-1) and an amino acid having functional group (D-2) include a method described in PCT International Publication No. WO2012/074129.

Examples of the amino acid of (E-1) include N-3-chloromethylbenzoyl-L-phenylalanine, N-3-chloromethylbenzoyl-L-tyrosine, N-3-chloromethylbenzoyl-L-tryptophan, and D-amino acid derivatives corresponding thereto.

Examples of the amino acid of (E-2) include cysteine, homocysteine, mercaptonorvaline, mercaptonorleucine, 2-amino-7-mercaptoheptanoic acid, and 2-amino-8-mercaptooctanoic acid.

Cyclization methods with an amino acid having functional group (E-1) and an amino acid having functional group (E-2) can be performed with reference to, for example, the cyclization methods for (A-1) and (A-2) and the cyclization methods for (D-1) and (D-2).

More preferred examples of the cyclic peptide of the present invention include a cyclic peptide having a structure of the following formula (IV): 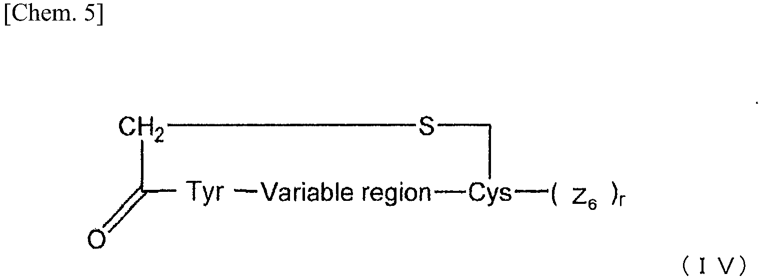 wherein, Tyr may be a D-form or an L-form, preferably a D-form, S means a thiol group of Cys, the moiety referred to as "Variable region" is a peptide chain containing at least the unit structure of Formula (I) (the amino acid length can be 4 to 28, 5 to 26, 6 to 18, 10 to 16, or 11 to 15), and may be a peptide chain corresponding to -(Z₄)p-X₁-Asn-X₂-Val-(Z₅)q- of formula (III), and (Z₆)r has the same meaning as defined in formula (III).

In the present specification and the drawings, the structure represented by - CH₂-C(=O)- in the above structure may be denoted as "Ac".

In the above formula (IV), "Variable region" can be represented by the following formula (V):

-(Z₄)p-X₁-Asn-X₂-Val-(Z₅)q- (V)

wherein, X₁ and X₂ have the same meaning as defined in formula (I), Z₄ and Z₅ and p and q have the same meaning as defined in formula (III), and the specific description and the preferred aspect are as described in formula (III).

Preferred specific examples of the cyclic peptide represented by the above formula (IV) include those having any of the structures represented by the following formula (1) (MuSK7/SEQ ID NO: 2), formula (2) (MuSK4/SEQ ID NO: 3), and formula (3) (MuSK9/SEQ ID NO: 4). However, in the following formulas (1) to (3), the linear structural part corresponding to (Z₆)r bonded to Cys of the above formula (IV) is omitted. Therefore, in the following formulas (1) to (3), (Z₆)r may be bonded to C (Cys) bonded to -SCH₂- (Z₆ and r have the same meaning as defined in formula (III)).

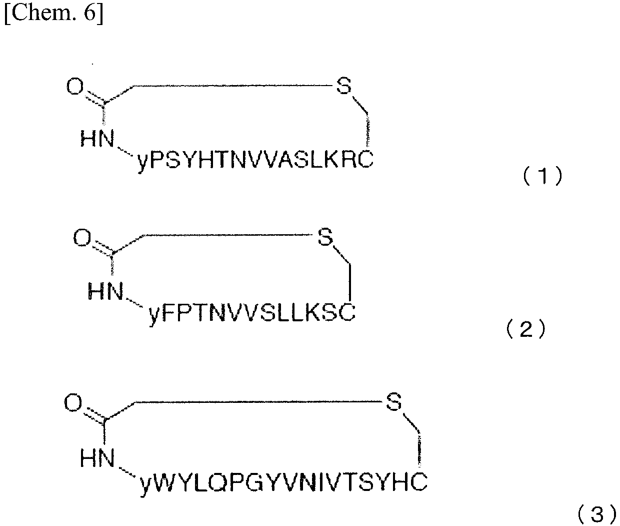

In addition, in the above formulas (1) to (3), a carboxyl group of C (Cys) (-C(=O)-OH) may form an acid amide (-C(=O)-NH₂). Such compounds are the following formulas (1a), (2a) and (3a).

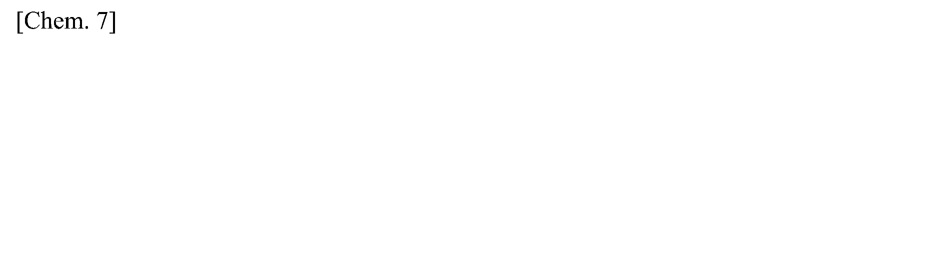

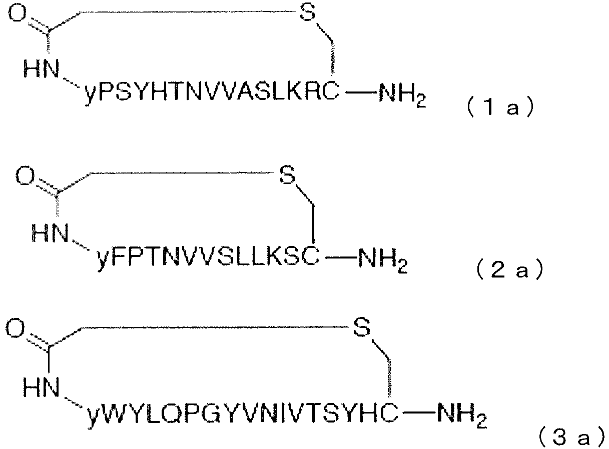

The cyclic peptide in the present invention has high affinity with MuSK, can strongly bind to MuSK, and can be used as a MuSK agonist. In addition, as described below, the complex containing the cyclic peptide of the present invention binds to the extracellular domain of MuSK, thereby causing homodimerization of MuSK, thereby selectively inducing downstream MuSK signaling, and thus can activate nerve-to-muscle signaling at the neuromuscular junction. For this reason, the cyclic peptide of the present invention is advantageous in that it can provide a therapeutic method and a research method different from the existing methods since it can strongly activate signal transmission at the neuromuscular junction unlike the approach targeting the anti-acetylcholine receptor autoantibody which has been conventionally studied. That is, the cyclic peptide of the present invention and a pharmaceutically acceptable salt thereof are useful as medicaments and research reagents, and according to the present invention, a pharmaceutical composition containing the cyclic peptide of the present invention and a pharmaceutically acceptable salt thereof and use of the cyclic peptide of the present invention and a pharmaceutically acceptable salt thereof as a pharmaceutical are provided.

According to another aspect of the present invention, a peptide having a structure of the following formula (I) or a pharmaceutically acceptable salt thereof is provided.

-X₁-Asn-X₂-Val- (I)

wherein, X₁ represents Thr or Val, and X₂ represents Val or Ile.

The lower limit value (or more) of the number of amino acid residues of the peptide of the present invention described above can be, for example, 7, 8, 9, 10, 11, 12, or 13, and the upper limit value (or less) thereof can be 30, 25, 20, 19, 18, 17, 16, or 15. These lower and upper limit values can be arbitrarily combined to define the range of the number of amino acid residues forming the above peptide. For example, the range can be 6 or more and 30 or less, 7 or more and 25 or less, 8 or more and 20 or less, 12 or more and 18 or less, or 13 or more and 17 or less.

The peptide of the present invention above can be a peptide having the same amino acid sequence as or a part of the amino acid sequence of the cyclic peptide of the present invention, and can be preferably a peptide containing an amino acid sequence constituting a region which is present in the cyclic peptide of the present invention and has MuSK molecule-binding activity (that is, the MuSK-binding region), and more preferably a peptide containing an amino acid sequence of YX₂₂SYHTNVVASLKR (X₂₂ is P or L) (SEQ ID NO: 11). The peptide of the present invention above can also be an acyclic peptide.

The peptide of the present invention above can be used by being grafted onto a desired biomolecule, like a MuSK-binding-region-grafted ubiquitin molecule described below. Examples of biomolecules to be grafted include bioproteins such as ubiquitin molecules. The peptide of the present invention above can be a peptide having binding properties to a desired molecule, and can be grafted onto a biomolecule so that the binding properties by the peptide are exhibited. When the peptide of the present invention above is a peptide containing an amino acid sequence constituting a region which is present in the cyclic peptide of the present invention and has MuSK molecule-binding activity, the peptide of the present invention can be grafted onto a biomolecule so that the MuSK-binding property by the peptide is exhibited. The peptide of the present invention above grafted onto a biomolecule is within the scope of the present invention as long as the binding property of the peptide to a desired molecule is exhibited. For grafting onto a biomolecule, for example, as in the ubiquitin molecule described below, the peptide of the present invention above can be grafted onto a loop structure region between two consecutive domain structures in a protein.

### [Method for producing cyclic peptide]

The cyclic peptide of the present invention can be suitably produced by a translation synthesis method using a cell-free translation system. Specifically, it can be prepared by preparing a nucleic acid encoding a cyclic peptide and translating the nucleic acid in a cell-free translation system. The nucleic acid encoding the cyclic peptide can be appropriately designed by those skilled in the art using a genetic code used in a translation system of a living body, a reprogrammed genetic code, or a combination thereof. The nucleic acid may be DNA or RNA.

According to the method using a cell-free translation system, tRNAs aminoacylated with non-natural amino acids can be used to efficiently introduce the non-natural amino acids into a peptide in addition to natural amino acids. For example, using an artificial aminoacyl-tRNA synthetase, Flexizyme, developed by the present inventors, it is possible to aminoacylate tRNAs having arbitrary anticodons with arbitrary natural or non-natural amino acids. Accordingly, by using this technique, the genetic code consisting of triplets of mRNA can be reprogrammed so as to code for amino acids different from those in the biological translation system (see, for example, PCT International Publication No. WO2008/059823).

### [Cyclic peptide complex]

A cyclic peptide complex of the present invention includes two cyclic peptides of the present invention and has a structure in which one cyclic peptide is bonded to the other cyclic peptide via a linker.

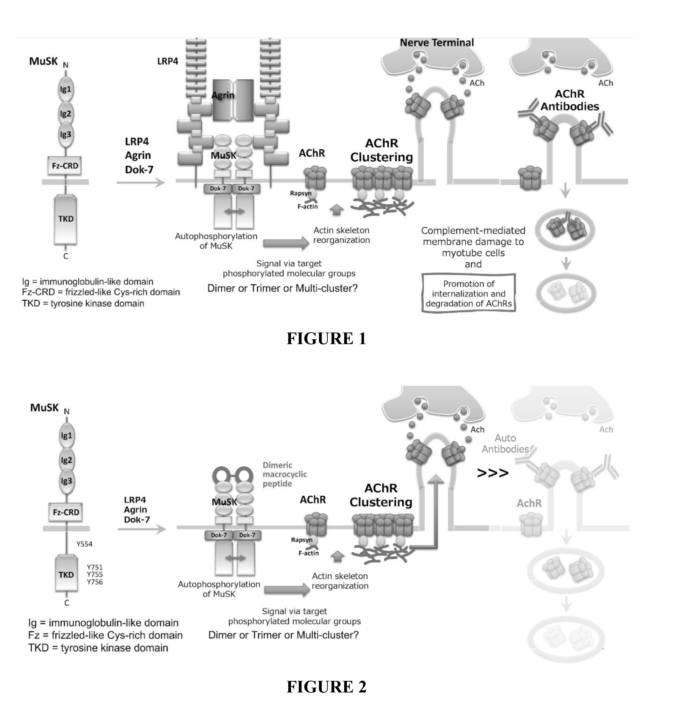

As shown in Fig. 1, Agrin binds to the first β-propeller structure of its receptor LRP4. Since MuSK binds to another propeller structure of LRP4 at its extracellular domain (the Ig1 domain, which is an immunoglobulin-like domain), it is considered that binding of Agrin to LRP4 results in the formation of an Agrin-LRP4-MuSK complex and induces homodimerization of MuSK. In addition, it is known that, upon formation of this complex and the action of an intracellular factor Dok-7, the intracellular kinase domain of MuSK shifts to an active state and activates downstream signaling through autophosphorylation of MuSK (Zong Y. et al., GENES & DEVELOPMENT, 2012; 26:247-258). In addition, it is known that activation of downstream signaling of MuSK leads to the formation of clusters of acetylcholine receptors at postsynaptic sites (Xing G. et al., Neurosci Lett, 2020 Jul 13;731:135013). Since the cyclic peptide complex of the present invention has a configuration including two cyclic peptides having a MuSK-binding region, each cyclic peptide in the cyclic peptide complex is considered to be able to bind to the extracellular domain of MuSK and induce homodimerization of MuSK (Fig. 2). Accordingly, the cyclic peptide complex of the present invention is considered to be able to activate downstream signaling through autophosphorylation of MuSK and induce clustering of acetylcholine receptors. As a result, the cyclic peptide complex of the present invention is considered to be able to activate nerve-to-muscle signaling at the neuromuscular junction. Therefore, it can be said that the cyclic peptide complex of the present invention is useful as a therapeutic agent for myasthenia gravis caused by abnormal signal transduction at the neuromuscular junction. That is, the cyclic peptide complex of the present invention and a pharmaceutically acceptable salt thereof are useful as medicaments, and according to the present invention, a pharmaceutical composition containing the cyclic peptide complex of the present invention and a pharmaceutically acceptable salt thereof and use of the cyclic peptide complex of the present invention and a pharmaceutically acceptable salt thereof as a pharmaceutical are provided.

The cyclic peptide complex of the present invention can exert the above action as long as it includes two cyclic peptides of the present invention in which one cyclic peptide is bonded to the other cyclic peptide via a linker, and the two cyclic peptides may be identical or different. From the viewpoint of further promoting homodimerization of MuSK, it is preferable that the two cyclic peptides in the peptide complex be identical.

The linker connecting the two cyclic peptides in the cyclic peptide complex is not particularly limited, and structures known in the field of peptide synthesis as linkers for connecting peptides to each other can be adopted. From the viewpoint of further promoting homodimerization of MuSK, the length of the linker is preferably 20Å to 100Å.

Non-limiting examples of the linker connecting the two cyclic peptides in the peptide complex include polymers such as polyethylene glycol (PEG), peptides, nucleic acids, saccharides, and combinations thereof. Examples of linkers containing PEG include those in which two PEG are bonded to each of small molecules having two or more functional groups, and non-limiting examples thereof include those in which carboxylic acid-modified PEG is bonded to an amino group of 2,3-diaminopropionic acid. Other examples of linkers containing PEG include PEG having two identical or different functional groups at both ends, and non-limiting examples thereof include PEG having two maleimide groups at both ends. A linker containing PEG can be specified as one including an ethylene glycol structure (-CH₂O-), and the number of polymerizations of ethylene glycol may be 2 to 20 or 5 to 15.

From the viewpoint of further improving blood stability, the peptide complex of the present invention may have at least one saturated hydrocarbon group having 8 to 20 carbon atoms (preferably 12 to 18 or 14 to 18 carbon atoms) as a side chain. This saturated hydrocarbon group can preferably be introduced into the linker. For example, when the linker is a linker in which two PEGs are bonded to a small molecule having two or more functional groups, a structure in which a saturated fatty acid is bonded directly or indirectly to the third functional group of the small molecule is exemplified, and non-limiting examples thereof include a linker in which carboxylic acid-modified PEG is bonded to an amino group of 2,3-diaminopropionic acid and a saturated fatty acid having 8 to 20 carbon atoms (preferably 12 to 18 or 14 to 18 carbon atoms) is indirectly bonded to a carboxyl group of the 2,3-diaminopropionic acid. Non-limiting examples of the saturated fatty acid having 8 to 20 carbon atoms include palmitic acid (16 carbon atoms) and myristic acid (14 carbon atoms).

### [Method for producing cyclic peptide complex]

The cyclic peptide complex of the present invention can be produced by linking the cyclic peptides of the present invention with an appropriate linker. The step of linking the cyclic peptides of the present invention with a linker can be carried out by a person skilled in the art using a known method or an equivalent method for linking a linker and a peptide, depending on the type of linker used. For example, when the linker is a linker in which two PEGs are respectively bonded to a small molecule having two or more functional groups, a peptide complex can be prepared by preliminarily introducing amino groups into respective terminals of the two PEGs and linking the amino groups to carboxyl groups at the C-termini of the cyclic peptides of the present invention by amide bonds. In addition, when the linker is PEG having two identical or different functional groups (for example, maleimide groups) at both ends, a peptide complex can be prepared by chemically bonding the two functional groups to functional groups (for example, thiol groups of cysteine) at the C-termini of the cyclic peptides of the present invention. Furthermore, when the linker is a peptide, a peptide complex can also be prepared by linking nucleic acids encoding the two cyclic peptides of the present invention and nucleic acids encoding the linker peptide, and expressing a fusion protein of the cyclic peptides of the present invention and the linker peptide.

### [MuSK-binding-region-grafted ubiquitin molecule]

The MuSK-binding-region-grafted ubiquitin molecule of the present invention is one in which a part or all of a loop structure region between a β1 domain and a β2 domain of the ubiquitin molecule is replaced with a peptide chain containing a MuSK-binding region. The MuSK-binding-region-grafted ubiquitin molecule of the present invention is also one in which a peptide chain containing a MuSK-binding region is inserted into the loop structure region between the β1 domain and the β2 domain of a ubiquitin molecule. In the present invention, a ubiquitin molecule in which the entire loop structure region between the β1 domain and the β2 domain of the ubiquitin molecule is replaced with a peptide of structure (A) is preferable.

Here, ubiquitin is a protein universally present in eukaryotes, and is known to be highly evolutionarily conserved and thermally stable. Ubiquitin also includes two α-helices and five β-sheets, specifically having α-helices and β-sheets in the order of β1 (β-sheet 1), β2 (β-sheet 2), α1 (α-helix 1), β3 (β-sheet 3), β4 (β-sheet 4), α2 (α-helix 2), and β5 (β-sheet 5) from the N-terminus to the C-terminus. The regions connecting between β-sheets and between an α-helix and a β-sheet are called loops, and the ubiquitin molecule of the present invention is characterized in that a peptide chain containing a MuSK-binding region is grafted onto the loop structure region between β1 and β2.

The ubiquitin molecule to be grafted (that is, the ubiquitin molecule serving as a scaffold) can be, for example, a protein consisting of the amino acid sequence of SEQ ID NO: 1 (a human ubiquitin molecule). β1 corresponds to the region from the 1st to the 6th of the amino acid sequence of SEQ ID NO: 1, the β1-β2 loop corresponds to the region from the 7th to the 10th of the amino acid sequence of SEQ ID NO: 1, and β2 corresponds to the region from the 11th to the 17th of the amino acid sequence of SEQ ID NO: 1. Hereinafter, the ubiquitin molecule of the present invention will be described taking as an example the case where the ubiquitin molecule to be grafted is the amino acid sequence of SEQ ID NO: 1, but the ubiquitin molecule of the present invention is not limited thereto.

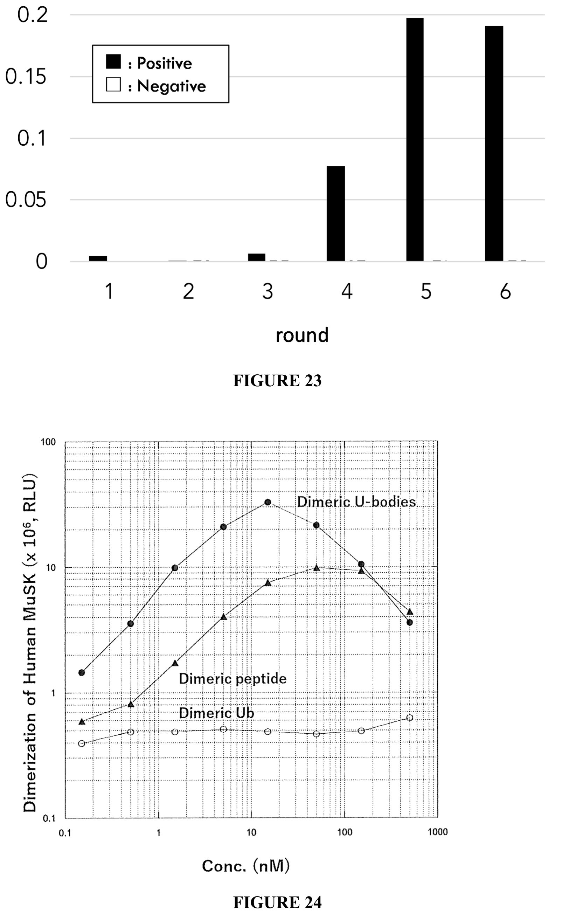

In the case where the ubiquitin molecule to be grafted is the amino acid sequence of SEQ ID NO: 1, the phrase "the entire loop structure region between the β1 domain and the β2 domain of the ubiquitin molecule is replaced with a peptide of structure (A)" means that the ubiquitin molecule has a structure in which the region from the 7th to the 10th of the amino acid sequence of SEQ ID NO: 1 is replaced with the peptide of the structure (A). In this case, in the ubiquitin molecule of the present invention, the peptide of structure (A) is present downstream of the 6th amino acid and upstream of the 11th amino acid in the amino acid sequence of SEQ ID NO: 1 (see Fig. 23).

In the case where the ubiquitin molecule to be grafted is the amino acid sequence of SEQ ID NO: 1, the phrase "a part of the loop structure region between the β1 domain and the β2 domain of the ubiquitin molecule is replaced with a peptide of structure (A)" means that the ubiquitin molecule has a structure in which any one of the 7th amino acid, the 8th amino acid, the 9th amino acid, the 10th amino acid, the region from the 7th to the 8th, the region from the 8th to the 9th, the region from the 9th to the 10th, the region from the 7th to the 9th, or the region from the 8th to the 10th of the amino acid sequence of SEQ ID NO: 1 is replaced with the peptide of structure (A). For example, when the region from the 8th to the 9th amino acid of the amino acid sequence of SEQ ID NO: 1 is replaced with the peptide of structure (A), the ubiquitin molecule of the present invention has the peptide of structure (A) located downstream of the 7th amino acid and upstream of the 10th amino acid in the amino acid sequence of SEQ ID NO: 1.

In the case where the ubiquitin molecule to be grafted is the amino acid sequence of SEQ ID NO: 1, the phrase "the peptide of structure (A) is inserted into the loop structure region between the β1 domain and the β2 domain of the ubiquitin molecule" means that the ubiquitin molecule has a structure in which the peptide of the structure (A) is inserted at any position between the 6th and the 7th amino acids, between the 7th and the 8th amino acids, between the 8th and the 9th amino acids, between the 9th and the 10th amino acids, and between the 10th and the 11th amino acids of the amino acid sequence of SEQ ID NO: 1. For example, when the peptide of structure (A) is inserted between the 8th and the 9th amino acids of the amino acid sequence of SEQ ID NO: 1, the ubiquitin molecule of the present invention has the peptide of structure (A) located downstream of the 8th amino acid and upstream of the 9th amino acid in the amino acid sequence of SEQ ID NO: 1.

The ubiquitin molecule to be grafted may be a protein consisting of an amino acid sequence other than the amino acid sequence of SEQ ID NO: 1, as long as the binding activity to MuSK is not impaired. In the present invention, for example, a protein consisting of an amino acid sequence having 90% or more sequence identity (preferably 91% or more, 92% or more, 93% or more, 94% or more, 95% or more, 96% or more, 97% or more, or 98% or more) with the amino acid sequence of SEQ ID NO: 1 can be the ubiquitin molecule to be grafted. Here, the term "identity" refers to the degree of identity when the sequences to be compared are appropriately aligned, and means the percentage (%) of occurrence of exact matches of amino acids between the sequences. In calculating identity, for example, the presence of gaps in the sequence and the properties of amino acids are taken into account (Wilbur, Natl. Acad. Sci. U.S.A. 80:726-730 (1983)). The alignment can be performed by using an arbitrary algorithm, and specifically, publicly available homology search software such as Basic Local Alignment Search Tool (BLAST) (Altschul et al., J. Mol. Biol. 215:403-410 (1990)), FASTA (Peasron et al., Methods in Enzymology 183:63-69 (1990)), or Smith-Waterman (Meth. Enzym., 164, 765 (1988)) can be used. In addition, the calculation of identity can be carried out by using publicly available homology search programs as described above, for example, by using default parameters in the homology algorithm BLAST of the National Center for Biotechnology Information (NCBI) (https://blast.ncbi.nlm.nih.gov/Blast.cgi).

In the present invention, a protein having a modification selected from the group consisting of deletion, substitution, insertion, and addition with respect to one or a plurality of amino acids in the amino acid sequence of SEQ ID NO: 1 can be used as a ubiquitin molecule to be grafted. The number of amino acids to be modified can be a number of mutations as occurs by known methods such as a site-directed mutagenesis method, or a number of mutations as occurs naturally. The number of amino acids to be modified may also be, for example, 1 to 8, 1 to 7, 1 to 6, 1 to 5, 1 to 4, 1 to 3, 1 to 2, or 1. When there are multiple modifications, the types of modifications may be the same or different. That is, the modifications may be a plurality of modifications of the same type (for example, a plurality of substitutions), or a plurality of modifications of different types (for example, a combination of one or more deletions and one or more substitutions).

The amino acid modification in the amino acid sequence of SEQ ID NO: 1 may be a conservative modification (for example, a conservative mutation). The term "conservative modification" or "conservative mutation" means modifying or mutating one or a plurality of amino acids so as not to substantially modify the function of the protein. The substitution of the amino acid may also be a conservative substitution. The term "conservative substitution" means substituting one or a plurality of amino acids with other amino acids and/or amino acid derivatives so as not to substantially modify the function of the protein. In the conservative substitution, it is preferable that the amino acid to be substituted and the substituted amino acid have, for example, similar properties and/or functions, such as chemical properties including hydrophobicity and hydrophilicity indexes, polarity, and charge, or physical properties such as secondary structure. In this manner, amino acids or amino acid derivatives having similar properties and/or functions are known in the technical field. Non-limiting examples are as follows. Examples of non-polar amino acids (hydrophobic amino acids) include alanine, valine, isoleucine, leucine, proline, tryptophan, phenylalanine, and methionine. Examples of polar amino acids (neutral amino acids) include glycine, serine, threonine, tyrosine, glutamine, asparagine, and cysteine. Examples of amino acids having a positive charge (basic amino acids) include arginine, histidine, and lysine, and examples of amino acids having a negative charge (acidic amino acids) include aspartic acid and glutamic acid.

The modification of amino acids (preferably substitution, more preferably conservative modification or conservative substitution) in the amino acid sequence of SEQ ID NO: 1 may also be present in a region other than the β1-β2 loop (the region from the 7th to the 10th of the amino acid sequence of SEQ ID NO: 1) (that is, in the region from the 1st to the 6th and the region from the 11th onward of the amino acid sequence of SEQ ID NO: 1). Preferably, the modification may be present in a region other than β1 (the region from the 1st to the 6th of the amino acid sequence of SEQ ID NO: 1) and the β1-β2 loop (the region from the 7th to the 10th of the amino acid sequence of SEQ ID NO: 1) (that is, in the region from the 11th onward of the amino acid sequence of SEQ ID NO: 1). More preferably, the modification may be present in a region other than β1 (the region from the 1st to the 6th of the amino acid sequence of SEQ ID NO: 1), the β1-β2 loop (the region from the 7th to the 10th of the amino acid sequence of SEQ ID NO: 1), and β2 (the region from the 11th to the 17th of the amino acid sequence of SEQ ID NO: 1) (that is, in the region from the 18th onward of the amino acid sequence of SEQ ID NO: 1).

Non-limiting examples of positions of amino acid modification in the amino acid sequence of SEQ ID NO: 1 include the 11th (K), the 12th (T), the 37th (P), the 54th (R), the 57th (S), the 62nd (Q), and the 65th (S), and the 11th (K), the 54th (R), and the 57th (S) are preferable. The amino acid sequence of SEQ ID NO: 1 may have a modification (preferably a substitution mutation) at one, two, or three of the above modification positions.

Non-limiting examples of amino acid modifications in the amino acid sequence of SEQ ID NO: 1 include substitution mutations of K11M, K11E, K11T, T12L, T12K, P37R, R54S, R54G, S57T, Q62K, and S65T, and the amino acid sequence of SEQ ID NO: 1 may have one, two, or three of these mutations.

Other non-limiting examples of positions of amino acid modifications in the amino acid sequence of SEQ ID NO: 1 include the 75th (G) and the 76th (G), and the amino acid sequence of SEQ ID NO: 1 may have a modification (preferably a deletion) at one or two of the above modification positions.

The position of substitution or insertion of the peptide of structure (A) in a ubiquitin molecule consisting of an amino acid sequence other than the amino acid sequence of SEQ ID NO: 1 can be specified by appropriately aligning the amino acid sequence of the ubiquitin molecule to be grafted with the amino acid sequence of SEQ ID NO: 1. That is, the amino acid sequence of the ubiquitin molecule to be grafted is aligned with the amino acid sequence of SEQ ID NO: 1 using the above-described homology search software or program, and the region corresponding to the 1st to the 6th positions of the amino acid sequence of SEQ ID NO: 1 can be designated as β1, the region corresponding to the 7th to 10th positions of the amino acid sequence of SEQ ID NO: 1 can be designated as the β1-β2 loop structure, and the region corresponding to the 11th to 17th positions of the amino acid sequence of SEQ ID NO: 1 can be designated as β2.

The peptide having structure (A) to be grafted onto a ubiquitin molecule consists of a MuSK-binding region flanked by two spacer regions. The MuSK-binding region consists of the amino acid sequence YX₂₁SYHTNVVASLKR (X₂₁ is P or L) (SEQ ID NO: 11), which is a sequence derived from MuSK7 of formula (1). Spacer 1 can be selected from the group consisting of IQPR (SEQ ID NO: 12), KPGV (SEQ ID NO: 13), WDRG (SEQ ID NO: 14), KPGT (SEQ ID NO: 15), PFGV (SEQ ID NO: 16), YFVG (SEQ ID NO: 17), and LEGM (SEQ ID NO: 18), and spacer 2 can be selected from the group consisting of IRY, MMP, QGI, FMP, TGPQ (SEQ ID NO: 19), WNTP (SEQ ID NO: 20), and IRPQ (SEQ ID NO: 21).

According to the present invention, a ubiquitin molecule grafted with a MuSK-binding region having spacer regions of several amino acid residues at both ends unexpectedly exhibited higher MuSK-binding activity compared to a ubiquitin molecule grafted only with the MuSK-binding region. From the viewpoint of MuSK-binding activity, it is preferable that the MuSK-binding region has spacer sequences 1 and 2 upstream and downstream thereof in any combination of 1 to 7 shown in Table 2 below.

**[Table 2]**

| Table2 : Combination of spacer | | |
|---|---|---|
| | Spacer 1 | Spacer 2 |
| 1 | IQPR | IRY |
| 2 | KPGV | MMP |
| 3 | WDRG | QGI |
| 4 | KPGT | FMP |
| 5 | PFGV | TGPQ |
| 6 | YFVG | WNTP |
| 7 | LEGM | IRPQ |

Non-limiting examples of peptides of structure (A) include peptides consisting of any of the amino acid sequences shown below.
(Ub006) IQPRYPSYHTNVVASLKRIRY (SEQ ID NO: 22)
(Ub007) KPGVYPSYHTNVVASLKRMMP (SEQ ID NO: 23)
(Ub008) WDRGYPSYHTNVVASLKRQGI (SEQ ID NO: 24)
(Ub009) KPGTYPSYHTNVVASLKRFMP (SEQ ID NO: 25)
(Ub010) PFGVYPSYHTNVVASLKRTGPQ (SEQ ID NO: 26)
(Ub011) YFVGYPSYHTNVVASLKRWNTP (SEQ ID NO: 27)
(Ub012) LEGMYLSYHTNVVASLKRIRPQ (SEQ ID NO: 28)
(Ub013) LEGMYPSYHTNVVASLKRIRPQ (SEQ ID NO: 29)

Whether the ubiquitin molecule grafted with the peptide of structure (A) has binding activity to MuSK can be determined according to the examples described below. For example, by using surface plasmon resonance (SPR), the binding kinetics of the molecule to MuSK immobilized on a sensor chip can be quantitatively measured, and if the binding affinity K_{D} by SPR is, for example, 1 µM or less or 500 nM or less, it can be determined that the molecule has binding activity to MuSK. As the MuSK molecule, the extracellular portion (for example, amino acids 1-493) of the human MuSK isoform 1 precursor (GenBank Accession No. NP_005583.1) can be used.

According to a preferred aspect of the present invention, there is provided
a MuSK-binding-region-grafted ubiquitin molecule in which a part or all of a loop structure region between a β1 domain and a β2 domain of the ubiquitin molecule is replaced with a peptide of the structure (A), or the peptide of the structure (A) (preferably any one of peptides Ub006 to Ub013) is inserted into the loop structure region between the β1 domain and the β2 domain of the ubiquitin molecule, in which the ubiquitin molecule is:
a protein consisting of the amino acid sequence of SEQ ID NO: 1;
a protein consisting of an amino acid sequence having 90% or more sequence identity with the amino acid sequence of SEQ ID NO: 1; or
a protein having a modification selected from the group consisting of deletion, substitution, insertion, and addition with respect to one or a plurality of amino acids in the amino acid sequence of SEQ ID NO: 1.

The MuSK-binding-region-grafted ubiquitin molecule of the present invention has high affinity with MuSK, can strongly bind to MuSK, and can be used as a MuSK agonist. In addition, as described below, the complex containing the ubiquitin molecule of the present invention binds to the extracellular domain of MuSK, thereby causing homodimerization of MuSK, thereby selectively inducing downstream MuSK signaling, and thus can activate nerve-to-muscle signaling at the neuromuscular junction. For this reason, the MuSK-binding-region-grafted ubiquitin molecule of the present invention is advantageous in that it can provide a therapeutic method and a research method different from the existing methods since it can strongly activate signal transmission at the neuromuscular junction unlike the approach targeting the anti-acetylcholine receptor autoantibody which has been conventionally studied. That is, the MuSK-binding-region-grafted ubiquitin molecule of the present invention and a pharmaceutically acceptable salt thereof are useful as medicaments and research reagents, and according to the present invention, a pharmaceutical composition containing the MuSK-binding-region-grafted ubiquitin molecule of the present invention or a pharmaceutically acceptable salt thereof and use of the MuSK-binding-region-grafted ubiquitin molecule of the present invention or the pharmaceutically acceptable salt thereof as a pharmaceutical are provided.

### [Method for producing grafted ubiquitin molecule]

The MuSK-binding-region-grafted ubiquitin molecule of the present invention can be suitably produced by a translation synthesis method using a cell-free translation system. It can be prepared by preparing a nucleic acid encoding the ubiquitin molecule of the present invention and translating the nucleic acid in a cell-free translation system. The nucleic acid encoding the ubiquitin molecule of the present invention can be appropriately designed by those skilled in the art using a genetic code used in a translation system of a living body, a reprogrammed genetic code, or a combination thereof. The nucleic acid may be DNA or RNA.

As described above, according to the method using a cell-free translation system, tRNAs aminoacylated with non-natural amino acids can be used to efficiently introduce the non-natural amino acids into a peptide in addition to natural amino acids.

### [Grafted ubiquitin molecule complex]

A grafted ubiquitin molecule complex of the present invention includes a plurality of the ubiquitin molecules of the present invention and has a structure in which the plurality of grafted ubiquitin molecules are bonded to each other via linkers.

As described above, it is known that Agrin binds to the first β-propeller structure of its receptor LRP4 and induces homodimerization of MuSK, which is bound to another β-propeller structure of LRP4, thereby activating downstream signaling through autophosphorylation of MuSK (see Fig. 1). In addition, it is known that activation of downstream signaling of MuSK leads to the formation of clusters of acetylcholine receptors at postsynaptic sites. Since the grafted ubiquitin molecule complex of the present invention has a plurality of pharmacophore configurations corresponding to the MuSK-binding region, each pharmacophore region in the grafted ubiquitin molecule complex is considered to be able to bind to the extracellular domain of MuSK and induce homodimerization of MuSK (see Fig. 2). Accordingly, the grafted ubiquitin molecule complex of the present invention is considered to activate downstream signaling through autophosphorylation of MuSK and induce clustering of acetylcholine receptors. As a result, the grafted ubiquitin molecule complex of the present invention is considered to be able to activate nerve-to-muscle signaling at the neuromuscular junction. Therefore, the grafted ubiquitin molecule complex of the present invention is useful as a therapeutic agent for myasthenia gravis caused by abnormal signaling at the neuromuscular junction. That is, the grafted ubiquitin molecule complex of the present invention and a pharmaceutically acceptable salt thereof are useful as medicaments, and according to the present invention, a pharmaceutical composition containing the grafted ubiquitin molecule complex of the present invention or a pharmaceutically acceptable salt thereof and use of the grafted ubiquitin molecule complex of the present invention or the pharmaceutically acceptable salt thereof as a pharmaceutical are provided.

The grafted ubiquitin molecule complex of the present invention includes the plurality of ubiquitin molecules of the present invention and can exert the above-described action as long as the plurality of ubiquitin molecules of the present invention are bonded to each other via linkers, and the plurality of grafted ubiquitin molecules may be identical or different. From the viewpoint of further promoting homodimerization of MuSK, it is preferable that the plurality of grafted ubiquitin molecules in the complex be identical.

In the grafted ubiquitin molecule complex of the present invention, the linkers connecting the plurality of ubiquitin molecules are not particularly limited, and structures known in the field of peptide synthesis as linkers for connecting peptides or proteins to each other can be adopted. From the viewpoint of further promoting homodimerization of MuSK, the length of the linker is preferably 20Å to 100Å.

Non-limiting examples of the linkers connecting the plurality of grafted ubiquitin molecules in the grafted ubiquitin molecule complex of the present invention include peptides, polymers such as polyethylene glycol (PEG), nucleic acids, saccharides, and combinations thereof. Examples of linkers containing peptides include a peptide having a repeating structure of PA (for example, a peptide of A(PA)v, in which v is an integer of 6 to 8, preferably 7) and a peptide having a repeating structure of EAAAK (for example, a peptide of (EAAAK)x, in which x is an integer of 2 to 4, preferably 3). When a peptide is used as a linker connecting the plurality of grafted ubiquitin molecules, for example in the case of a dimer, the C-terminus of one grafted ubiquitin molecule can be linked to the N-terminus of the other grafted ubiquitin molecule via a peptide. In the case of a trimer or higher-order complex, a similar structure can be adopted. By adopting such a structure, it is possible to express a complex protein of grafted ubiquitin molecules from a single nucleic acid. In addition to the above, a linker containing PEG can be used for the grafted ubiquitin molecule of the present invention, and specifically, the linkers described in the cyclic peptide complex of the present invention can be employed.

### [Method for producing grafted ubiquitin molecule complex]

In the grafted ubiquitin molecule complex of the present invention, the ubiquitin molecules of the present invention can be linked by peptides. In this case, the grafted ubiquitin molecule complex of the present invention can be suitably produced by a translation synthesis method using a cell-free translation system. It can be prepared by preparing a nucleic acid encoding the grafted ubiquitin molecule complex of the present invention and translating the nucleic acid in a cell-free translation system. The nucleic acid encoding the grafted ubiquitin molecule complex of the present invention can be appropriately designed by those skilled in the art using a genetic code used in a translation system of a living body, a reprogrammed genetic code, or a combination thereof. The nucleic acid may be DNA or RNA.

As described above, according to the method using a cell-free translation system, tRNAs aminoacylated with non-natural amino acids can be used to efficiently introduce the non-natural amino acids into a peptide in addition to natural amino acids.

The grafted ubiquitin molecule complex of the present invention can also be prepared using a linker other than peptides. The step of linking the grafted ubiquitin molecule complex of the present invention with a linker can be carried out by a person skilled in the art using a known method or an equivalent method for linking a linker and a peptide, depending on the type of linker used. Specifically, the grafted ubiquitin molecule complex of the present invention can be prepared according to the procedure described in the method for producing a cyclic peptide complex of the present invention.

### [Pharmaceutical composition]

A therapeutic agent and pharmaceutical composition of the present invention contain, as an active ingredient, at least one selected from the group consisting of the cyclic peptide of the present invention and the pharmaceutically acceptable salt thereof, the cyclic peptide complex of the present invention and the pharmaceutically acceptable salt thereof, the MuSK-binding-region-grafted ubiquitin molecule of the present invention and the pharmaceutically acceptable salt thereof, and the MuSK-binding-region-grafted ubiquitin molecule complex of the present invention and the pharmaceutically acceptable salt thereof (hereinafter, these compounds and their salts are referred to as "active ingredients of the present invention").

As described above, the active ingredients of the present invention are considered to be able to induce homodimerization of MuSK, activate downstream signaling through autophosphorylation of MuSK, and induce clustering of acetylcholine receptors. Accordingly, since the active ingredients of the present invention can activate nerve-to-muscle signaling at the neuromuscular junction, they can be used as therapeutic agents for myasthenia gravis caused by abnormal signaling at the neuromuscular junction. That is, the therapeutic agent and pharmaceutical composition of the present invention can be preferably used for the treatment of myasthenia gravis. In the present specification, "treatment" includes therapeutic treatment and prophylactic treatment, and means alleviating or eliminating the cause of a disease in a patient having the disease, delaying or stopping the progression of the disease, alleviating, mitigating, improving, or eliminating the symptoms of the disease, and/or suppressing aggravation of the symptoms of the disease.

Patients with myasthenia gravis include patients positive for anti-acetylcholine receptor antibodies, patients positive for anti-MuSK antibodies, patients positive for anti-LRP4 antibodies, and antibody-negative (seronegative type) patients in whom all of these autoantibodies are negative. Since the active ingredients of the present invention can activate MuSK and induce clustering of acetylcholine receptors at postsynaptic sites of neuromuscular junctions, they are advantageous in that they can be used to treat not only anti-acetylcholine receptor antibody-positive myasthenia gravis but also anti-MuSK antibody-positive myasthenia gravis, anti-LRP4 antibody-positive myasthenia gravis, and antibody-negative patients.

When the active ingredients of the present invention are administered to a subject, the administration route is not particularly limited as long as the desired therapeutic effect can be obtained, but oral administration or parenteral administration (for example, intravenous administration or subcutaneous administration) can be selected. The therapeutic agent and pharmaceutical composition of the present invention may be formulated by adding a pharmaceutically acceptable carrier or the like in addition to the above active ingredients.

Dosage forms of pharmaceutical formulations include, for example, liquid formulations (such as injections), dispersions, suspensions, tablets, pills, powders, suppositories, powdered formulations, fine granules, granules, capsules, syrups, troches, inhalants, ointments, eye drops, nasal drops, ear drops, and cataplasms. These formulations can be formulated using pharmaceutically acceptable carriers by methods commonly practiced in the field (for example, known methods described in the General Rules for Preparations in the Japanese Pharmacopoeia, 18th Edition).

Formulation can be carried out by conventional methods using appropriate additives such as excipients, binders, disintegrants, lubricants, solvents, solubilizing aids, coloring agents, flavoring and odor-correcting agents, stabilizers, emulsifiers, absorption promoters, surfactants, pH adjusters, preservatives, humectants, dispersants, and antioxidants. Additives are not particularly limited, and examples thereof include purified water, saline, phosphate buffer, dextrose, and pharmaceutically acceptable organic solvents such as, glycerol and ethanol, animal and vegetable oils, lactose, mannitol, glucose, sorbitol, crystalline cellulose, hydroxypropylcellulose, starch, cornstarch, anhydrous silicic acid, magnesium aluminum silicate, collagen, polyvinyl alcohol, polyvinylpyrrolidone, carboxyvinyl polymer, carboxymethylcellulose sodium, sodium polyacrylate, sodium alginate, water-soluble dextran, carboxymethyl starch sodium, pectin, methylcellulose, ethyl cellulose, xanthan gum, gum arabic, tragacanth, casein, agar, polyethylene glycol, diglycerin, glycerin, propylene glycol, petrolatum, paraffin, octyldodecyl myristate, isopropyl myristate, higher alcohols, stearyl alcohol, stearic acid, and human serum albumin.

Liquid formulations may contain distilled water for injection, physiological saline, propylene glycol, polyethylene glycol, vegetable oils, and alcohols. Humectants, emulsifiers, dispersants, stabilizers, solvents, solubilizing aids, and preservatives may be added to the liquid formulations. Tablets or pills may be coated tablets covered with sugar coating, gastric-soluble substances, enteric-soluble substances, and the like.

The dosage of the active ingredients in the present invention can be determined depending on factors such as the type of active ingredient, sex, age, and body weight of the administration subject, symptoms, dosage forms, administration routes, and the like. In the present invention, when the active ingredients are administered for the purpose of treating myasthenia gravis, the single dose for an adult can be determined, for example, in the range of 0.0001 mg/kg body weight to 1000 mg/kg body weight, but is not limited thereto. In addition, the above dose of the active ingredients can be administered once a day or divided into two to four doses. The active ingredients of the present invention can be administered not only to humans in need thereof but also to non-human mammals (for example, mice, rats, rabbits, dogs, cats, cattle, horses, pigs, sheep, goats, and monkeys).

According to the present invention, there is provided a method for treating myasthenia gravis, including administering to a subject in need of a therapeutically effective amount of at least one selected from the group consisting of the cyclic peptide of the present invention and the pharmaceutically acceptable salt thereof, the cyclic peptide complex of the present invention and the pharmaceutically acceptable salt thereof, the MuSK-binding-region-grafted ubiquitin molecule of the present invention and the pharmaceutically acceptable salt thereof, and the MuSK-binding-region-grafted ubiquitin molecule complex of the present invention and the pharmaceutically acceptable salt thereof, or a therapeutic agent or pharmaceutical composition containing the same.

According to the present invention, there is also provided use of at least one selected from the group consisting of the cyclic peptide of the present invention or the pharmaceutically acceptable salt thereof, the cyclic peptide complex of the present invention and the pharmaceutically acceptable salt thereof, the MuSK-binding-region-grafted ubiquitin molecule of the present invention and the pharmaceutically acceptable salt thereof, and the MuSK-binding-region-grafted ubiquitin molecule complex of the present invention and the pharmaceutically acceptable salt thereof, for the production of a therapeutic agent for myasthenia gravis, in a method for treating myasthenia gravis, or as a therapeutic agent for myasthenia gravis.

### [Method for presenting cyclic peptide and method for producing ubiquitin molecule on which cyclic peptide is presented]

According to the present invention, there is provided a method for presenting a cyclic peptide on a ubiquitin molecule together with a flanking spacer sequence. In addition, according to the present invention, as a method for fusing a cyclic peptide to a protein, there is provided a method for producing a modified protein in which the cyclic peptide is fused to a ubiquitin molecule and thereby presented on the ubiquitin molecule. For fusing a cyclic peptide to a ubiquitin molecule together with a spacer sequence, ordinary genetic engineering techniques can be employed. The cyclic peptide may have binding activity to a desired molecule, and preferably is a cyclic peptide having binding activity to a MuSK molecule.

As the cyclic peptide, it is preferable to use a cyclic peptide selected by an mRNA display method such as the RaPID method or the TRAP method, or a phage display method, and it is more preferable to use an mRNA display method. According to the mRNA display method and the like, among cyclic peptides, the nucleotide sequence of the partial amino acid sequence to be inserted can be easily understood.

In the presentation method and production method of the present invention, a cyclic peptide having MuSK-binding activity can be presented on a loop structure of a ubiquitin molecule by a lasso-grafting method (LassoGraft Technology). The lasso-grafting method is a technique for grafting a cyclic peptide having binding activity to a pharmaceutical target such as a receptor onto a protein. In carrying out the presentation method and production method of the present invention, reference can be made to Mihara, E. et al., Nat Commun 2021, 12:1543, and Japanese Patent No. 6598344. According to this technique, by embedding (grafting) an internal sequence of only a dozen or so amino acids of a cyclic peptide obtained by the RaPID method into a loop structure on the surface of a protein in a lasso-like manner, very high specificity and affinity for a pharmaceutical target protein possessed by the original cyclic peptide can be imparted intact to the scaffold protein.

The method of the present invention is characterized in that, when presenting a cyclic peptide having binding activity to a desired molecule (preferably MuSK-binding activity) on a loop structure of a ubiquitin molecule, a spacer sequence consisting of an amino acid sequence of 0 to 4 residues (preferably 3 or 4 residues) randomly generated is added upstream and downstream of the cyclic peptide. That is, one spacer sequence of the two spacers can be disposed upstream of the cyclic peptide, and the other spacer sequence can be disposed downstream of the cyclic peptide. In addition, the cyclic peptide having MuSK-binding activity can be selected from the cyclic peptides of the present invention, and can be preferably selected from the cyclic peptides of formulas (1) to (3).

In a preferred aspect of the method of the present invention, when the desired molecule is a MuSK molecule, the cyclic peptide and spacer sequence presented on a loop structure region of a ubiquitin molecule can have the structure (B). In structure (B), spacer 3 is preferably a peptide chain consisting of 3 or 4 arbitrary amino acids, and spacer 4 is preferably a peptide chain consisting of 3 or 4 arbitrary amino acids. The MuSK-binding region is a region which is present in the cyclic peptide and has MuSK molecule-binding activity, and can be selected, for example, from MuSK molecule-binding activity regions identified in the cyclic peptides of formulas (1) to (3), and can be a sequence excluding the cysteine at the C-terminus involved in the cyclic structure. The MuSK-binding region is preferably a peptide chain having the amino acid sequence YX₂₂SYHTNVVASLKR (X₂₂ is P or L) (SEQ ID NO: 11).

In the presentation method and production method of the present invention, the loop structure region of the ubiquitin molecule may be any one of:
- a loop structure region between a β1 domain and the β2 domain,
- a loop structure region between a β2 domain and an α1 domain,
- a loop structure region between an α1 domain and a β3 domain,
- a loop structure region between a β3 domain and a β4 domain,
- a loop structure region between a β4 domain and an α2 domain, and
- a loop structure region between an α2 domain and a β5 domain,
and is preferably a loop structure region between a β1 domain and a β2 domain.

The loop structure region between a β1 domain and a β2 domain of the ubiquitin molecule corresponds to the 7th to the 10th amino acid residues based on the amino acid sequence of SEQ ID NO: 1. Here, the ubiquitin molecule on which the cyclic peptide is presented in the method of the present invention is not limited to the amino acid sequence of SEQ ID NO: 1. That is, a protein consisting of an amino acid sequence having 90% or more (preferably 91% or more, 92% or more, 93% or more, 94% or more, 95% or more, 96% or more, 97% or more, or 98% or more) sequence identity to the amino acid sequence of SEQ ID NO: 1, or a protein having a modification selected from the group consisting of deletion, substitution, insertion, and addition of one or a plurality of amino acids in the amino acid sequence of SEQ ID NO: 1 can be the ubiquitin molecule on which the cyclic peptide is presented. In addition, in such a case, the position of substitution or insertion of the cyclic peptide in a ubiquitin molecule consisting of an amino acid sequence other than the amino acid sequence of SEQ ID NO: 1 can be specified by appropriately aligning the amino acid sequence of the ubiquitin molecule with the amino acid sequence of SEQ ID NO: 1. In the method of the present invention, when carried out on a ubiquitin molecule consisting of an amino acid sequence other than the amino acid sequence of SEQ ID NO: 1, reference can be made to the description of the grafted ubiquitin molecule of the present invention.

In the presentation method and production method of the present invention, the 7th amino acid residue and the 10th amino acid residue of a ubiquitin molecule can be selected as two amino acid residues constituting the loop structure region between a β1 domain and a β2 domain. In this case, the nucleotide sequence of a modified protein in which a cyclic peptide is presented on the ubiquitin molecule can be prepared as follows. That is, in the nucleotide sequence encoding the ubiquitin molecule, a nucleotide sequence corresponding to two amino acid residues constituting the loop structure region between a β1 domain and a β2 domain of the ubiquitin molecule can be selected, and bases present between the selected nucleotide sequences can be deleted as necessary to prepare a nucleic acid having an incorporated nucleotide sequence obtained by inserting the nucleotide sequence corresponding to the partial amino acid sequence of the selected cyclic peptide and to spacer sequences upstream and downstream thereof.

In the present invention, the method for selecting a nucleotide sequence corresponding to two amino acid residues constituting a loop structure to delete bases present between the selected nucleotide sequences as necessary or the method for preparing a nucleic acid having an incorporated nucleotide sequence obtained by inserting the nucleotide sequence corresponding to the partial amino acid sequence of the selected cyclic peptide is not particularly limited and can be carried out by employing conventionally known methods. In addition, since methods for translating the prepared nucleic acid are already known in the technical field to which the present invention belongs, translation of the nucleic acid can be performed by appropriately applying such known methods.

A modified protein obtained by the presentation method and production method of the present invention, in which a cyclic peptide having binding activity to a desired molecule (preferably MuSK-binding activity) is presented on a protein using a ubiquitin molecule as a scaffold, presents the cyclic peptide while maintaining the structure and activity of the cyclic peptide, and thus can be used as a reagent, a pharmaceutical, or the like based on the activity of the cyclic peptide. In addition, since the modified protein fused with the cyclic peptide exhibits, in addition to the activity possessed by ubiquitin itself, the activity possessed by the cyclic peptide, it can also be used as a MuSK-binding protein that stably exists in vivo (for example, in blood or in tissues).

### [Screening method]

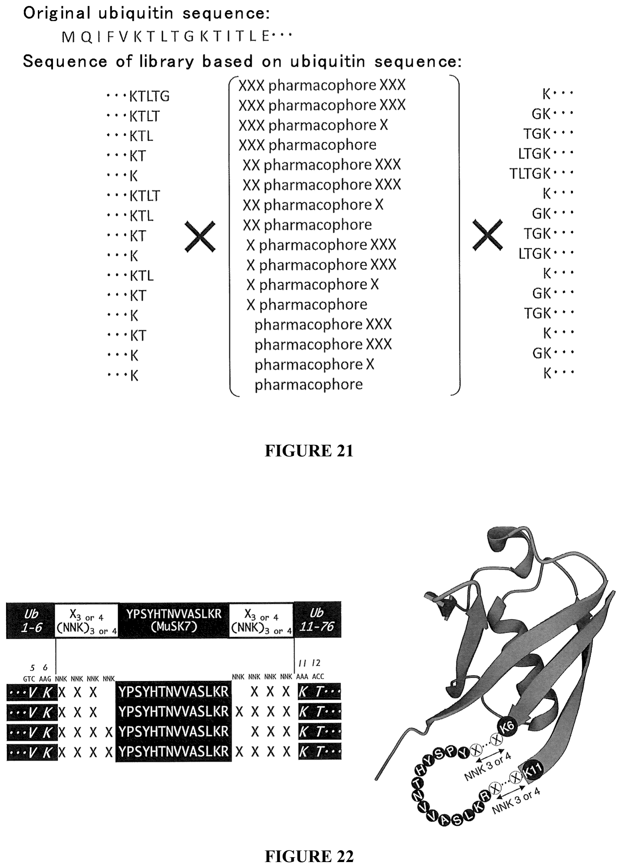

As shown in examples described below, by designing a combination of randomly generated spacer sequences and a MuSK-binding region (see Figs. 22 and 23), preparing an mRNA library encoding the amino acid sequence thereof, translating mRNA from the mRNA library to prepare a modified protein, and selecting a MuSK-binding modified protein from the obtained modified proteins using MuSK-binding activity as an index, it is possible to screen for a MuSK-binding modified protein. That is, according to the present invention, there is provided a method for screening for a modified protein which binds to a desired molecule (preferably a MuSK-binding modified protein), the method including: designing an mRNA library including a nucleotide sequence encoding a ubiquitin molecule in which a sequence of a cyclic peptide having binding activity to the desired molecule (preferably a cyclic peptide having binding activity to a MuSK molecule) and spacer sequences consisting of amino acid sequences of 0 to 4 residues (preferably 3 or 4 residues) randomly generated and linked upstream and downstream of the cyclic peptide are fused to a loop structure region between a β1 domain and a β2 domain; translating mRNA from the mRNA library to prepare a modified protein; and selecting, from the obtained modified protein, a modified protein which binds to the desired molecule (preferably the MuSK-binding modified protein), using MuSK-binding activity as an index. Preparation of the mRNA library (that is, preparation of a nucleic acid to be translated) and the step of translating a nucleic acid from the mRNA library to prepare a modified protein can be carried out according to the production method of the present invention. In addition, the screening method of the present invention can also be carried out according to the presentation method and production method of the present invention.

In the screening method of the present invention, when screening for a MuSK-binding modified protein, whether a ubiquitin molecule grafted with a MuSK-binding cyclic peptide (preferably a peptide of structure (B)) has binding activity to MuSK can be determined according to the examples described below. For example, by using surface plasmon resonance (SPR), the binding kinetics of the molecule to MuSK immobilized on a sensor chip can be quantitatively measured, and if the binding affinity K_{D} by SPR is, for example, 1 µM or less or 500 nM or less, it can be determined that the molecule has binding activity to MuSK. As the MuSK molecule, the extracellular portion (for example, amino acids 1-493) of the human MuSK isoform 1 precursor (GenBank Accession No. NP_005583.1) can be used.

### EXAMPLES

The present invention will be described in more detail based on the following examples, but the present invention is not limited to these examples.

### Example 1: Preparation of cyclic peptides and evaluation of MuSK-binding activity

### (1) Preparation of libraries of cyclic peptides and selection of cyclic peptides binding to MuSK

Two thioether-cyclic peptide libraries (L-library and D-library) were constructed using a flexible in vitro translation (FIT) system. N-(2-chloroacetyl)-L-tyrosine (L-ClAcTyr) and N-(2-chloroacetyl)-D-tyrosine (D-ClAcTyr) were introduced into the L-library and the D-library, respectively, using previously reported methods (Kawakami, T.; Murakami, H.; Suga, H., Messenger RNA-programmed incorporation of multiple N-methyl-amino acids into linear and cyclic peptides. Chem Biol 2008, 15 (1), 32-42.; Morimoto, J.; Hayashi, Y.; Suga, H., Discovery of macrocyclic peptides armed with a mechanism-based warhead: isoform-selective inhibition of human deacetylase SIRT2. Angew Chem Int Ed Engl 2012, 51 (14), 3423-7.; Goto, Y.; Katoh, T.; Suga, H., Flexizymes for genetic code reprogramming. Nat Protoc 2011, 6 (6), 779-90.; Hayashi, Y.; Morimoto, J.; Suga, H., In vitro selection of anti-Akt2 thioether-macrocyclic peptides leading to isoform-selective inhibitors. ACS Chem Biol 2012, 7 (3), 607-13.).

mRNA libraries corresponding to the thioether-cyclic peptide libraries were designed to contain, in order, an AUG initiation codon encoding L-ClAcTyr or D-ClAcTyr, 8 to 15 NNK random codons encoding random proteinogenic amino acid residues (N is G, C, A, or U; K is G or U), and a UGC codon encoding Cys. After in vitro translation, a thioether bond was spontaneously formed between the acetylchloride group of the Tyr residue at the N-terminus and the sulfhydryl group (thiol group) of the downstream cysteine residue.

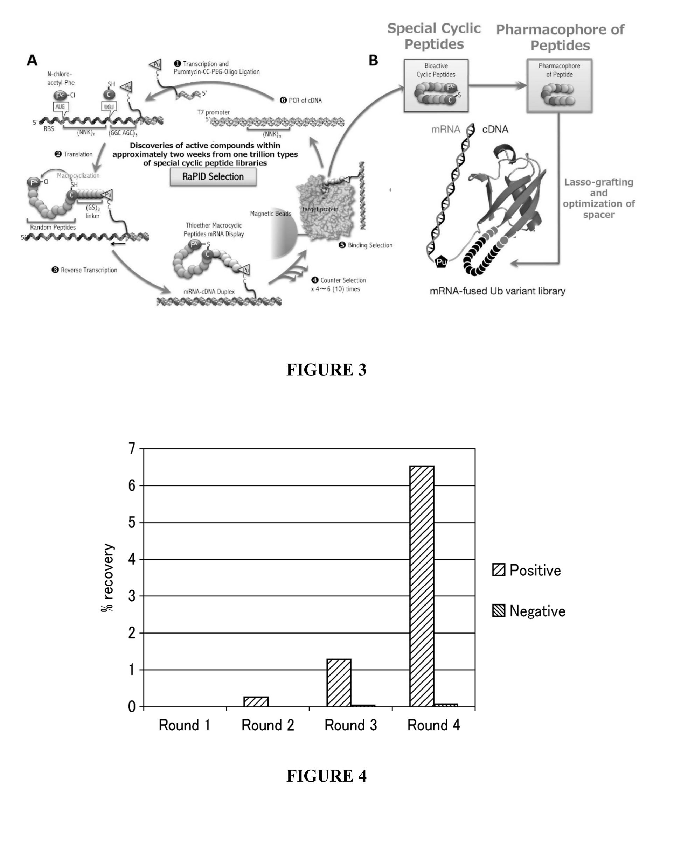

Selection of cyclic peptides binding to human MuSK (hereinafter also referred to as "affinity selection") was carried out using a random non-standard peptides integrated discovery (RaPID) system in combination with a flexible in vitro translation (FIT) system (Fig. 3A) (Hayashi, Y.; Morimoto, J.; Suga, H., In vitro selection of anti-Akt2 thioether-macrocyclic peptides leading to isoform-selective inhibitors. ACS Chem Biol 2012, 7 (3), 607-13.; Hipolito, C.J. & Suga, H. Ribosomal production and in vitro selection of natural product-like peptidomimetics: the FIT and RaPID systems. Curr Opin Chem Biol. 16, 196-203, 2012). In this example, selection of cyclic peptides was performed using the D-library.

More specifically, preparation of the library of cyclic peptides and selection of cyclic peptides binding to MuSK were carried out as follows.

The mRNA library, ClAc-L-Tyr-tRNA^{fMet}_{CAU}, and ClAc-D-Tyr-tRNA^{fMet}_{CAU} were prepared as previously reported (Hayashi, Y.; Morimoto, J.; Suga, H., In vitro selection of anti-Akt2 thioether-macrocyclic peptides leading to isoform-selective inhibitors. ACS Chem Biol 2012, 7 (3), 607-13.; Hipolito, C.J. & Suga, H., Ribosomal production and in vitro selection of natural product-like peptidomimetics: the FIT and RaPID systems. Curr Opin Chem Biol. 16, 196-203, 2012).

A 1 µM mRNA library was ligated with a 1.5 µM puromycin linker at 25 °C for 30 minutes using T4 RNA ligase. The DNA of the above puromycin linker was bound to the 3' terminal constant region of the mRNA library. After purification by phenol-chloroform extraction and ethanol precipitation, the obtained 1.2 µM mRNA-puromycin conjugate and 50 µM ClAc-L-Tyr-tRNA^{fMet}_{CAU} or ClAc-D-Tyr-tRNA^{fMet}_{CAU} were used for translation at 37°C for 30 minutes in a methionine-deficient FIT system. Subsequently, the reaction mixture was further incubated at 25°C for 12 minutes to promote formation of an mRNA-peptide conjugate. Furthermore, incubation was performed at 37°C for 30 minutes in the presence of 16.7 mM EDTA. Thereafter, the product was reverse-transcribed at 42°C for 1 hour using RNase H-minus reverse transcriptase (Promega), thereby tagging the cyclic peptide to an mRNA-cDNA hybrid. Then, an equal amount of Blocking buffer (100 mM Tris-HCl (pH 7.6), 300 mM NaCl, 0.1 v/v% Tween-20, 0.2 w/v% bovine serum albumin (acetylated)) was added to the reaction solution.

Next, affinity selection was performed on the obtained peptide-mRNA/cDNA conjugate library. First, as control selection, the peptide-mRNA/cDNA conjugate library was passed three times through Dynabeads Protein G (Thermo Fisher Scientific Inc.) (each passage was carried out under the condition of 4°C for 10 minutes). This process is called negative selection and removes undesirable bead binding. Next, affinity selection was performed on the peptide-mRNA/cDNA conjugate library using the above beads immobilized with 200 to 400 nM human MuSK protein (extracellular portion) under the condition of 4°C for 30 minutes. This process is called positive selection.

By heating at 95°C for 5 minutes, cDNA was eluted from the beads, and a portion of the eluted cDNA was evaluated by quantitative PCR using SYBR Green I and a thermal cycler (LightCycler, Roche). The remaining cDNA was amplified by PCR and used to transcribe an mRNA library for use in the next round of affinity selection.

The human MuSK protein (extracellular portion) was prepared as a recombinant protein as follows. Using a Flexi clone (FXC31275, Promega Corporation) encoding Human MuSK isoform 1 precursor (NCBI Reference Sequence: NP_005583.1) as a template, DNA corresponding to the extracellular portion (amino acids 1-493) was amplified by a PCR method. XhoI and EcoRI restriction enzyme recognition sequences were added to both the 5' and 3' ends of the amplified DNA sequence, and these were used for insertion into the multiple cloning site of a pCAG-Neo hIgG1-Fc plasmid (Fujifilm Wako Pure Chemical Corporation). This plasmid (pCAG-Neo hMuSK-Fc) enables expression of a chimeric protein of the secretory human MuSK extracellular portion and human IgG1 Fc (hMuSK-Fc; molecular weight of approximately 200 kDa) in animal cells. In practice, ExpiFectamine 293 reagent (Invitrogen) was used to transfect the pCAG-Neo hMuSK-Fc plasmid into Expi293F cells (Invitrogen), and shaking culture was carried out (for 6 days) in an incubator containing 8% CO₂. Next, the culture supernatant was collected, centrifuged, and sterilized by filtration through a 0.22 µm filter. The obtained culture supernatant was subjected to Protein G purification, and finally, a purified hMuSK-Fc sample with 99.96% purity (AKTA-FPLC analysis) was obtained.

Preparation of the library, negative selection, and positive selection constituted one round, and four such rounds were carried out. After four rounds, significant enrichment of cDNA was observed (compared with a case where the MuSK protein (extracellular portion) was not immobilized on the beads in the positive selection) (Fig. 4). The recovered cDNA was amplified by PCR, purified using a column (NucleoSpin, Machery-Nagel), and then sequenced using a high-throughput sequencer (MiSeq, Illumina). Data analysis was carried out using software (CLC Sequence Viewer 7, Qiagen).

An example of the structure of a cyclic peptide and the like binding to MuSK, selected by the RaPID system, is shown below. 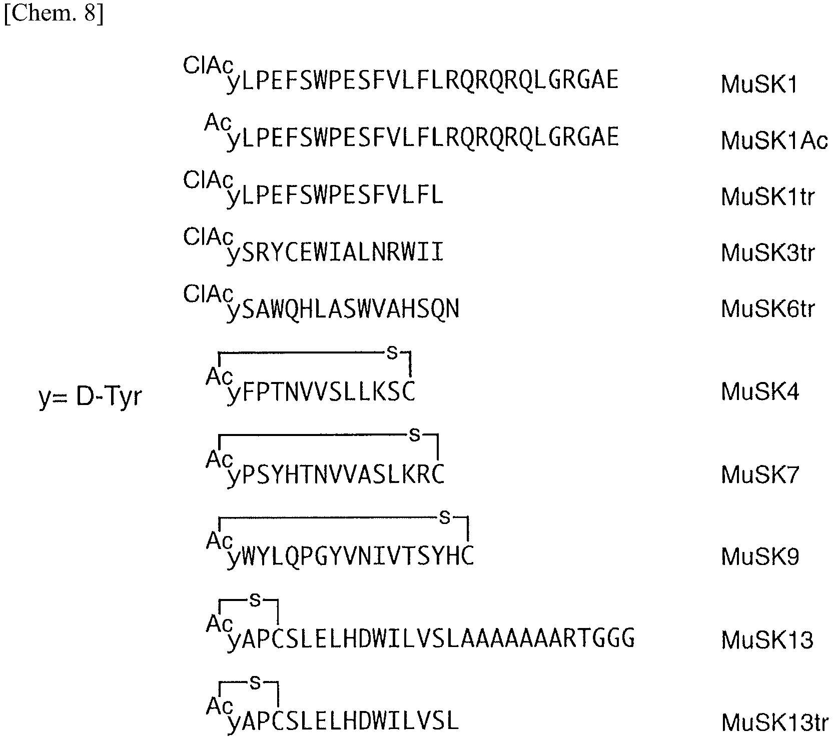 In the above formula, the sequence numbers are as follows. MuSK1 and MuSK1Ac: SEQ ID NO: 5, MuSK1tr: SEQ ID NO: 6, MuSK3tr: SEQ ID NO: 7, MuSK6tr: SEQ ID NO: 8, MuSK4: SEQ ID NO: 3, MuSK7: SEQ ID NO: 2, MuSK9: SEQ ID NO: 4, MuSK13: SEQ ID NO: 9, MuSK13tr: SEQ ID NO: 10.

### (2) Chemical synthesis of cyclic peptides

From the cyclic peptides whose sequences had been determined, sequences with a high abundance ratio in the L-library and the D-library (MuSK1, MuSK1Ac, MuSK4, MuSK7, and MuSK9) were selected and prepared by chemical synthesis. That is, in accordance with known methods, cyclic peptides were synthesized by Fmoc solid-phase peptide synthesis (SPPS) using a Syro Wave automated peptide synthesizer (Biotage) (Ito, K. et al. Artificial human Met agonists based on macrocycle scaffolds. Nature Communications 6, 6372, 2015). After automated synthesis of each peptide, an N-terminal chloroacetyl cap was introduced into the obtained product by final coupling with chloroacetic acid. The peptides were cleaved from the resin over 3 hours using a solution of 92.5% trifluoroacetic acid (TFA), 2.5% triisopropylsilane (TIPS), 2.5% ethanedithiol (EDT), and 2.5% water. The peptide products were precipitated from ice-cold ether. After threefold dilution with ice-cold ether, the pellet was suspended in 4/1 DMSO/H₂O (30 mL at 75 µmol scale). Then, 60 µL of triethylamine (or an amount that makes pH > 8) was added, and the reaction was carried out at 42°C (for about 1 hour). The cyclization reaction was monitored by MALDI/TOFMS (α-cyano-4-hydroxycinnamic acid matrix), and after confirming completion of the reaction, the reaction was quenched with TFA.

The cyclized cyclic peptides were purified by reverse-phase HPLC (Shimadzu Prominence LC-20AP with a Merck Chromolith Prep column, 200 × 25 mm i.d.). As mobile phases, water containing 0.1 v/v% TFA and acetonitrile containing 0.1 v/v% TFA were used. After identifying the fraction containing the desired peptide by MALDI/TOFMS (α-cyano-4-hydroxycinnamic acid matrix), the fraction was lyophilized. In addition, in the case of peptides to be used for cell tests, the operation of "dissolving the dried peptide in a 50 v/v% acetonitrile-water mixture containing 5 mM HCl, followed by lyophilization" was repeated two to three times.

### (3) Evaluation of cyclic peptides

For the cyclic peptides obtained as described above (MuSKl, MuSK1Ac, MuSK4, MuSK7, and MuSK9), affinity for MuSK was evaluated (binding activity test).

The strength of binding between MuSK and the cyclic peptides was evaluated by quantitatively measuring the binding kinetics (binding affinity (dissociation constant) K_{D}) of the cyclic peptides to MuSK immobilized on a sensor chip via an Fc tag, using surface plasmon resonance (SPR).

The binding affinity of each monomer peptide, dimer peptide, monomer U-body, and dimer U-body to target proteins human MuSK, mouse MuSK, and rat MuSK was analyzed by surface plasmon resonance (SPR) at 25°C using a Biacore T200 (Cytiva). According to the standard immobilization protocol provided by the manufacturer, Fc-tagged human/mouse/rat MuSK was immobilized on a Protein G chip or a Protein A chip (Cytiva). A running buffer for the monomer/dimer peptides contained 0.010 M phosphate, 0.14 M NaCl, 0.0027 M KCl, 0.05% (v/v) Tween 20, and 0.1% (v/v) DMSO. A running buffer for the monomer/dimer U-bodies contained 0.010 M phosphate, 0.14 M NaCl, 0.0027 M KCl, and 0.05% (v/v) Tween 20. A series of concentrations of each monomer peptide, dimer peptide, monomer U-body, and dimer U-body was injected as analytes. A 1:1 binding fitting analysis was applied using Biacore evaluation software, and ka, kd, and K_{D} values were determined.

Fc-tagged human MuSK was prepared according to the procedure described in Example 1(1). In addition, Fc-tagged mouse MuSK was used by expressing a nucleotide sequence encoding the Fc-tagged mouse MuSK extracellular domain (SEQ ID NO: 34). In addition, Fc-tagged rat MuSK was used as a chimeric protein of rat MuSK and an Fc protein, namely, Recombinant Rat MuSK Fc Chimera Protein (catalog number: 9847-MK-050, R&D Systems).

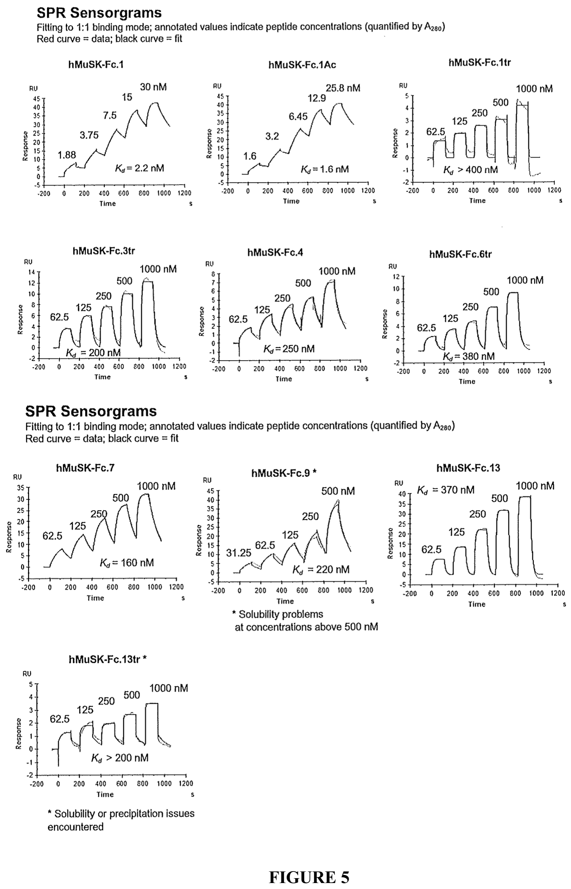

The SPR response of each peptide to human MuSK is shown in Fig. 5, and the binding affinity K_{D} to human MuSK is shown in Table 3.

**[Table 3]**

| Table 3: Human MuSK-binding activity of various peptides | |
|---|---|
| | Binding activity (K_{D}) |
| MuSK7 | 160 nM |
| MuSK4 | 250 nM |
| MuSK9 | NA |
| MuSK1 | 2.2 nM |
| MuSK1Ac | 1.6 nM |

| | |
|---|---|
| *NA : Could not be measured due to low solubility. | |

As shown in Fig. 5 and Table 3, all of the peptides had high binding activity to MuSK.

### Example 2: Verification of efficacy of cyclic peptide complexes against the onset mechanism of myasthenia gravis

### (1) Preparation of cyclic peptide homodimers

In this example, compounds were prepared in which MuSK4, MuSK7, and MuSK9, which are human MuSK-binding cyclic peptides, and MuSK1Ac, which is a human MuSK-binding linear peptide, were each homodimerized by linking with two types of linkers. For MuSK1Ac, a complex in which the N-terminal sides were linked with a linker and a complex in which the C-terminal sides were linked with a linker were prepared. Specifically, they were prepared by the following procedure.

First, MuSK1Ac, MuSK4, MuSK7, and MuSK9 were prepared by chemical synthesis. That is, in accordance with known methods, cyclic peptides were synthesized by Fmoc solid-phase peptide synthesis (SPPS) using a Syro Wave automated peptide synthesizer (Biotage) (Ito, K. et al. Artificial human Met agonists based on macrocycle scaffolds. Nature Communications 6, 6372, 2015). Specifically, a di-t-butyl disulfide (StBu) protecting group was introduced into the terminal Cys, and the peptides were prepared on a Rink amide resin. After automated synthesis of each peptide, an N-terminal chloroacetyl cap was introduced into the obtained product by final coupling with chloroacetic acid. The peptides were cleaved from the resin over 3 hours using a solution of 88% trifluoroacetic acid (TFA), 5% phenol, 5% water, and 2% TIPS. The peptide products were precipitated from ice-cold ether. After threefold dilution with ice-cold ether, the pellet was suspended in 4/1 DMSO/H₂O (30 mL at 75 µmol scale). Then, 60 µL of triethylamine (or an amount that makes pH > 8) was added, and the reaction was carried out at 42°C (for about 1 hour). The cyclization reaction was monitored by MALDI/TOFMS (α-cyano-4-hydroxycinnamic acid matrix), and after confirming completion of the reaction, the reaction was quenched with TFA. Thereafter, the pH was checked and adjusted to about 7 with sodium hydroxide. 1.2 mL of tributylphosphine (for 75 µmol scale) was added in two portions: 0.6 mL initially, followed by 0.6 mL after 2 hours. To complete the reductive cleavage of the StBu group from the terminal Cys, the mixture was vigorously stirred at room temperature for a total of 4 hours.

The deprotection reaction of the StBu group was monitored by MALDI/TOFMS (α-cyano-4-hydroxycinnamic acid matrix), and after confirming completion of the reaction, the reaction was quenched with TFA. The deprotected cyclic peptides were purified by reverse-phase HPLC (Shimadzu Prominence LC-20AP with a Merck Chromolith Prep column, 200 × 25 mm i.d.). As mobile phases, water containing 0.1 v/v% TFA and acetonitrile containing 0.1 v/v% TFA were used. After identifying the fraction containing the desired peptide by MALDI/TOFMS (α-cyano-4-hydroxycinnamic acid matrix), the fraction was lyophilized. In addition, in the case of peptides to be used for cell tests, the operation of "dissolving the dried peptide in a 50 v/v% acetonitrile-water mixture containing 5 mM HCl, followed by lyophilization" was repeated two to three times.

For MuSK4, MuSK7, and MuSK9 used in the linking process for preparing complexes, another cysteine residue (with its carboxyl group forming an acid amide) was introduced downstream of the C-terminal cysteine residue for linking with a linker. For MuSK1Ac to be linked at the N-terminal sides with a linker, a cysteine residue (with its amino group acetylated) for linking with the linker was introduced upstream of the N-terminal D-tyrosine residue. For MuSK1Ac to be linked at the C-terminal sides with a linker, a cysteine residue (with its carboxyl group forming an acid amide) for linking with the linker was introduced downstream of the C-terminal glutamic acid residue.

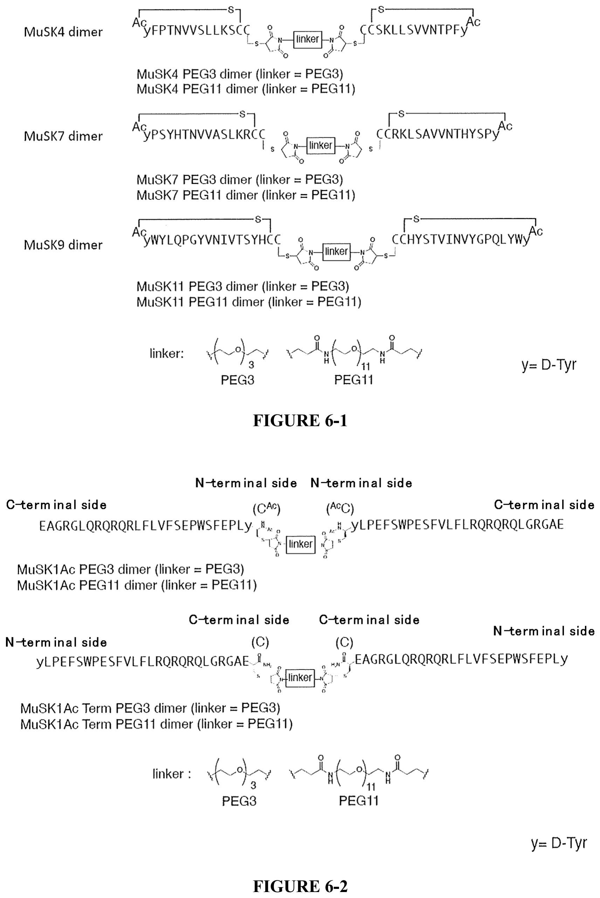

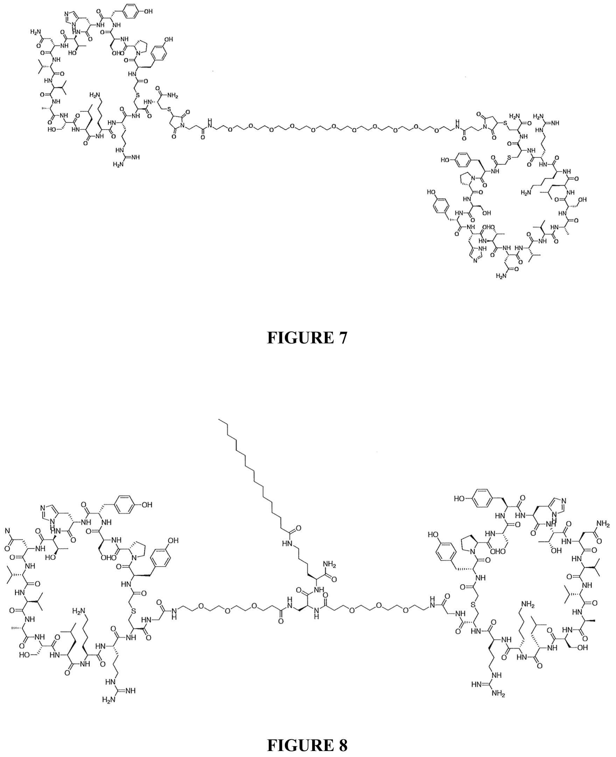

Next, the lyophilized cyclic peptide monomer (prepared as described above) was resuspended in 1/1 ACN/(50 mM sodium phosphate buffer, pH 7.5) at a concentration of about 2 mg/mL. While stirring, Bis-Mal-dPEG (trademark) 11 (Quanta Biodesign) or Bis-Mal-dPEG (trademark) 3 (Quanta Biodesign), each as a 100 mM solution in DMSO, was slowly added dropwise in 0.5 equivalents. The consumption of the starting material was checked by UPLC/LCMS, and reactants were added as necessary (dimerization was completed within 2 hours). In the cases of MuSK1Ac, MuSK4, and MuSK7, the mixture was directly purified by HPLC. In the case of MuSK9, a dimer precipitated from the solution. After the reaction, the precipitate was isolated by centrifugation. The obtained pellet was redissolved in 100% DMSO, loaded onto HPLC, and purified by a conventional method. For reference, the structures of the prepared cyclic peptide homodimers are shown in Figs. 6-1 and 6-2. In addition, the chemical structure of the homodimer of MuSK7 prepared using Bis-Mal-dPEG (trademark) 11 as a linker is shown in Fig. 7.

### (2) Evaluation of MuSK-binding activity of cyclic peptide homodimers

For each of the homodimers (10 species) of MuSK1Ac, MuSK4, MuSK7, and MuSK9 obtained according to the procedure described in (1) above, affinity for human MuSK was evaluated (binding activity test). The evaluation test was carried out according to the procedure described in Example 1(3). The results are shown in Table 4.

**[Table 4]**

| Table 4: MuSK-binding activity of various peptide complexes | | | |
|---|---|---|---|
| peptide | K_{d} (nM) | kₒₙ (M⁻¹s⁻¹) | k_{off} (s⁻¹) |
| MuSK1Ac PEG3 dimer | 4.4 | 3.0 x 10⁵ | 1.3 x 10⁻³ |
| MuSK1Ac PEG11 dimer | 1.5 | 6.6 x 10⁵ | 1.0 x 10⁻³ |
| MuSK1Ac Term PEG3 dimer | 6.0 | 2.0 x 10⁵ | 1.2 x 10⁻³ |
| MuSK1Ac Term PEG11 dimer | 3.6 | 5.2 x 10⁵ | 1.8 x 10⁻³ |
| MuSK4 PEG3 dimer | 6.8 | 6.5 x 10⁵ | 4.4 x 10⁻³ |
| MuSK4 PEG11 dimer | 6.9 | 8.1 x 10⁵ | 5.6 x 10⁻³ |
| MuSK7 PEG3 dimer | 0.62 | 5.4 x 10⁵ | 3.4 x 10⁻⁴ |
| MuSK7 PEG11 dimer | 1.3 | 3.9 x 10⁵ | 5.1 x 10⁻⁴ |
| MuSK9 PEG3 dimer | 2.1 | 7.8 x 10⁵ | 1.7 x 10⁻³ |
| MuSK9 PEG11 dimer | 3.9 | 4.4 x 10⁵ | 1.7 x 10⁻³ |

### (3) Preparation of cyclic peptide homodimers with introduced palmitoyl group

A homodimer of MuSK7 with an introduced palmitoyl group (Fig. 8/MuSK7-pal) was prepared according to the following scheme. That is, in MuSK7 synthesized in Example 1(2), a cyclic peptide in which a linker was introduced into the carboxyl group of the C-terminal cysteine residue was prepared, and, in the presence of a coupling reagent, two molecules of the cyclic peptide and a linking unit having palmitic acid were subjected to a condensation reaction by a conventional method to obtain a complex. The protecting group (PG) was then removed from the obtained complex, which was purified to obtain a homodimer of MuSK7 with an introduced palmitoyl group (R).

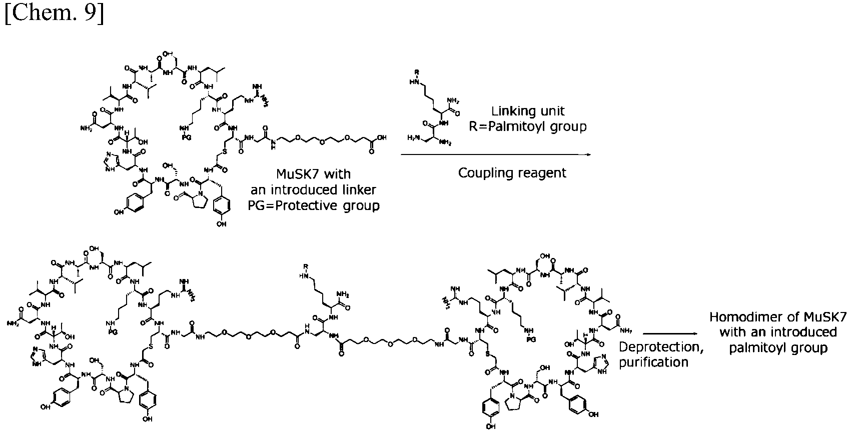

### (4) Evaluation of MuSK-binding activity of cyclic peptide homodimers with introduced palmitoyl group

For the homodimer of MuSK7 with an introduced palmitoyl group (MuSK7-pal) obtained according to the procedure described in (3) above, affinity for MuSK of human, rat, and mouse was evaluated (binding activity test). The test was carried out according to the procedure described in Example 1(3). The results are shown in Table 5.

**[Table 5]**

| Table 5: MuSK-binding activity of various peptides | | | |
|---|---|---|---|
| | Binding activity (K_{D}) (nM) | | |
| | Human | Rat | Mouse |
| Homodimer of MuSK7 with an introduced palmitoyl group (MuSK7-pal) | 0.9 | 2.0 | 8.8 |

### (5) Evaluation of effect on clustering of acetylcholine receptors

### A. Culture of myotube cells

The mouse myoblast-derived cell line C2C12 (CRL-1772) was purchased from the American Type Culture Collection and used. For subculture, Dulbecco's Modified Eagle Medium (DMEM, high glucose, GlutaMAX supplement, pyruvate; GIBCO) supplemented with 20% fetal bovine serum (FBS; GIBCO) was used. For passage, after the culture medium was removed by suction, adherent cells were washed with phosphate buffered saline (PBS(-)), and HBSS (GIBCO) containing no Mg²⁺ and Ca²⁺ and containing 0.25% trypsin and 1 mM ethylenediaminetetraacetic acid was added, and the mixture was reacted in a CO₂ incubator (37°C, 5% CO₂) for 5 minutes. The detached cells were collected and then centrifuged at 1000 rpm at 4°C for 5 minutes (Sorvall X4R Pro, Thermo Fisher Scientific Inc.). After the supernatant was removed by suction, the cells were suspended in fresh culture medium, and 100 µL was seeded into a 96-well plate (BioCoat Collagen I 96-well Black/Clear plate, Corning Inc.) and cultured in a CO₂ incubator (37°C, 5% CO₂) until reaching confluence, typically within 72 hours. Subsequently, the culture medium was removed and the first medium change was performed with DMEM containing 2% horse serum (GIBCO) for induction of myotube cell differentiation. Further, after 48 hours, the second medium change was performed, and on the following day, whether or not the differentiation into myotube cells was successfully induced was confirmed by bright-field observation with a general-purpose inverted microscope.

### B. Induction of AChR clusters and quantitative analysis thereof

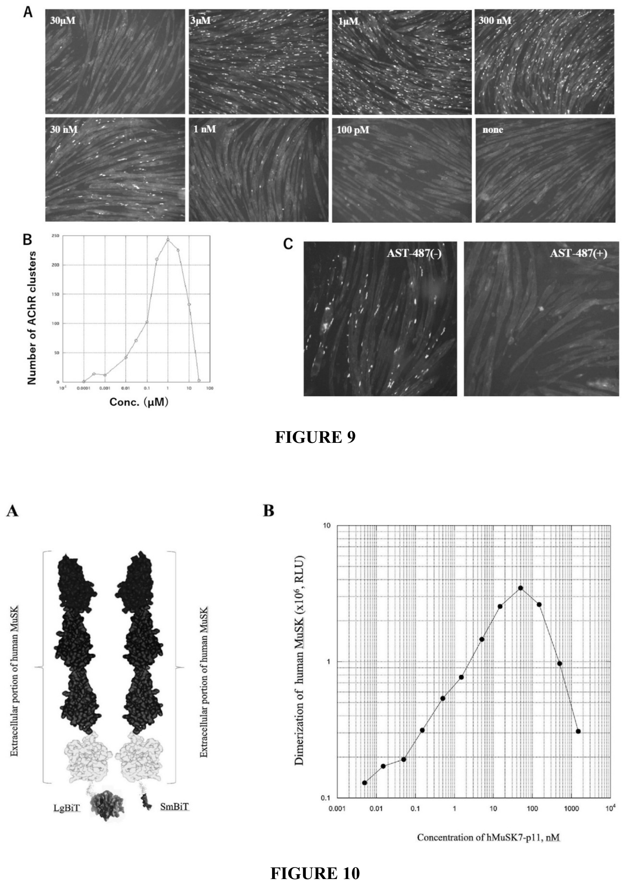

A differentiation medium supplemented with an appropriate amount of the homodimer of the cyclic peptide MuSK7 (MuSK7 PEG11 dimer) was prepared in advance in a 96-well plate (Nunclon Δ Surface, 167008, Nunc), and used at 100 µL/well at the time of the second medium change in the process of inducing differentiation into myotubes. 20 hours after addition of the homodimer of the cyclic peptide MuSK7, the medium was removed, the cells were washed with PBS(-), and then fixed at room temperature for 15 minutes with PBS(-) containing 4% formaldehyde. After washing several times with 1% BSA-PBS(-), fluorescently labeled α-bungarotoxin (α-Btx, Alexa Fluor 488 conjugate; Invitrogen) was added to 1% BSA-PBS(-) at a final concentration of 100 nM, and after adding this to the wells, the mixture was allowed to stand at room temperature under a light-shielded condition for 30 minutes. After washing the cells several times with 1% BSA-PBS(-), 100 µL of PBS(-) was added to each well, and fluorescence observation was performed using a fluorescence imaging device. For observation of fluorescently labeled AChR clusters, an EVOS M7000 imaging system (Thermo Fisher Scientific Inc.) was used. During fluorescence image observation, a 10× objective lens was used, and tiff images of an area equivalent to 1 mm² were obtained. Three tiff images were acquired per well, and structures with a long diameter of 10 µm or more exhibiting Alexa-488 fluorescence signals were defined as AChR clusters and measured. The average number of AChR clusters counted from the three tiff images derived from the same well was calculated and drawn on a graph. The results were as shown in Figs. 9A and 9B. The number of AChR clusters increased in a dose-dependent manner in the concentration range from the pM order to 1 µM with increasing amounts of the homodimer of the cyclic peptide MuSK7 added, indicating induction of clustering of acetylcholine receptors. On the other hand, when the compound concentration exceeded 1 µM, the number of clusters conversely tended to decrease. This bell-shaped dose-response curve is presumed to reflect the properties of the homodimer structure of the homodimer of the cyclic peptide MuSK7. From the above, it was confirmed that the homodimer of the human MuSK-binding cyclic peptide promotes clustering of acetylcholine receptors at the muscle cell level.

### C. Evaluation of drug efficacy of MuSK agonist using MuSK inhibitor

MuSK inhibitor candidates were searched in the KinaseNET database (http://www.kinasenet.ca/) published as an open resource by Kinexus Bioinformatics Corporation (Canada), and three tyrosine kinase inhibitors (AST-487, Foretinib, and Sorafenib) were identified. Next, groups were prepared in which 10 pM recombinant rat agrin protein (550-AG, R&D Systems) derived from C2C12 myotubes was added alone, or in which each tyrosine kinase inhibitor (3 nM) was added simultaneously, and detection and observation of Agrin-induced AChR clusters were performed 20 hours after compound addition (five types, Crizotinib, Linifanib, AST-1306, Dovitinib, and BMS-754807, were used as non-MuSK inhibitory tyrosine kinase inhibitors). As a result, in the group in which ATS-487 was added simultaneously with Agrin, no AChR cluster formation was observed even in a low concentration range of 1 nM. The two inhibitors, Foretinib and Sorafenib, were presumed to have weaker MuSK inhibitory effects compared with AST-487. On the other hand, for the five other tyrosine kinase inhibitors (Crizotinib, Linifanib, AST-1306, Dovitinib, and BMS-754807), the number of AChR clusters was comparable to that in the negative control group (DMSO alone). From the above, it was demonstrated for the first time at the cellular level that AST-487 is a tyrosine kinase inhibitor exhibiting MuSK selectivity.

Next, the effect of AST-487 on the AChR cluster-inducing activity of the homodimer of the cyclic peptide MuSK7 was examined. The results are shown in Fig. 9C. In the absence of AST-487, AChR cluster formation by the homodimer of the cyclic peptide MuSK7 was observed, whereas in the presence of AST-487, no AChR clusters were detected. That is, similar to Agrin, a natural MuSK activator, it was demonstrated at the cellular level that the AChR cluster-inducing process by the homodimer of the cyclic peptide MuSK7 depends on MuSK kinase activity.

### (6) Evaluation of MuSK dimerization activity (NanoBiT assay)

### A. Culture of non-muscle strain cells

Expi293 cells were purchased from Thermo Fisher Scientific Inc. and used. For subculture, DMEM (high glucose, GlutaMAX supplement, pyruvate; GIBCO) supplemented with 10% fetal bovine serum (FBS; GIBCO) was used. For passage, after the culture medium was removed by suction, adherent cells were washed with phosphate buffered saline (PBS(-)), and HBSS (GIBCO) containing no Mg²⁺ and Ca²⁺ and containing 0.25% trypsin and 1 mM ethylenediaminetetraacetic acid was added, and the mixture was reacted in a CO₂ incubator (37°C, 5% CO₂) for 5 minutes. The detached cells were collected and then centrifuged at 1000 rpm at 4°C for 5 minutes (Sorvall X4R Pro, Thermo Fisher Scientific Inc.). After the supernatant was removed by suction, the cells were suspended in fresh culture medium and expanded in a new culture vessel.

### B. Evaluation of MuSK dimerization activity related to MuSK agonist

LgBiT (17.6 kDa) and SmBiT (11 amino acids) are split-type luciferases designed to exhibit luciferase activity when both form a complex (Dixon, A.S. et al. ACS Chem. Biol. 2016, 11, 400-8.). In the steady state, their affinity is estimated to be about 190 µM, and the amount of luminescence is very small even in the presence of substrate. On the other hand, when both are in close proximity, they form a complex that exhibits luciferase activity. A technique for evaluating interactions between two arbitrary proteins by utilizing this property is the NanoBiT assay. Since dimerization between two MuSK molecules is presumed to be one of the requirements for activation of MuSK, which is a receptor-type tyrosine kinase, the NanoBiT assay was employed as one technique to evaluate the MuSK activation ability of MuSK7-pal (Fig. 10A). Specifically, synthetic DNA was prepared in which SmBiT or LgBiT was fused downstream of the extracellular portion (amino acids 1-493) of human MuSK cDNA clone (FXC31275, Promega Corporation) and six histidines were arranged at the C-terminus, and this was incorporated into an EBMulti-Neo plasmid (Fujifilm Wako Pure Chemical Corporation). Using FuGENE6 (Promega Corporation), two types of expression plasmids, MuSK-SmBiT and MuSK-LgBiT, were simultaneously transfected into Expi293 cells, and after culture for 48 to 72 hours in Opti-MEM medium (Invitrogen), the culture supernatant was collected. Next, a plurality of dilutions of the MuSK7-pal solution, as a test compound, were prepared at arbitrary dilution ratios. 50 µL of the collected culture supernatant and 50 µL of the diluted MuSK agonist solution were each added to a 96-well microplate (white, Nunc) and set on a shaker (inVitro Shaker Mix-EVR, TAITEC Corporation). Then, shaking was performed at room temperature for 15 minutes (200 rpm). According to the manual of the Nano-Glo Live Cell Assay (Promega Corporation), 25 µL of substrate solution consisting of the supplied buffer and substrate was added to each well, and then luminescence of luciferase in the wells was immediately detected using a GloMax Navigator (Promega Corporation) (measurement time: 0.5 seconds/well). The results were as shown in Fig. 10B. With increasing amounts of MuSK7-pal added, luminescence increased in a dose-dependent manner in the concentration range from the pM order to 40 nM, indicating that MuSK dimerization was promoted. On the other hand, when the compound concentration exceeded 40 nM, MuSK dimerization conversely tended to decrease. This bell-shaped dose-response curve is presumed to reflect the properties of the homodimer structure of MuSK7-pal. Combining the above results with the results of(5) above, it was confirmed that the homodimer of the human MuSK-binding cyclic peptide promotes MuSK kinase activity-dependent acetylcholine receptor clustering.

### Example 3: Evaluation of therapeutic effect of cyclic peptide complexes on myasthenia gravis model animals

### (1) Evaluation of therapeutic effect on myasthenia gravis model animals (1)

### A. Objective

The effect of a dimer (high-dose) of the cyclic peptide (p7) of Formula (1) (MuSK7-pal/Example 2(3)) on various disease parameters in a rat PTMG model using anti-acetylcholine receptor monoclonal antibody (mAb35) was evaluated.

### B. Test method

5-week-old female rats (n=20, LEW/CrlCrlj, 4 weeks old when carried in, Jackson Laboratory Japan) were divided into four groups (Group A, Group B, Group C, and Group D) using body weight measurement results as indexes. Vehicle was administered to Group A, and the test compound MuSK7-pal dissolved in vehicle was administered intravenously to each of Group B (3 mg/kg), Group C (2 mg/kg), and Group D (1 mg/kg) (Day 0). Two hours after intravenous administration, an anti-nicotinic acetylcholine receptor monoclonal antibody (mAb35, National Hospital Organization Nagasaki Kawatana Medical Center) dissolved in PBS (pH 7.4) as a drug for inducing the pathology of myasthenia gravis was administered intraperitoneally to all animals at 200 µg/rat (Day 0). The vehicle, which was a solvent of the compound, was phosphate-buffered saline (PBS) at pH 7.4 containing 20% hydroxypropyl-β-cyclodextrin and 0.02% polysorbate 80 (the same applies to Example 3(2)).

Next, the body weight of the animals was measured before antibody administration (pre) and at 0, 9, 24, 33, 48, 57, and 72 hours (hr) after antibody administration. The movement, muscle strength, and grip strength of the animals were evaluated by the following tests.

### <Movement score>

The movement of the animals was evaluated at each time point of 9, 24, 33, 48, 57, and 72 hours (hr) after antibody administration according to six-level evaluation criteria (Score). The evaluation criteria were as follows.
0: Normal
1: Reduced grip strength in forelimbs and hindlimbs
2: Incomplete paralysis of hindlimbs
3: Complete paralysis of hindlimbs and inability to stand
4: Moribund
5: Death

The average score of the rats in each group was calculated. In addition, in a cage in which a rat determined to have a score of 2 or higher was present, crushed transport agar (manufactured by Oriental Yeast Co., Ltd.) and pellet feed were provided.

### <Wire-hang Test>

Approximately 30 hours after antibody administration, the muscle strength of the rats was measured using a lid (made of wire mesh) of a lidded plastic cage (Clean 200-TPX cage; CL-0108-2, CLEA Japan, Inc.). Specifically, the rats were left for 15 minutes or longer, and after confirming that they were in a resting state, they were placed on the cage lid. Next, the cage lid was lightly shaken about three times so that the rats would grasp the cage lid, and the cage lid was turned upside down. The inverted lid was held at a height (about 60 cm from the floor), which was high enough to prevent the rats from getting down easily but low enough not to injure them if they fell. The latency until the rats fell from the cage lid was measured for up to 120 seconds.

### <Grip Strength Test>

Approximately 30 hours after antibody administration, the muscle strength of the rats was measured using a mouse grip strength meter (GRIP STRENGTH METER FOR MICE, MODEL MK-380M, Muromachi Kikai Co., Ltd.). Specifically, the rats were left for 15 minutes or longer, and after confirming that they were in a resting state, the forelimbs of the rats were made to grasp the mouse grip strength meter. Next, the bodies of the rats were pulled, and the scale of the grip strength meter at the point when the rats released the wire mesh was read (first measurement). The above procedure was repeated once more (second measurement). When the difference between the first and second measurement values was 20% or less, the average value thereof was used as the measurement value. When the difference was 20% or more, the above procedure was further performed once more (third measurement), and the average value of two measurements with close values among the three measurements was used as the measurement value.

### C. Results

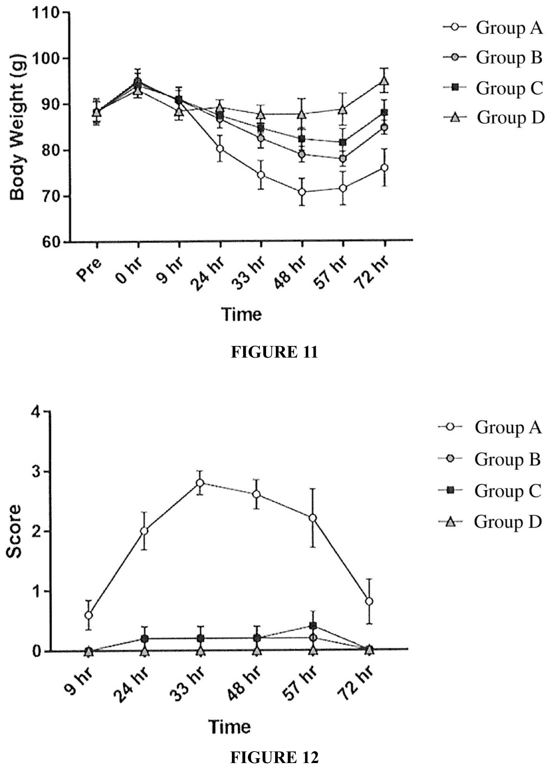

The body weight measurement results in each group are shown in Fig. 11. That is, in the control Group A, a decrease in body weight was observed from 0 hr to 57 hr, followed by a slight increase. In Groups B and C, changes in body weight similar to those in Group A were observed, but the degree of decrease in body weight was milder than that in Group A. In Group D, there was almost no significant decrease in body weight as seen in the other groups.

The movement score measurement results in each group are shown in Fig. 12. That is, in the score results of the control Group A, onset began to be observed from 9 hr, reached a peak at 33 hr, and then declined. In Groups B and C, onset was hardly observed in most individuals, and overall, the scores remained low at all measurement points. In Group D, all individuals showed a score of 0 at all measurement points, and no onset of pathology was observed.

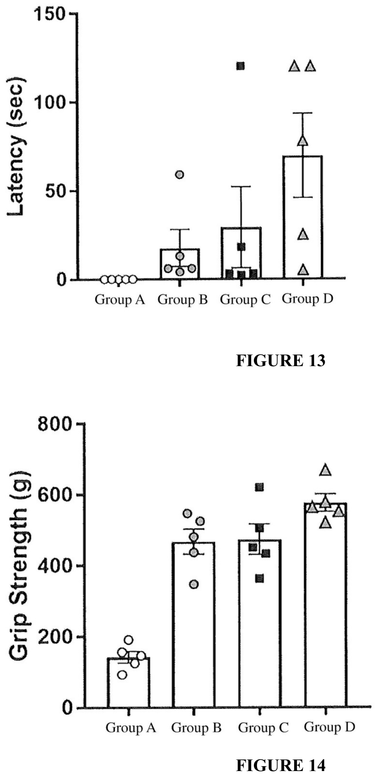

The measurement results of latency to fall (Latency) in the Hanging Wire Test in each group are as shown in Fig. 13. That is, the mean latency to fall in the control Group A was 0.0 seconds (±0.00 SEM), indicating an immediate fall at the start of the test. The mean latency to fall of the test compound administration groups was 17.6 seconds (±10.46 SEM) in Group B, 29.2 seconds (±22.89 SEM) in Group C, and 69.6 seconds (±23.78 SEM) in Group D, which were longer than that of Group A. In Groups B and D, the latencies were significantly longer compared to Group A (Kruskal-Wallis test).

The grip strength measurement results in the Grip Strength Test in each group are as shown in Fig. 14. That is, while the mean grip strength in the control Group A was 142 g (±16.28 SEM), the grip strength in Group B was 467 g (±35.54 SEM), the grip strength in Group C was 474 g (±43.03 SEM), and the grip strength in Group D was 577 g (±24.89 SEM), which were significantly stronger than the grip strength in Group A (one-way analysis of variance (p < 0.0001) and Dunnett's test (p < 0.0001)).

### E. Discussion

As described in C above, the myasthenia gravis-like symptoms observed in the control group (Group A) were alleviated in Groups B and C, and were hardly observed and suppressed in Group D. Accordingly, the efficacy of all test compounds against the symptoms of myasthenia gravis was confirmed, and the effect was particularly remarkable in Group D.

### (2) Evaluation of therapeutic effect on myasthenia gravis model animals (2)

### A. Objective

The effect of MuSK7-pal (Example 2(3)) (low-dose) on various disease parameters in a rat PTMG model using anti-acetylcholine receptor monoclonal antibody (mAb35) was evaluated.

### B. Test method

5-week-old female rats (n=24, LEW/CrlCrlj, 4 weeks old when carried in, Jackson Laboratory Japan) were divided into four groups (Group A, Group B, Group C, and Group D) using body weight measurement results as indexes. Vehicle was administered to Group A, and the test compound MuSK7-pal dissolved in vehicle was administered intravenously to each of Group B (0.5 mg/kg), Group C (0.25 mg/kg), and Group D (0.1 mg/kg) (Day 0). Two hours after intravenous administration, an anti-nicotinic acetylcholine receptor monoclonal antibody (mAb35, National Hospital Organization Nagasaki Kawatana Medical Center) dissolved in PBS (pH 7.4) as a drug for inducing the pathology of myasthenia gravis was administered intraperitoneally to all animals at 200 µg/rat (Day 0).

Next, the body weight of the animals was measured before antibody administration (pre) and at 0, 9, 24, 33, 48, 57, and 72 hours (hr) after antibody administration. The movement, muscle strength, and grip strength of the animals were evaluated by the following tests.

### <Movement score>

The movement of the animals was evaluated at each time point of 9, 24, 33, 48, 57, and 72 hours (hr) after antibody administration according to six-level evaluation criteria (Score). The evaluation criteria were the same as those in (1) B above.

### <Wire-hang Test>

The Wire-hang Test was performed approximately 30 hours after antibody administration. The implementation procedure was the same as that in (1) B above.

### <Grip Strength Test>

The Grip Strength Test was performed approximately 30 hours after antibody administration. The implementation procedure was the same as that in (1) B above.

### C. Results

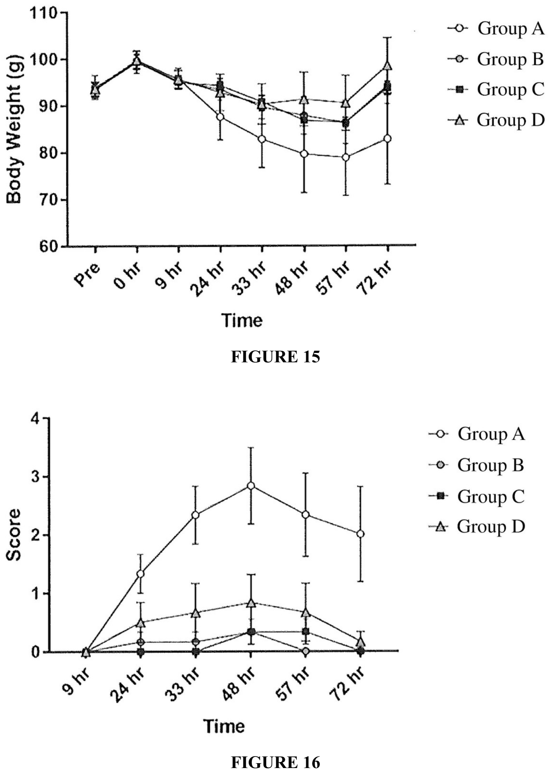

The body weight measurement results in each group are shown in Fig. 15. That is, in the control Group A, a decrease in body weight was observed from 0 hr to 57 hr, followed by a slight increase. In Groups B, C, and D, changes in body weight similar to those in Group A were observed, but the degree of decrease in body weight was milder than that in Group A.

The movement score measurement results in each group are shown in Fig. 16. That is, in the score results of the control Group A, onset began to be observed from 24 hr, reached a peak at 48 hr, and then declined. In Group B, onset was hardly observed in most individuals, and overall, the scores remained low at all measurement points. In Group B, the scores were significantly lower than those in Group A at all measurement points after 24 hr (Dunn's test). In Group C, similar to Group B, onset was hardly observed in most individuals, and the scores remained low at all measurement points. In Group C, the scores were significantly lower than those in Group A at all measurement points except at 9 hr and at 57 hr (Dunn's test). In Group D, no onset was observed in half of the group, and overall, the scores remained low compared to the control group. In Group D, no significant difference from Group A was observed at any measurement point (Dunn's test).

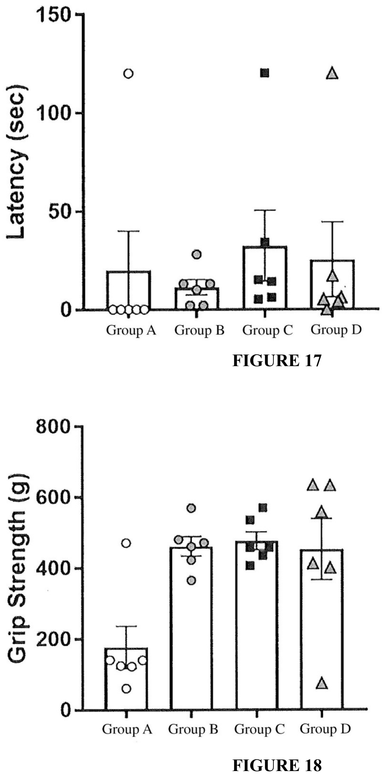

The measurement results of latency to fall (Latency) in the Hanging Wire Test in each group are as shown in Fig. 17. That is, the mean latency to fall in the control Group A was 20.0 seconds (±20.00 SEM), but almost all individuals fell immediately at the start of the test except for some animals. The mean latency to fall of the test compound administration groups was 11.3 seconds (±3.91 SEM) in Group B, 32.3 seconds (±18.04 SEM) in Group C, and 25.3 seconds (±19.07 SEM) in Group D. In Group B, the latency to fall was shorter than that in Group A. However, although no animals except for animal No. D-01 fell immediately at the start of the test, statistical significance was not reached (Kruskal-Wallis test; H(3) = 6.63, n.s.).

The grip strength measurement results in the Grip Strength Test in each group are as shown in Fig. 18. That is, while the mean grip strength in the control Group A was 177 g (±60.05 SEM), the grip strength in Group B was 461 g (±27.61 SEM), the grip strength in Group C was 476 g (±25.51 SEM), and the grip strength in Group D was 452 g (±86.32 SEM). Although the grip strength in Groups B, C, and D was stronger than that in Group A, statistical significance was not reached (Kruskal-Wallis test).

### E. Discussion

As described in C above, regarding the myasthenia gravis-like symptoms observed in the control group (Group A), although decrease in body weight and muscle strength was also observed in the test compound administration groups, the movement scores were mild in Group D and suppressed in Groups B and C. Accordingly, the efficacy of all test compounds against the symptoms of myasthenia gravis was confirmed.

### (3) Summary

From the results of Example 2(4) and those in (1) and (2) above, it was demonstrated that MuSK7-pal, which is one of the cyclic peptides of the present invention, not only has MuSK-binding activity but also actually exerts a therapeutic effect in myasthenia gravis model animals.

Example 4: Grafting of cyclic peptide pharmacophore sequences onto ubiquitin molecules and evaluation of MuSK-binding activity of artificial ubiquitin molecules

### (1) Verification of effectiveness of lasso-grafting method

### A. Outline

When internal sequences of aMD4 and aMD5, which are macrocyclic peptides with MET (hepatocyte growth factor receptor)-binding property (Ito, K. et al., Nat Commun 2015, 6 (1): 6373.), were grafted onto ubiquitin molecules by the lasso-grafting method (Mihara, E. et al., Nat Commun 2021, 12:1543.), it was confirmed that the MET-binding activity of the ubiquitin molecules grafted with the macrocyclic peptides was weaker than that of the original macrocyclic peptides. Therefore, spacer sequences of 0 to 3 amino acid residues randomized at the N-terminal side and the C-terminal side of the pharmacophore sequence of the grafted macrocyclic peptides were added, while leaving the pharmacophore sequence of the macrocyclic peptides unchanged, and a library was prepared and selected to evaluate the MET-binding activity. Specifically, the procedure was carried out as follows.

### B. Ubiquitin molecules as scaffolds for lasso-grafting method

A family of ubiquitin molecule-based ligands in which pharmacophore sequences were lasso-grafted onto a loop structure region (β1-β2 loop) between a β1 domain and a β2 domain of a ubiquitin molecule was prepared (Fig. 19a). The pharmacophore sequence of aMD4 (YRQFNRRTHEVWNLD; SEQ ID NO: 35) or the pharmacophore sequence of aMD5 (YWYYAWDQTYKAFP; SEQ ID NO: 36) was flanked by a "G" or "GG" spacer and substituted for L8-T9 residues or T9-G10 residues in the β1-2 loop (Ub-aMD4_n1-4 and Ub-aMD5_n1-4, naive U-body; Figs. 20a and 20b).

The dissociation constants and binding rate constants of each U-body construct for the human MET ectodomain were measured by SPR at 25°C using a Biacore 8K instrument (Cytiva). The His- and hFc-tagged human MET ectodomain was purchased from Sino Biological Inc. (10692-H03H). The running buffer was an HBS-EP+ buffer (Cytiva) containing 0.01 M HEPES pH 7.4, 0.15 M NaCl, 3 mM EDTA, and 0.05% Surfactant P20. The His- and hFc-tagged human MET ectodomain was immobilized on a Series S Sensor Chip Protein G (Cytiva) according to the standard immobilization protocol provided by the manufacturer. The dissociation constants of the naive U-body constructs were determined by a multi-cycle method in parallel mode. Eight concentrations of each U-body construct were injected at a flow rate of 30 µL/min. The obtained binding responses were analyzed using Biacore Insight evaluation software and fitted with a steady-state affinity model.

As a result, the naive U-bodies bound to MET, but their affinity was significantly weaker in all cases than that of the parent macrocyclic peptides (Figs. 20a and 20b). In addition, since the naive U-body constructs showed almost the same circular dichroism spectrum as that of wild-type Ub, it was found that the ubiquitin molecule serving as a scaffold in the naïve U-body constructs was correctly folded. These results indicate that this decrease in affinity was not due to misfolding but was due to conformational changes in the lasso-grafted pharmacophores. Rather, it was presumed that the decrease in binding activity might be caused by an inappropriate distance between the N-terminal region and the C-terminal region of the pharmacophores in lasso-grafting. It was considered that the decrease in binding activity could be resolved by optimizing the spacers at the grafting sites, taking into account spacer residues that would affect the macrocyclic active form of the pharmacophore peptides (see Fig. 3B).

### C. Optimization of flanking spacer sequences by mRNA display method

In order to optimize the flanking sequences of the pharmacophores, new flanking spacer sequences with excellent binding ability on the ubiquitin scaffold were reselected by the mRNA display method (Figs. 19b and 19c). Two types of mRNA libraries encoding aMD4-grafted U-body (Ub-aMD4) or aMD5-grafted U-body (Ub-aMD5) were constructed. In the construction, various flanking spacer sequences were used, and 0 to 4 consecutive residues in the β1-2 loop were replaced with pharmacophore sequences flanked upstream and downstream by randomized 0 to 3 spacer residues (Figs. 19b and 21). The theoretical diversity of the library was 9.9 × 10⁸, which was less than the number of practical variants translated in in vitro translation cocktail (>10¹¹). Accordingly, all possible combinations were exhaustively explored. Specifically, the procedure was carried out as follows.

### Preparation of mRNA library

A DNA template of an mRNA library was prepared by assembling DNA oligos in two steps. As the first step, a forward primer and a reverse primer were mixed and an extension reaction was performed to obtain double-stranded DNA encoding the β1-β2 loop sequence, spacer sequence, and pharmacophore sequence (the spacer sequences were randomized with NNK codons). The extension reaction was carried out in a 30 µL reaction scale using 1× KOD One PCR Master Mix -Blue-(Toyobo Co., Ltd.) and 0.3 µM forward primer and reverse primer.

As the second step, the extension products were mixed and amplified by PCR to add a constant region. The PCR was carried out in a 300 µL reaction scale using 1× KOD One PCR Master Mix -Blue- (Toyobo Co., Ltd.), the 10% (v/v) mixed extension products, and 0.3 µM forward primer and reverse primer. PCR products were extracted with phenol/chloroform, precipitated with ethanol, dissolved in water, and used for in vitro transcription at 37°C overnight in a 660 µL reaction scale. The transcription reaction contained 40 mM Tris-HCl (pH 8.0), 1 mM spermidine, 0.01% (v/v) Triton X-100, 10 mM DTT, 30 mM MgCl₂, 5 mM NTP, 30 mM KOH, template DNA solution, 0.1% (v/v) RNasin ribonuclease inhibitor (Promega Corporation), and 0.12 µM T7 RNA polymerase. Furthermore, 76 µL of DNase buffer (400 mM Tris-HCl (pH 8.0), 100 mM MgSO₄, 10 mM CaCl₂) and 20 µL of RQ1 RNase-free DNase (Promega Corporation) were added, and the mixture was reacted at 37°C for 1 hour. The obtained mRNA transcripts were precipitated by adding 132 µL of EDTA (pH 8.0), 89 µL of 3 M NaCl, and 785 µL of isopropanol, followed by centrifugation. After washing with 70% (v/v) ethanol, the pellet was dissolved in 150 µL of water, and an equal amount of 2× RNA loading buffer (8 M urea, 2 mM Na₂EDTA-2H₂O, 2 mM Tris-HCl, pH 7.5) was added. The mRNA was purified with a 6% (v/v) polyacrylamide gel containing 8 M urea, extracted with 0.3 M NaCl solution, precipitated, and dissolved in water to a final concentration of 10 µM. Before translation, the mRNA library was covalently bonded to a puromycin linker using T4 RNA ligase.

### Selection by mRNA display

The translation system used for generating the U-body library consists of 50 mM HEPES-KOH (pH 7.6), 100 mM potassium acetate, 12.3 mM magnesium acetate, 2 mM ATP, 2 mM GTP, 1 mM CTP, 1 mM UTP, 20 mM creatine phosphate, 0.1 mM 10-formyl-5,6,7,8- tetrahydrofolate, 2 mM spermidine, 1 mM dithiothreitol, 1.5 mg/mL E. coli total tRNA, 1.2 µM E. coli ribosomes, 0.6 µM methionyl-tRNA formyltransferase, 2.7 µM IF1, 0.4 µM IF2, 1.5 µM IF3, 0.26 µM EF-G, 10 µM EF-Tu, 0.66 µM EF-Ts, 0.25 µM RF2, 0.17 µM RF3, 0.5 µM RRF, 4 µg/mL creatine kinase, 3 µg/mL myokinase, 0.1 µM inorganic pyrophosphatase, 0.1 µM nucleotide diphosphate kinase, 0.1 µM T7 RNA polymerase, 0.73 µM AlaRS, 0.03 µM ArgRS, 0.38 µM AsnRS, 0.13 µM AspRS, 0.02 µM CysRS, 0.06 µM GlnRS, 0.23 µM GluRS, 0.09 µM GlyRS, 0.02 µM HisRS, 0.40 µM IleRS, 0.04 µM LeuRS, 0.11 µM LysRS, 0.03 µM MetRS, 0.68 µM PheRS, 0.16 µM ProRS, 0.04 µM SerRS, 0.09 µM ThrRS, 0.03 µM TrpRS, 0.02 µM TyrRS, and 0.02 µM ValRS, 0.5 mM each of Ala, Arg, Asn, Asp, Cys, Gln, Glu, Gly, His, Ile, Leu, Lys, Met, Phe, Pro, Ser, Thr, Trp, Tyr, and Val, and 1.5 µM mRNA library bonded to a puromycin linker. The U-body library was translated in 2.5 µL of the translation system at 37°C for 30 minutes.

Next, the reaction mixture was incubated at 25°C for 7 minutes. Then, 0.5 µL of 500 mM EDTA (pH 8.0) was added, and the mixture was further incubated at 37°C for 5 minutes to induce dissociation of ribosomes from the mRNA-protein complexes. Reverse transcription was carried out at 42°C for 30 minutes using a reverse primer (SSG2an13.R36 for the Ub-aMD4 library and SGS2an13.R36 for the Ub-aMD5 library) and M-MLV reverse transcriptase (Promega Corporation) lacking RNase H activity. Next, the cDNA-mRNA-protein complexes were mixed with 2.5 µL of Dynabeads Protein G (Thermo Fisher Scientific Inc.) having human IgG1 Fc on the surface, and incubated at 4°C for 5 minutes. This was performed three times as negative selection. The supernatant from the negative selection was recovered and mixed with Dynabeads Protein G immobilized with MET (see (1) B) at 4°C for 10 minutes, followed by washing of the beads three times (first round) or six times (second to sixth rounds) with 100 µL of TBS-T buffer (50 mM Tris-HCl, 150 mM NaCl, 0.05% Tween 20, pH 7.6).

Next, 1 µL of 100 µL of 1× PCR buffer [120 mM Tris-HCl (pH 8.0), 2.5 mM MgCl₂, 6 mM (NH₄)₂SO₄, 10 mM KCl, 0.5 mM dNTPs, 0.001% (w/v) AcBSA, 0.1% (v/v) Triton X-100, 0.25 µM T7G10Ub6.F63, and 0.25 µM reverse primer (SSG2an13.R36 for the Ub-aMD4 library and SGS2an13.R36 for the Ub-aMD5 library)] was added to the beads, and cDNA was eluted by heating the mixture at 95°C for 5 minutes. 1 µL of the eluate was mixed with 19 µL of 1× PCR buffer containing SYBR Green I and KOD DNA polymerase, and the amount of cDNA was quantified by real-time PCR (LightCycler Nano, Roche). The remaining eluate was diluted eightfold with 1× PCR buffer containing KOD DNA polymerase, amplified by PCR, extracted with phenol/chloroform, precipitated with ethanol, dissolved in 40 µL of 50 mM KCl, and used for in vitro transcription according to the same procedure as library preparation. The transcribed mRNA library was precipitated and dissolved in water to a final concentration of 10 µM. If necessary, the transcribed mRNA library was precipitated and then purified using a 4% (v/v) polyacrylamide gel containing 8 M urea.

After six rounds of selection, the entire region of all cDNA libraries was sequenced by a next-generation sequencer. Sequencing by the next-generation sequencer (NGS) was carried out as follows.

Recovered cDNA was amplified by PCR using KOD One PCR Master Mix - Blue- (Toyobo Co., Ltd.) with 0.5 µM Rd1T7g10M.F70 and an13Rd2.R49 as primers according to the manufacturer's instructions. The product was subjected to a second PCR step, in which sequence barcodes were installed in each sample using KOD One PCR Master Mix -Blue- and Nextera XT v2 Set (Illumina, Inc., sequence) primers. Thereafter, the PCR products were combined, extracted with phenol/chloroform, and column-purified using a NucleoSpin kit (Takara Bio Inc.) according to the manufacturer's instructions. The concentration of the cDNA samples was measured with a Qubit (Thermo Fisher Scientific Inc.) with a dsDNA HS kit. The obtained DNA was appropriately diluted, denatured, and combined with denatured PhiX Control v3 (Illumina, Inc.). The denatured DNA was sequenced on a MiSeq instrument of Illumina, Inc. in paired-end read 2×300 cycle mode using a v3 kit, and data were collected in fastq format after stitching.

As a result of sequence analysis, U-bodies with MET-binding activity comparable to that of the parent macrocyclic peptides were obtained (Figs. 20a and 20b).

### E. Discussion

In this manner, when grafting pharmacophores of cyclic peptides onto ubiquitin molecules, it was shown that it is effective to introduce randomized spacer sequences upstream and downstream of the pharmacophore sequences and to carry out selection while optimizing the spacer sequences by the mRNA display method.

### (2) Grafting of cyclic peptides onto ubiquitin molecules and selection of grafted ubiquitin molecules (U-bodies) binding to MuSK

Based on the findings of (1) above, lasso-grafting of the pharmacophore of MuSK7 (Example 1(1)) onto the β1-2 loop of ubiquitin molecules was examined. Optimization of the spacer sequences adjacent upstream and downstream of the pharmacophore was carried out as follows. That is, in order to optimize the flanking sequences of the pharmacophores, new flanking spacer sequences with excellent binding ability on the ubiquitin scaffold were reselected by the mRNA display method. An mRNA library encoding a part of the amino acid sequence of MuSK7 (YPSYHTNVVASLKR) (SEQ ID NO: 37) was constructed. In the construction, as shown in Fig. 22, the β1-2 loop (T7-G10) sequence was replaced with "random sequence (3 or 4 amino acids)-YPSYHTNVVASLKR-random sequence (3 or 4 amino acids)". The theoretical diversity of the library was 2.8 × 10¹⁰, which was less than the number of practical variants translated in in vitro translation cocktail (>10¹¹). Accordingly, all possible combinations were exhaustively explored. Preparation of the mRNA library and selection by mRNA display using MuSK-binding property as an index were carried out in the same manner as in (1) C above.

The recovery rates (%) in each of the six rounds are as shown in Fig. 23. In addition, after six rounds of selection, the entire region of all cDNA libraries was sequenced by a next-generation sequencer. Sequencing by next-generation sequencing was carried out in the same manner as in (1) C above.

As a result of sequence analysis, ubiquitin molecules grafted with the following MuSK-binding regions (including spacer regions) (SEQ ID NOs: 38 to 45) (hereinafter sometimes referred to simply as Ub006 to Ub013) were obtained.
(Ub006) IQPRYPSYHTNVVASLKRIRY (SEQ ID NO: 22)
(Ub007) KPGVYPSYHTNVVASLKRMMP (SEQ ID NO: 23)
(Ub008) WDRGYPSYHTNVVASLKRQGI (SEQ ID NO: 24)
(Ub009) KPGTYPSYHTNVVASLKRFMP (SEQ ID NO: 25)
(Ub010) PFGVYPSYHTNVVASLKRTGPQ (SEQ ID NO: 26)
(Ub011) YFVGYPSYHTNVVASLKRWNTP (SEQ ID NO: 27)
(Ub012) LEGMYLSYHTNVVASLKRIRPQ (SEQ ID NO: 28)
(Ub013) LEGMYPSYHTNVVASLKRIRPQ (SEQ ID NO: 29)

### (3) Preparation of MuSK-binding-region-grafted ubiquitin molecules

Ub006 to Ub013, and a homodimer and a homotetramer of Ub012, were expressed from recombinant microorganisms and prepared. The homodimer and homotetramer of Ub012 were each prepared by linking the C-terminus and N-terminus of Ub012 with a peptide linker (A(PA)₇). As for complexes, as a control, a dimer of ubiquitin molecules was prepared. Specifically, a dimer of ubiquitin molecules was prepared by linking the C-terminus of one ubiquitin molecule and the N-terminus of another ubiquitin molecule with a peptide linker (A(PA)₇).

Polynucleotides encoding Ub006 to Ub013 (SEQ ID NOs: 38 to 45), a polynucleotide encoding the homodimer of Ub012 (SEQ ID NO: 54), a polynucleotide encoding the homotetramer of Ub012 (SEQ ID NO: 56), and a polynucleotide encoding the dimer of ubiquitin molecules (SEQ ID NO: 58) were each cloned into a pET22b(+) vector and expressed with a His6-tag attached to the N-terminus. The amino acid sequences of the expressed proteins and the polynucleotides encoding them were as follows.
- Amino acid sequences of expressed Ub006 to Ub013: amino acid sequences of SEQ ID NOs: 38 to 45 with "MHHHHHH" added to the N-terminus
- DNA sequences of expressed Ub006 to Ub013: SEQ ID NOs: 46 to 53
- Amino acid sequence of expressed homodimer of Ub012: amino acid sequence of SEQ ID NO: 54 with "MHHHHHHAS" added to the N-terminus and "TS" added to the C-terminus
- DNA sequence of expressed homodimer of Ub012: SEQ ID NO: 55
- Amino acid sequence of expressed homotetramer of Ub012: amino acid sequence of SEQ ID NO: 56 with "MHHHHHHAS" added to the N-terminus and "TS" added to the C-terminus
- DNA sequence of expressed homotetramer of Ub012: SEQ ID NO: 57
- Amino acid sequence of expressed homodimer of ubiquitin molecules: amino acid sequence of SEQ ID NO: 58 with "MHHHHHHAS" added to the N-terminus and "TS" added to the C-terminus
- DNA sequence of expressed homodimer of ubiquitin molecules: SEQ ID NO: 59

All proteins were expressed in E. coli BL21-gold (DE3) cells (Agilent Technologies). The cells were grown in Lysogeny broth medium (Miller) containing 100 µg/mL ampicillin to OD600 > 0.4 and induced with 0.4 mM IPTG at 16°C for 24 hours. The cells were lysed by sonication in a lysis buffer [150 mM NaCl (pH 7.6) containing 50 mM Tris-HCl and 20 mM imidazole-HCl (pH 7.2)], and proteins were captured with Ni Sepharose 6 Fast Flow (Cytiva). The Sepharose beads were washed with a wash buffer [1 M NaCl (pH 7.6) containing 50 mM Tris-HCl and 20 mM imidazole-HCl (pH 7.2)] and the above lysis buffer. The proteins were eluted from the Sepharose beads with an elution buffer [150 mM NaCl (pH 7.6) containing 50 mM Tris-HCl and 500 mM imidazole-HCl (pH 7.2)].

Ub006 to Ub013, and the homodimer and homotetramer of Ub012, were further purified by a size exclusion chromatography on an AKTA avant 25 (Cytiva) or AKTA pure (Cytiva) system using a Superdex 75 Increase 10/300 GL (Cytiva) or HiLoad 16/600 Superdex 200 pg (Cytiva) column [running buffer: 50 mM Tris-HCl, 150 mM NaCl (pH 7.6)]. The purified proteins were stored at -80°C for further use. Protein concentrations were determined by ultraviolet absorbance at 280 nm measured with a NanoDrop 2000c or NanoDrop One C spectrophotometer (Thermo Fisher Scientific Inc.).

### (4) Evaluation of binding affinity of MuSK-binding-region-grafted ubiquitin molecules and their complexes to MuSK (in vitro test)

The affinity of the U-bodies and their complexes obtained as described above for MuSK of human, rat, and mouse was evaluated (binding activity test). The test was carried out according to the procedure described in Example 1(3). However, for the complexes of U-bodies, surface plasmon resonance (SPR) analysis was carried out using a Biacore 8K (Cytiva). The results are shown in Table 6.

**[Table 6]**

| Table 6: MuSK-binding activity of various U-bodies and complexes | | | |
|---|---|---|---|
| | Binding activity (K_{D}) (nM) | | |
| | Human | Rat | Mouse |
| Ub006 | 38 | 117 | 306 |
| Ub007 | 62 | 178 | 606 |
| Ub008 | 140 | 681 | - |
| Ub009 | 46 | 144 | 329 |
| Ub010 | 39 | 614 | - |
| Ub011 | 264 | - | - |
| Ub012 | 33 | 117 | 224 |
| Ub013 | 500 | - | - |
| Homodimer of Ub012 | 0.65 | 0.01 | 2.0 |
| Homotetramer of Ub012 | Less than 0.01 | Less than 0.01 | Less than 0.01 |

| | | | |
|---|---|---|---|
| * "-"indicates that measurement was not performed. | | | |

### (5) Evaluation of effect of U-bodies and their complexes on clustering of acetylcholine receptors (cellular level test)

The effect of the U-bodies and their complexes on clustering of acetylcholine receptors was evaluated. The test for evaluating AChR cluster formation using the mouse myoblast-derived cell line C2C12 was carried out according to the procedure described in Example 2(5). The evaluation test using the NanoBiT assay was carried out according to the procedure described in Example 2(6). The results were as shown in Figs. 24 to 26.

Fig. 24 shows the test results of the NanoBiT assay. From Fig. 24, it was confirmed that the homodimer of Ub012 (Dimeric U-bodies) had stronger MuSK dimerization activity than the homodimer of a cyclic peptide (Dimeric peptide/MuSK7-pal). Fig. 25 shows the test results of evaluation of AChR cluster formation using myotube cells. From Fig. 25, it was confirmed that the homodimer of Ub012 (dimeric U-body) had a concentration-dependent promoting effect on AChR cluster formation. Fig. 26A shows the test results of the NanoBiT assay. From Fig. 26A, it was confirmed that the homotetramer of Ub012 (Tetrameric U-bodies) had stronger MuSK dimerization activity than the homodimer of Ub012 (Dimeric U-bodies). Fig. 26B shows the test results of evaluation of AChR cluster formation using myotube cells. From Fig. 26B, it was confirmed that the homotetramer of Ub012 (tetrameric U-bodies) had a concentration-dependent promoting effect on AChR cluster formation.

### (6) Evaluation of therapeutic effect of U-body complexes on myasthenia gravis model animals

### A. Objective

The effect of U-body complexes (tetramers) on various disease parameters in a rat PTMG model using anti-acetylcholine receptor monoclonal antibody (mAb35) was evaluated. In this example, a homotetramer of Ub012 (SEQ ID NO: 60) constructed by linking the C-terminus and N-terminus of Ub012 with a peptide linker (EAAAK)₃, and a homotetramer of Ub012(ΔGG) (SEQ ID NO: 61) constructed by linking the C-terminus and N-terminus of Ub012 with a peptide linker (EAAAK)₃, in which the two amino acid residues (GG) at the C-terminus of Ub012 were deleted, were prepared and used in the following tests.

### B. Test method

5-week-old female rats (LEW/CrlCrlj, 4 weeks old when carried in, Jackson Laboratory Japan) were divided into three groups (Group A, Group B, and Group C) using body weight measurement results as indexes. The test compound (tetramer of Ub012 (EAAAK)₃) dissolved in vehicle was administered intravenously to Group A (5 mg/kg) (n=5), the test compound (tetramer of Ub012(ΔGG) (EAAAK)₃) dissolved in vehicle was administered intravenously to Group B (5 mg/kg) (n=5), and vehicle was administered intravenously to Group C (n=5), respectively (Day 0). Two hours after intravenous administration, an anti-nicotinic acetylcholine receptor monoclonal antibody (mAb35, National Hospital Organization Nagasaki Kawatana Medical Center) dissolved in PBS (pH 7.4) as a drug for inducing the pathology of myasthenia gravis was administered intraperitoneally to all animals at 200 µg/rat (Day 0).

Next, the body weight of the animals was measured before antibody administration (pre) and at 0, 9, 24, 33, 48, 57, and 72 hours (hr) after antibody administration. The movement, muscle strength, and grip strength of the animals were evaluated by the following tests.

### <Movement score>

The movement of the animals was evaluated at each time point of 9, 24, 33, 48, 57, and 72 hours (hr) after antibody administration according to six-level evaluation criteria (Score). The evaluation criteria were the same as those in Example 3(1) B.

### <Wire-hang Test>

The Wire-hang Test was performed approximately 30 hours after antibody administration. The implementation procedure was the same as that in Example 3(1) B.

### <Grip Strength Test>

The Grip Strength Test was performed approximately 30 hours after antibody administration. The implementation procedure was the same as that in Example 3(1) B.

### C. Results

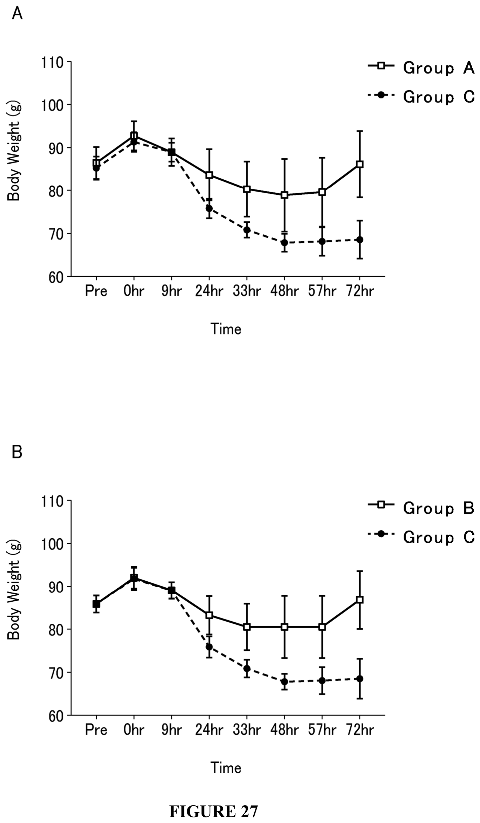

The body weight measurement results in each group are shown in Fig. 27. That is, in the control Group C, a decrease in body weight was observed from 0 hr to 57 hr. In Groups A and B, changes in body weight similar to those in Group C were observed, but the degree of decrease in body weight was significantly milder than that in Group C (Tukey's test; Groups A and B: 0 hr > 33 hr, 48 hr, 57 hr, p < 0.05).

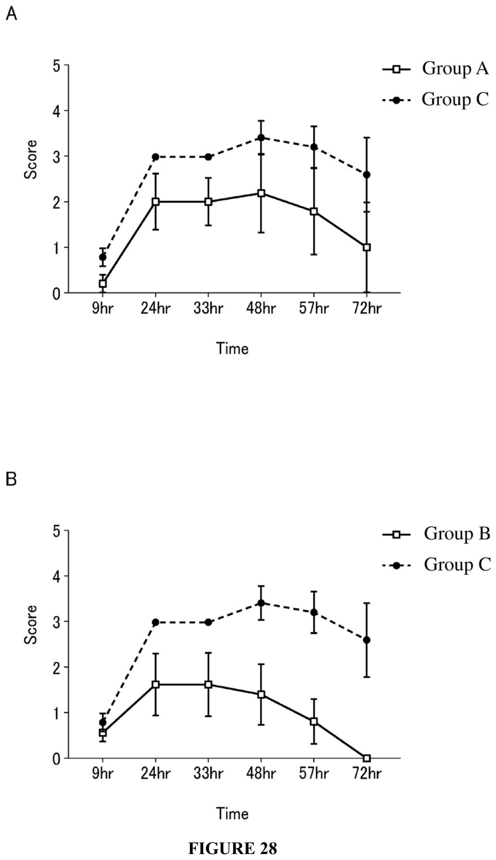

The movement score measurement results in each group are shown in Fig. 28. That is, in the score results of the control Group C, the onset of pathology began to be observed from 9 hr, reached a peak at 48 hr, and then declined. In Groups A and B, the onset of pathology was observed from 9 hr as in Group C, but the scores at the peak of Groups A and B were lower than the score at the peak of Group C. However, no significant difference was observed between the groups at any of the measurement points (Kruskal-Wallis test).

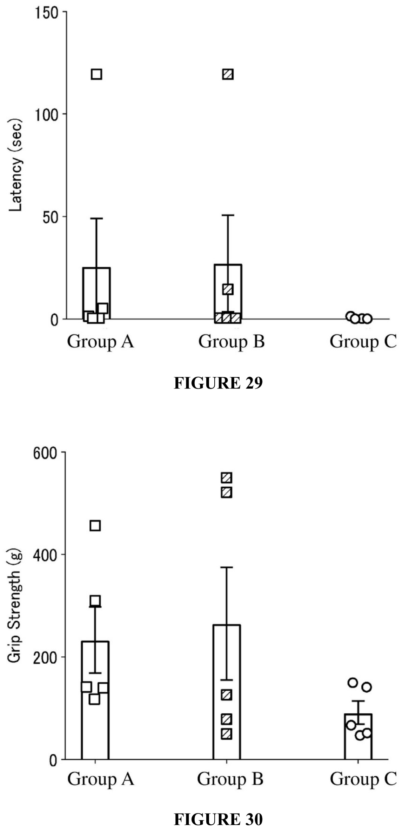

The measurement results of latency to fall (Latency) in the Hanging Wire Test in each group are as shown in Fig. 29. That is, the mean latency to fall in the control Group C was 0.2 seconds (±0.20 SEM), and 4 out of 5 cases fell immediately at the start of the test. On the other hand, the mean latencies to fall in Groups A and B were 25.2 seconds (±23.72 SEM) and 26.8 seconds (±23.46 SEM), respectively, and in two or more cases the fall did not occur immediately at the start of the test. Although the mean latencies to fall in Groups A and B were slightly longer than that in the control group, no statistically significant differences were observed between the groups (Kruskal-Wallis test; H(9) = 13.20, n.s.).

The grip strength measurement results in the Grip Strength Test in each group are as shown in Fig. 30. That is, the mean grip strength in the control Group C was 89 g (±22.24 SEM). On the other hand, the grip strengths in Groups A and B were 233 g (±65.16 SEM) and 264 g (±111.10 SEM), respectively, which were stronger than that in Group C, but no statistically significant differences were observed between the groups (Kruskal-Wallis test; H(9) = 8.72, n.s.).

### E. Discussion

As described in C above, myasthenia gravis-like symptoms such as decrease in body weight, increase in scores, and decrease in muscle strength observed in the control group (Group C) were also observed in the test compound-administered groups. However, although no statistically significant differences were observed, the pathology in Groups A and B was clearly milder than that in the control group, and therefore the efficacy of the two test compounds against myasthenia gravis symptoms was confirmed.

## Claims

1. A cyclic peptide or a pharmaceutically acceptable salt thereof, the cyclic peptide having a structure of the following formula (I):
-X₁-Asn-X₂-Val- (I)
wherein, X₁ represents Thr or Val, and X₂ represents Val or Ile.

2. The cyclic peptide or the pharmaceutically acceptable salt thereof according to claim 1, wherein the number of amino acid residues forming the cyclic structure of the cyclic peptide is 8 to 20.

3. The cyclic peptide or the pharmaceutically acceptable salt thereof according to claim 1 or 2, wherein the cyclic structure of the cyclic peptide includes an N-CO-CH₂-S structure.

4. The cyclic peptide or the pharmaceutically acceptable salt thereof according to claim 3, wherein the N is an amino group of tyrosine.

5. The cyclic peptide or the pharmaceutically acceptable salt thereof according to claim 3 or 4, wherein the S is a thiol group of cysteine.

6. The cyclic peptide or the pharmaceutically acceptable salt thereof according to claim 1 or 3, wherein the cyclic peptide has a structure selected from the group consisting of the following formula (1) (SEQ ID NO: 2), formula (2) (SEQ ID NO: 3), and formula (3) (SEQ ID NO: 4): 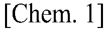 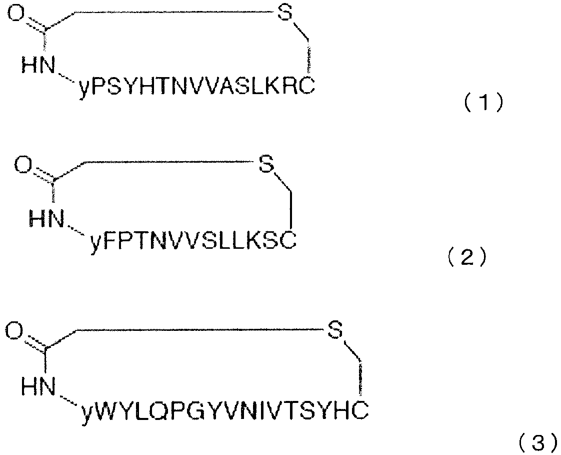 wherein, the carboxyl group of the cysteine residue (C) may form an acid amide.

7. A cyclic peptide complex or a pharmaceutically acceptable salt thereof, the cyclic peptide complex comprising: two cyclic peptides according to claim 1, wherein one of the cyclic peptides is bonded to the other cyclic peptide via a linker.

8. The cyclic peptide complex or the pharmaceutically acceptable salt thereof according to claim 7, wherein the two cyclic peptides are the same cyclic peptide.

9. The cyclic peptide complex or the pharmaceutically acceptable salt thereof according to claim 7 or 8, wherein the length of the linker is 20Å to 100Å.

10. The cyclic peptide complex or the pharmaceutically acceptable salt thereof according to claim 9, wherein the linker has a polyethylene glycol structure as at least a part of the structure.

11. The cyclic peptide complex or the pharmaceutically acceptable salt thereof according to claim 7 or 10, wherein the linker has at least one saturated hydrocarbon group having 8 to 20 carbon atoms as a side chain thereof.

12. A MuSK-binding-region-grafted ubiquitin molecule or a pharmaceutically acceptable salt thereof, wherein a part or all of a loop structure region between a β1 domain and a β2 domain of the ubiquitin molecule is replaced with a peptide of the following structure (A), or the peptide of the structure (A) is inserted into the loop structure region between the β1 domain and the β2 domain of the ubiquitin molecule: 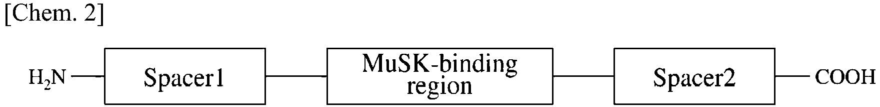 wherein,
the MuSK-binding region consists of the amino acid sequence YX₂₁SYHTNVVASLKR (X₂₁ is P or L) (SEQ ID NO: 11),
spacer 1 consists of an amino acid sequence selected from the group consisting of IQPR (SEQ ID NO: 12), KPGV (SEQ ID NO: 13), WDRG (SEQ ID NO: 14), KPGT (SEQ ID NO: 15), PFGV (SEQ ID NO: 16), YFVG (SEQ ID NO: 17), and LEGM (SEQ ID NO: 18); and
spacer 2 consists of an amino acid sequence selected from the group consisting of IRY, MMP, QGI, FMP, TGPQ (SEQ ID NO: 19), WNTP (SEQ ID NO: 20), and IRPQ (SEQ ID NO: 21).

13. The MuSK-binding-region-grafted ubiquitin molecule or the pharmaceutically acceptable salt thereof according to claim 12, wherein the ubiquitin molecule is a protein selected from the group consisting of:
(a) a protein consisting of the amino acid sequence of SEQ ID NO: 1,
(b) a protein consisting of an amino acid sequence having 90% or more identity to the amino acid sequence of SEQ ID NO: 1, and
(c) a protein consisting of an amino acid sequence in which one or a plurality of amino acids in the amino acid sequence of SEQ ID NO: 1 are modified.

14. The MuSK-binding-region-grafted ubiquitin molecule or the pharmaceutically acceptable salt thereof according to claim 12 or 13, wherein the structure (A) has an amino acid sequence selected from the group consisting of the following
(Ub006) IQPRYPSYHTNVVASLKRIRY (SEQ ID NO: 22)
(Ub007) KPGVYPSYHTNVVASLKRMMP (SEQ ID NO: 23)
(Ub008) WDRGYPSYHTNVVASLKRQGI (SEQ ID NO: 24)
(Ub009) KPGTYPSYHTNVVASLKRFMP (SEQ ID NO: 25)
(Ub010) PFGVYPSYHTNVVASLKRTGPQ (SEQ ID NO: 26)
(Ub011) YFVGYPSYHTNVVASLKRWNTP (SEQ ID NO: 27)
(Ub012) LEGMYLSYHTNVVASLKRIRPQ (SEQ ID NO: 28)
(Ub013) LEGMYPSYHTNVVASLKRIRPQ (SEQ ID NO: 29).

15. The MuSK-binding-region-grafted ubiquitin molecule or the pharmaceutically acceptable salt thereof according to claim 12 or 13, wherein the loop structure region is a region from the 7th amino acid residue to the 10th amino acid residue of the ubiquitin molecule.

16. A MuSK-binding-region-grafted ubiquitin molecule complex or a pharmaceutically acceptable salt thereof, the MuSK-binding-region-grafted ubiquitin molecule complex comprising two or more of the MuSK-binding-region-grafted ubiquitin molecules according to claim 12 or 13, wherein one of the MuSK-binding-region-grafted ubiquitin molecules is bonded to the other MuSK-binding-region-grafted ubiquitin molecule via a linker.

17. The MuSK-binding-region-grafted ubiquitin molecule complex or the pharmaceutically acceptable salt thereof according to claim 16, wherein the two or more MuSK-binding-region-grafted ubiquitin molecules are the same molecule.

18. The MuSK-binding-region-grafted ubiquitin molecule complex or the pharmaceutically acceptable salt thereof according to claim 16 or 17, wherein the length of the linker is 20Å to 100Å.

19. The MuSK-binding-region-grafted ubiquitin molecule complex or the pharmaceutically acceptable salt thereof according to claim 16 or 18, wherein the linker is a peptide chain.

20. A pharmaceutical composition or a therapeutic agent for myasthenia gravis, comprising, as an active ingredient: the cyclic peptide or the pharmaceutically acceptable salt thereof according to claim 1; the cyclic peptide complex or the pharmaceutically acceptable salt thereof according to claim 7; the MuSK-binding-region-grafted ubiquitin molecule or the pharmaceutically acceptable salt thereof according to claim 12; or the MuSK-binding-region-grafted ubiquitin molecule complex or the pharmaceutically acceptable salt thereof according to claim 16.

21. A method for presenting a cyclic peptide having binding activity to a desired molecule (preferably a MuSK molecule) together with two spacer sequences on a loop structure region of a ubiquitin molecule, the method comprising:
fusing the cyclic peptide to the ubiquitin molecule while retaining binding activity to the desired molecule (preferably the MuSK molecule) by replacing a chemical cross-linking structure of the cyclic peptide with two amino acid residues constituting the loop structure via the spacer sequences, wherein
the cyclic peptide has the chemical cross-linking structure for forming an intramolecular cyclic structure, and
the spacer sequences consist of an amino acid sequence of 0 to 4 residues randomly generated.

22. The presentation method according to claim 21, wherein
the desired molecule is a MuSK molecule, and the cyclic peptide and the spacer sequences presented on the loop structure region of the ubiquitin molecule have the following structure (B): 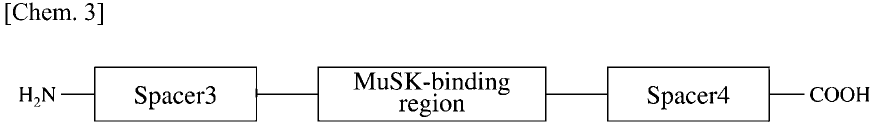
wherein, spacer 3 is a peptide chain consisting of 0 to 4 arbitrary amino acids, spacer 4 is a peptide chain consisting of 0 to 4 arbitrary amino acids, and the MuSK-binding region is a region which is present in the cyclic peptide and has MuSK molecule-binding activity.

23. The presentation method according to claim 21 or 22, wherein the loop structure region of the ubiquitin molecule is a loop structure region between a β1 domain and a β2 domain.

24. The presentation method according to claim 23, wherein the two amino acid residues constituting the loop structure region are the 7th amino acid residue and the 10th amino acid residue of the ubiquitin molecule.

25. The presentation method according to claim 22, wherein the MuSK-binding region is a peptide chain having the amino acid sequence YX₂₂SYHTNVVASLKR (X₂₂ is P or L) (SEQ ID NO: 11).

26. A method for producing a modified protein in which a cyclic peptide having binding activity to a desired molecule (preferably a MuSK molecule) is presented on a protein by fusing the cyclic peptide, together with two spacer sequences, to a loop structure region of a ubiquitin molecule, the method comprising:
selecting a partial amino acid sequence to be presented on the protein from the amino acid sequence of the cyclic peptide, and selecting a nucleotide sequence corresponding to the partial amino acid sequence;
selecting a nucleotide sequence corresponding to two amino acid residues constituting the loop structure region of the ubiquitin molecule, deleting bases present between the selected nucleotide sequences as necessary, and preparing a nucleic acid having an incorporated nucleotide sequence obtained by inserting the nucleotide sequence corresponding to the selected partial amino acid sequence of the cyclic peptide; and
translating the nucleic acid, wherein
the cyclic peptide has a chemical cross-linking structure for forming an intramolecular cyclic structure, and
the spacer sequences consist of an amino acid sequence of 0 to 4 residues randomly generated.

27. The production method according to claim 26,
wherein the desired molecule is a MuSK molecule, and the cyclic peptide and the spacer sequences presented on the loop structure region of the ubiquitin molecule have the following structure (B): 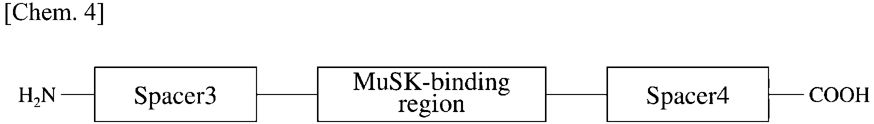
wherein, spacer 3 is a peptide chain consisting of 0 to 4 arbitrary amino acids, spacer 4 is a peptide chain consisting of 0 to 4 arbitrary amino acids, and the MuSK-binding region is a region which is present in the cyclic peptide and has MuSK molecule-binding activity.

28. The production method according to claim 26 or 27, wherein the loop structure region of the ubiquitin molecule is a loop structure region between a β1 domain and a β2 domain.

29. The production method according to claim 28, wherein the two amino acid residues constituting the loop structure region are the 7th amino acid residue and the 10th amino acid residue of the ubiquitin molecule.

30. The production method according to claim 27, wherein the MuSK-binding region is a peptide chain having the amino acid sequence YX₂₂SYHTNVVASLKR (X₂₂ is P or L) (SEQ ID NO: 11).

31. A method for screening for a modified protein which binds to a desired molecule (preferably a MuSK-binding modified protein), the method comprising:
designing an mRNA library including a nucleotide sequence encoding a ubiquitin molecule in which a sequence of a cyclic peptide having binding activity to the desired molecule (preferably a MuSK molecule) and spacer sequences consisting of amino acid sequences of 0 to 4 residues randomly generated and linked upstream and downstream of the cyclic peptide sequence are fused to a loop structure region between a β1 domain and a β2 domain;
preparing a modified protein according to the production method according to claim 28 on the basis of the designed mRNA library; and
selecting, from the obtained modified protein, a modified protein having binding activity to the desired molecule (preferably the MuSK-binding modified protein), using binding activity (preferably MuSK-binding activity) to the desired molecule as an index.

32. A peptide or a pharmaceutically acceptable salt thereof, the peptide having a structure of the following formula (I):
-X₁-Asn-X₂-Val- (I)
wherein, X₁ represents Thr or Val, and X₂ represents Val or Ile.

33. The peptide or the pharmaceutically acceptable salt thereof according to claim 32, wherein the peptide consists of 8 to 20 amino acid residues.

34. The peptide or the pharmaceutically acceptable salt thereof according to claim 32 or 33, the peptide being grafted into a biological molecule.
